# FASCICULE DE BREVET EUROPEEN

(11) **EP 1 268 429 B1**
(45) Date de publication et mention de la délivrance du brevet: **18.08.2004**
(21) Numéro de dépôt: 01969041.1
(22) Date de dépôt: 06.03.2001
(51) Int. Cl.: C07D 213/74, C07D 223/12, C07D 233/54, C07D 235/14, C07D 401/12, C07D 403/12, A61K 31/4164, A61K 31/4184, A61K 31/44, A61K 31/55, A61P 9/00

(54) **COMPOSES BICYCLIQUES ANTAGONISTES DES RECEPTEURS DE LA VITRONECTINE, LEUR PROCEDE DE PREPARATION ET LES COMPOSITIONS QUI LES CONTIENNENT**
BICYCLISCHE VERBINDUNGEN ALS VITRONECTINREZEPTORANTAGONISTEN, VERFAHREN ZUR IHRER HERSTELLUNG UND DIESE ENTHALTENDE PHARMAZEUTISCHE ZUBEREITUNGEN
VITRONECTIN RECEPTOR ANTAGONIST BICYCLIC COMPOUNDS, PREPARATION METHOD AND COMPOSITIONS CONTAINING SAME

(30) Priorité: 07.03.2000 FR 0002902
(43) Date de publication de la demande: 02.01.2003
(73) Titulaire: Les Laboratoires Servier, 92415 Courbevoie Cedex (FR)
(72) Inventeur: CASARA, Patrick, F-78670 Villennes sur Seine (FR); PERRON-SIERRA, Françoise, F-75014 Paris (FR); ATASSI, Ghanem, F-92210 Saint Cloud (FR); TUCKER, Gordon, F-75017 Paris (FR); SAINT-DIZIER, Dominique, F-92500 Rueil Malmaison (FR)
(86) Numéro de dépôt international: PCT/FR2001/000650
(87) Numéro de publication internationale: WO 2001/079172

(56) Documents cités:
- WO-A-96/00730
- WO-A-96/06087
- WO-A-98/14192
- WO-A-98/30542
- WO-A-99/05107
- WO-A-99/15508

## Description

La présente invention concerne de nouveaux composés bicycliques antagonistes des récepteurs de la vitronectine, leur procédé de préparation et les compositions pharmaceutiques qui les contiennent.

Les intégrines constituent une famille de glycoprotéines transmembranaires identifiées initialement dans les phénomènes dynamiques d'adhérence et de migration cellulaires et assurant un lien mécanique entre la matrice extracellulaire et d'autres molécules adhésives de surface et le cytosquelette. Ultérieurement leur capacité à transmettre directement un signal intracellulaire a été décrite. Ces deux propriétés d'agent de couplage et de récepteur sont utilisées par les cellules au cours du développement embryonnaire et lors de nombreux processus physiologiques (Cell., 1992, 69, 11-25 ; Endocrine Reviews, 1996, 17, 207-220 ; Cell. Mol. Life Sci., 1998, 54, 514-526).

Une intégrine est constituée de deux chaînes distinctes alpha et beta liées entre elles de façon non covalente. Au moins seize chaînes alpha et huit chaînes beta ont été identifiées, leur combinaison permettant de créer un vaste répertoire de possibilités. Parmi celle-ci seulement une vingtaine d'intégrines ont été décrites. Le type de combinaison des sous-unités dicte aussi le répertoire des ligands extracellulaires reconnus. La séquence peptidique Arg-Gly-Asp (RGD) est souvent reconnue par les intégrines et présente au sein de nombreux ligands (fribronectine, vitronectine, fibrinogène, collagène, ...), mais d'autres sites de reconnaissance ont été décrits. Une intégrine peut exister sous une forme incapable de fixer ses ligands et nécessitera donc une activation par convergence de divers signaux intracellulaires pour devenir fonctionnelle (Cell., réf. citée ; J. Clin. Invest., 1997, 99, 2302-2306).

Si les intégrines peuvent servir de voie d'accès pour les infections cellulaires virales, bactériennes ou parasitaires, la dérégulation de leur expression ou de leur activation est associée à de nombreuses pathologies incluant par exemple les maladies cardiovasculaires, les maladies inflammatoires, le cancer, l'ostéoporose, l'arthrite rhumatoïde, le psoriasis, les rétinopathies. Les intégrines participent au développement de ces pathologies en agissant sur l'adhérence et la migration cellulaires, sur la régulation de la différenciation, de la survie et de la prolifération cellulaire, et sur la transduction de divers signaux intracellulaires (Ann. Rep. In Med. Chem., 1996, 31, 191-200).

L'intégrine αᵥβ₃, un des récepteurs de la vitronectine, mais liant aussi la fibronectine, le fibrinogène et la thrombospondine, a été plus particulièrement impliquée dans trois événements pathologiques : la migration des cellules musculaires lisses dans la néointima, au cours de l'athérosclérose et de la resténose après angioplastie, à la surface des ostéoclastes lors de la résorption osseuse, et au cours des phases d'angiogenèse sur les cellules endothéliales (Cardiovasc. Res., 1994, 28, 1815-1820 ; J. Clin. Invest., 1997, 99, 2059 ; Science, 1994, 264, 569-571 ; Cell., 1994, 79, 1157-1164 ; Proc. Natl. Acad. Sci. USA, 1996, 93, 9764-9769). Les cellules tumorales utilisent aussi cette intégrine au cours de leur phase invasive, notamment pour les mélanomes, et pour assurer leur survie en contact avec la matrice extracellulaire (Proc. Natl. Acad. Sci. USA, 1992, 89, 1557-1561 et 1994, 91, 8856-8860).

L'intégrine αᵥβ₅, un autre récepteur de la vitronectine, est aussi associée à l'angiogenèse en complément de l'intégrine αᵥβ₃, les deux intégrines participant à deux voies distinctes d'induction de l'angiogenèse (Science, 1995, 270, 1500-1502).

Enfin l'intégrine α_{IIb}β₃ ou GPIIb/IIIa est un récepteur du fibrinogène, et est responsable de l'agrégation des plaquettes.

Le blocage de l'interaction des intégrines αᵥβ₃ et αᵥβ₅ avec leurs ligands est donc susceptible d'inhiber l'adhérence, la migration et la survie de divers types cellulaires, des effets contribuant à bloquer l'angiogenèse, l'inflammation, la résorption osseuse, la resténose, les métastases et la croissance tumorale.

Les composés de l'invention possèdent une structure originale qui leur confère un caractère antagoniste des récepteurs αᵥβ₃ et αᵥβ₅ de la vitronectine, et une sélectivité par rapport aux intégrines α_{IIb}β₃. Ils pourront donc être utiles dans le traitement de pathologies caractérisées par la dérégulation de l'expression ou de l'activation des intégrines αᵥβ₃ et/ou αᵥβ₅, tout en évitant les effets secondaires au niveau de l'agrégation des plaquettes. En particulier ils pourront être utiles dans le traitement des maladies cardiovasculaires, de l'inflammation, du cancer, de l'ostéoporose, de l'arthrite rhumatoïde, du psoriasis et des rétinopathies.

De façon avantageuse les composés de l'invention seront utiles en tant qu'inhibiteurs de croissance tumorale et de formation de métastases, dans le traitement du cancer.

La présente invention concerne les composés de formule (I) : 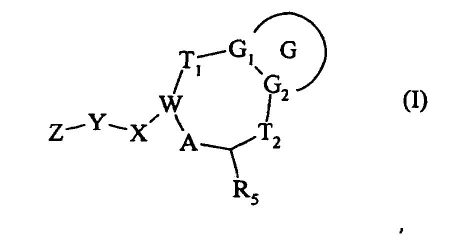 dans laquelle :
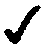 G représente un groupement phényle,
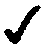 G₁ et G₂ représentent un atome de carbone,
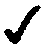 -T₁- représente un groupement choisi parmi -CH₂-CH₂-, -CH=CH-, =CH-CH₂-, et -T₂- représente une liaison,
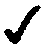 R₅ représente un groupement -(CH₂)ₘ-COOR₆,
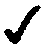 R₆ représente un atome d'hydrogène, un groupement alkyle, aryle éventuellement substitué, ou arylalkyle éventuellement substitué,
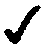 -W- représente un groupement -CH-, =C- ou -C= et -A- représente un groupement -CH₂-,ou =CH-,
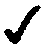 -X- représente un groupement choisi parmi -CO-X₁-, -CO-NR₆-X₁-, -NR₆-CO-X₁-, -O-X₁-, -SO₂-NR₆-X₁-, et -S(O)ₙ-X₁- dans lesquels n est compris inclusivement entre 0 et 2, et X₁ représente un groupement alkylène,
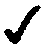 -Y- représente un groupement choisi parmi -Y₁-, -Y₂-Y₁- et -Y₁-Y₂-Y₁-, dans lesquels Y₁ représente un groupement alkylène, alkénylène ou alkynylène, et Y₂ représente un groupement arylène, hétéroarylène, cycloalkylène, ou hétérocycloalkylène,
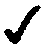 Z- représente un groupement choisi parmi Z₁-, Z₁₀-NR₆-, et Z₁₀-NR₆-CO- dans lesquels Z₁₀ représente un groupement alkyle ou Z₁, et Z₁ représente un groupement choisi parmi Z₂-, 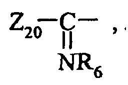 Z₂₀-NR₆-, et Z₂₀-NR₆-CO-, dans lesquels Z₂₀ représente un groupement alkyle, hétéroalkyle, ou Z₂, et Z₂ représente un groupement hétéroaryle éventuellement substitué, hétérocycloalkyle éventuellement substitué, hétéroarylalkyle éventuellement substitué, hétérocycloalkylalkyle éventuellement substitué, arylhétéroaryle fusionné éventuellement substitué, arylhétérocycloalkyle fusionné éventuellement substitué, hétéroarylhétérocycloalkyle fusionné éventuellement substitué, hétérocycloalkylhétéroaryle fusionné éventuellement substitué, hétéroarylhétéroaryle fusionné éventuellement substitué, ou cycloalkylhétérocycloalkyle fusionné,
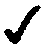 m est un entier compris inclusivement entre 1 et 6,
étant entendu que :
- le terme alkyle désigne un groupement linéaire ou ramifié, contenant de 1 à 6 atomes de carbone,
- le terme hétéroalkyle désigne un groupement alkyle dont un atome de carbone a été remplacé par un hétéroatome choisi parmi azote, oxygène et soufre,
- le terme alkylène désigne un groupement bivalent linéaire ou ramifié, contenant de 1 à 6 atomes de carbone,
- le terme alkénylène désigne un groupement bivalent linéaire ou ramifié, contenant de 2 à 6 atomes de carbone et de 1 à 3 doubles liaisons,
- le terme alkynylène désigne un groupement bivalent linéaire ou ramifié, contenant de 2 à 6 atomes de carbone et de 1 à 3 triples liaisons,
- le terme cycloalkyle désigne un groupement cyclique saturé contenant de 3 à 8 atomes de carbone,
- le terme cycloalkylène désigne un groupement bivalent cyclique saturé contenant de 3 à 8 atomes de carbone,
- le terme hétérocycloalkyle désigne un groupement cyclique saturé de 5 à 7 chaînons contenant de 1 à 3 hétéroatomes choisis parmi azote, oxygène et soufre,
- le terme aryle désigne un groupement phényle ou naphtyle,
- le terme hétéroaryle désigne un groupement mono ou bicyclique, insaturé ou partiellement insaturé, de 5 à 11 chaînons, contenant de 1 à 5 hétéroatomes choisis parmi azote, oxygène et soufre,
- le terme arylhétéroaryle fusionné représente un groupement polycyclique constitué d'un groupement aryle et d'un groupement hétéroaryle tels que définis précédemment et accolés par l'une quelconque de leur liaison,
- le terme arylhétérocycloalkyle fusionné représente un groupement bi- ou tricyclique constitué d'un groupement aryle et d'un groupement hétérocycloalkyle tels que définis précédemment et accolés par l'une quelconque de leur liaison,
- le terme hétéroarylhétérocycloalkyle fusionné représente un groupement bi- ou tricyclique constitué d'un groupement hétéroaryle et d'un groupement hétérocycloalkyle tels que définis précédemment et accolés par l'une quelconque de leur liaison,
- le terme hétérocycloalkylhétéroaryle fusionné représente un groupement bi- ou tricyclique constitué d'un groupement hétéroaryle et d'un groupement hétérocycloalkyle tels que définis précédemment et accolés par l'une quelconque de leur liaison,
- le terme hétéroarylhétéroaryle fusionné représente un groupement polycyclique constitué de deux groupements hétéroaryles tels que définis précédemment et accolés par l'une quelconque de leur liaison,
- le terme cycloalkylhétérocycloalkyle fusionné représente un groupement bicyclique constitué d'un groupement cycloalkyle et du groupement hétérocycloalkyle tels que définis précédemment et accolés par l'une quelconque de leur liaison,
- la terminaison "-ène" signifie que le groupement concerné est un radical bivalent possédant la même définition que le radical de base,
- l'expression "éventuellement substitué" associée aux groupements hétérocycloalkyle, aryle, arylalkyle, hétéroaryle, arylhétéroaryle fusionné, hétéroarylhétérocycloalkyle fusionné, hétéroarylhétéroaryle fusionné, et arylhétérocycloalkyle fusionné, signifie que ces groupements sont non substitués ou substitués par un ou plusieurs atomes d'halogène ou groupements alkyle, alkoxy, hydroxy, mercapto, cyano, amino (éventuellement substitué par un ou deux groupements alkyle), nitro, carboxy, alkoxycarbonyle, aminocarbonyle (éventuellement substitué par un ou deux groupements alkyle), étant entendu que les groupements hétéroaryle et hétérocycloalkyle peuvent en plus être substitués par un groupement oxo,
leurs énantiomères, diastéréoisomères, ainsi que leurs sels d'addition à un acide ou à une base pharmaceutiquement acceptable.

Le groupement R₅ préféré de l'invention est le groupement -CH₂-COOR₆, R₆ étant préférentiellement un groupement alkyle ou un atome d'hydrogène.

Des composés préférés de formule (I) sont ceux pour lesquels T₁ représente un groupement -CH=CH-.

Dans les composés de formule (I), X sera avantageusement choisi parmi -CO-NR₆-X₁, -NR₆-CO-X₁ et -O-X₁, R₆ étant plus particulièrement un atome d'hydrogène, et X₁ représentant préférentiellement un groupement méthylène.

Dans les composés préférés de formule (I), Y représente un groupement Y₁ ou Y₁-Y₂-Y₁ dans lesquels Y₁ est plus particulièrement un groupement alkylène, et Y₂ représente avantageusement un groupement arylène. Plus préférentiellement, Y représente un groupement -(CH₂)₃-.

Les composés préférés de l'invention sont les composés de formule (I) pour lesquels Z représente un groupement hétéroaryle, hétérocycloalkyle, arylhétéroaryle fusionné, hétérocycloalkylhétéroaryle fusionné ou -Z₁₀-NR₆, R₆ étant plus particulièrement un atome d'hydrogène, et Z₁₀ étant avantageusement choisi parmi les groupements hétéroaryle, hétérocycloalkyle, arylhétéroaryle fusionné, et hétérocycloalkylhétéroaryle fusionné. Les groupements cycliques ainsi préférés pour Z comportent avantageusement un ou deux atomes d'azote, comme par exemple les groupements pyridine, aminopyridine, (dihydro)pyrrolopyridine, (dihydro)imidazole, 5,6,7,8-tétrahydro[1,8]naphtyridine ou (tétrahydro)pyrimidine.

Un aspect particulièrement avantageux de l'invention concerne les composés de formule (I) pour lesquels G représente un groupement phényle, G₁ et G₂ étant chacun un atome de carbone, R₅ représente un groupement -CH₂-COOR₆, R₆ étant choisi parmi un atome d'hydrogène et un groupement alkyle, T₂ représente une liaison, T₁ représente un groupement choisi parmi -CH=CH-, et =CH-CH₂-, A représente un groupement -CH₂-, ou =CH- et W représente un groupement =C- ou -C=. Parmi ceux-ci, on préférera les composés pour lesquels X est choisi parmi les groupements -CO-NR₆-X₁-, -NR₆-CO-X₁-, et -O-X₁-, X₁ étant un groupement méthylène, Y représente un groupement -Y₁- ou -Y₁-Y₂-Y₁ dans lesquels Y₁ est un groupement alkylène et Y₂ représente un groupement arylène, et Z représente un groupement hétéroaryle, hétérocycloalkyle, arylhétéroaryle fusionné, hétérocycloalkylhétéroaryle fusionné ou Z₁₀-NR₆ dans lequel Z₁₀ représente un groupement choisi parmi les groupements hétéroaryle, hétérocycloalkyle, arylhétéroaryle fusionné et hétérocycloalkylhétéroaryle fusionné, R₆ représentant un atome d'hydrogène.

Le groupement aryle préféré de l'invention est le groupement phényle.

Encore plus préférentiellement, l'invention concerne les composés de formule (I) qui sont :
- l'acide [7-({[4-(2-pyridinylamino)butanoyl]amino}méthyl)-6,9-dihydm-5*H*-benzo[a] cycloheptèn-5-yl]acétique, chlorhydrate
- l'acide [7-({[5-(2-pyridinylamino)pentanoyl]amino}méthyl)-6,9-dihydro-5*H*-benzo [a]cycloheptèn-5-yl]acétique, chlorhydrate
- l'acide [7-({[4-(4,5-dihydro-1*H*-imidazol-2-ylamino)butanoyl]amino}méthyl)-6,9-dihydro-5*H*-benzo[a]cycloheptèn-5-yl]acétique, chlorhydrate
- l'acide [7-({[4-(1,4,5,6-tétrahydro-2-pyrimidinylamino)butanoyl]amino}méthyl)-6,9-dihydro-5*H*-benzo[a]cycloheptèn-5-yl]acétique, chlorhydrate
- l'acide [7-({[4-(5,6,7,8-tétrahydro[1,8]naphtyridin-2-yl)butanoyl]amino}méthyl)-6,9-dihydro-5*H*-benzo[a]cycloheptèn-5-yl]acétique, chlorhydrate
- l'acide [7-(2-oxo-2-{3-(2-pyridinylamino)propyl]amino}éthyl)-5*H*-benzo[a] cycloheptèn-5-yl]acétique
- l'acide [7-({[4-(2-pyridinylamino)butanoyl]amino}méthyl)-5*H*-benzo[a]cycloheptèn-5-yl]acétique
- l'acide [7-({[4-(1*H*-benzimidazol-2-ylamino)butanoyl]amino}méthyl)-5*H*-benzo[a] cycloheptèn-5-yl]acétique
- l'acide [7-({[4-(1*H*-benzimidazol-2-ylamino)butanoyl]amino}méthyl)-6,9-dihydro-5*H*-benzo[a]cycloheptèn-5-yl]acétique

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer l'hydroxyde de sodium, l'hydroxyde de potassium, la triéthylamine, la tertbutylamine, etc...

La présente invention concerne également le procédé de préparation des composés de formule (I).

Le procédé de préparation des composés de formule (I) est caractérisé :
- en ce que l'on utilise comme produit de départ un composé de formule (II/a) : 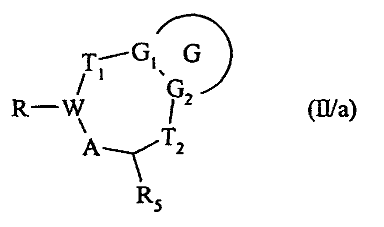 dans laquelle G, G₁, G₂, T₁, T₂ et R₅ ont la même signification que dans la formule (I), W et A sont tels que définis précédemment, et R représente un groupement CHO, CN ou AlkOOC-CH=,
qui lorsque R représente un groupement formyle, est soumis à une réaction de Wittig ou d'Homer Emons de façon à homologuer la chaîne portant la fonction aldéhyde, en utilisant le réactif approprié, pour conduire au composé de formule (III/a) : 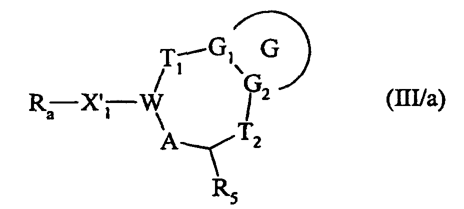 dans laquelle G, G₁, G₂, T₁, T₂, R₅, A et W sont tels que définis précédemment, X'₁ représente un groupement alkylène ou alkénylène de deux à six atomes de carbone et Rₐ représente un groupement cyano, formyle, ou alkoxycarbonyle en fonction du réactif choisi,
composé (III/a) qui, avec les composés de formule (III/b) : 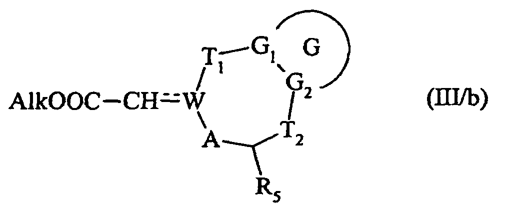 cas particulier des composés de formule (II/a) pour lequel G, G₁, G₂, T₁, T₂, R₅, W et A sont tels que définis précédemment, et Alk représente un groupement alkyle, les pointillés indiquant la présence éventuelle d'une double liaison,
forment l'ensemble des composés de formule (III) : 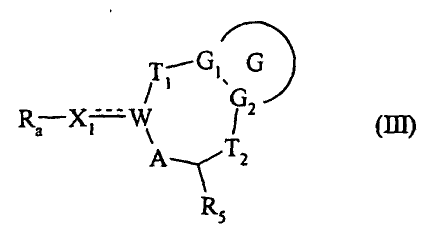 dans laquelle G, G₁, G₂, T₁, T₂, W, A et Rₐ sont tels que définis précédemment, et X₁ a la même signification que dans la formule (I),
composé (III) dont le groupement Rₐ est soit réduit en alcool, ou déprotégé lorsqu'il représente un groupement hydroxy masqué, pour conduire au composé de formule (IV) : 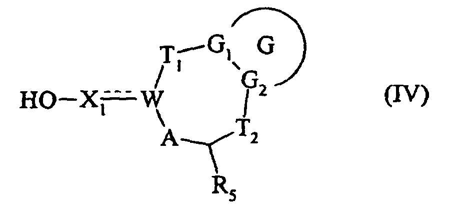 dans laquelle G, G₁, G₂, T₁, T₂, R₅, W, A et X₁ sont tels que définis dans la formule (I),
qui est soumis à une réaction d'halogénation, pour conduire au composé de formule (V) : 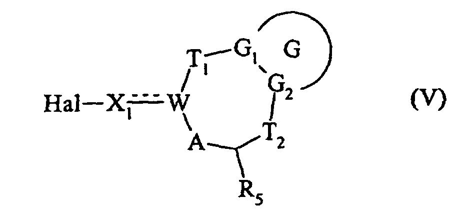 dans laquelle G, G₁, G₂, T₁, T₂, R₅, A, W et X₁ sont tels que définis précédemment, et Hal représente un atome d'halogène,
qui est traité, en milieu basique, par un composé de formule Z-Y-OH dans laquelle Z et Y ont la même signification que dans la formule (I), pour conduire au composé de formule (I/a) : 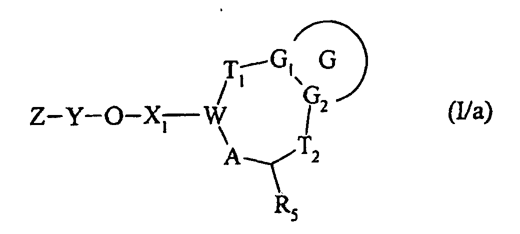 cas particulier des composés de formule (I) pour lequel G, G₁, G₂, T₁, T₂, R₅, X₁, W, A, Y et Z sont tels que définis dans la formule (I),
ou bien qui est traité, en milieu basique, par un composé de formule Z-Y-SH, dans laquelle Z et Y ont la même signification que dans la formule (I), pour conduire au composé de formule (I/b) : 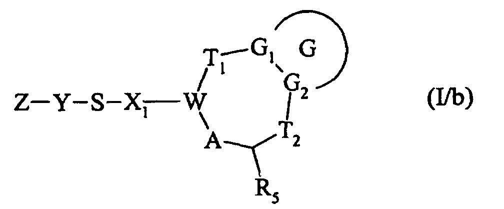 cas particulier des composés de formule (I) pour lequel G, G₁, G₂, T₁, T₂, R₅, X₁, W, A, Y et Z sont tels que définis dans la formule (I),
dont l'atome de soufre peut être oxydé pour conduire un composé de formule (I/c) : 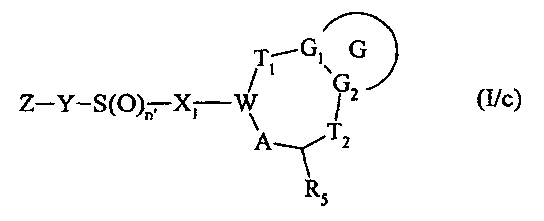 cas particulier des composés de formule (I) pour lequel G, G₁, G₂, T₁, T₂, R₅, X₁, W, A, Y et Z sont tels que définis dans la formule (I), et n' représente un entier égal à 1 ou 2,
ou bien,
composé de formule (V), qui soumis à l'action d'une imide cyclique en milieu basique, suivi d'un traitement par l'hydrazine en milieu alcoolique, conduit à l'amine de formule (VI) : 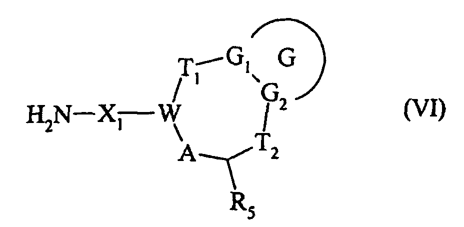 dans laquelle G, G₁, G₂, T₁, T₂, R₅, X₁, A et W sont tels que définis précédemment,
composé de formule (VI) pouvant par ailleurs être obtenu directement lorsque X₁ représente un groupement CH₂, par réduction du composé de formule (II/a) dans laquelle R représente un groupemnet CN,
composé de formule (VI) qui est traité par un composé de formule (VII) : 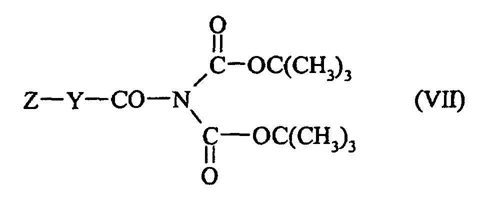 dans laquelle Z et Y sont tels que définis dans la formule (I),
pour conduire au composé de formule (I/d) : 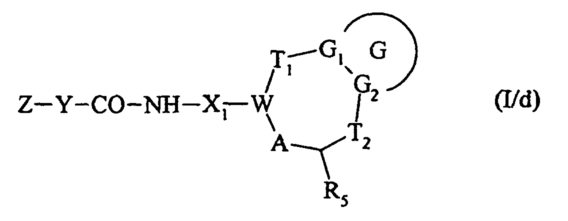 cas particulier des composés de formule (I) pour lequel G, G₁, G₂, T₁, T₂, R₅, X₁, A, W, Z et Y sont tels que définis dans la formule (I),
ou bien qui est traité par un chlorure de sulfonyle de formule Z-Y-SO₂-Cl, dans laquelle Z et Y sont tels que définis dans la formule (I), pour conduire à un composé de formule (I/e) : 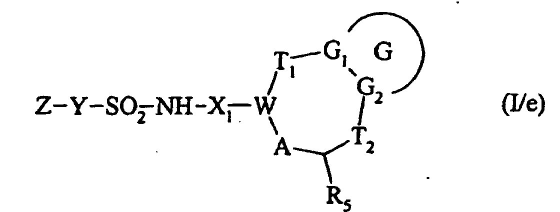 cas particulier des composés de formule (I) pour lequel G, G₁, G₂, T₁, T₂, R₅, X₁, A, W, Z et Y sont tels que définis précédemment dans la formule (I),
ou bien, composé de formule (III), dont le groupement Rₐ est transformé en acide correspondant pour conduire au composé de formule (VIII) : 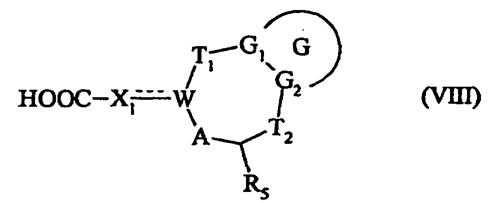 dans laquelle G, G₁, G₂, T₁, T₂, R₅, W, A et X₁ sont tels que définis dans la fomlule (I),
qui est soit soumis à l'action d'une amine de formule Z-Y-NH₂ ou du sel correspondant, pour conduire à un composé de formule (I/f) : 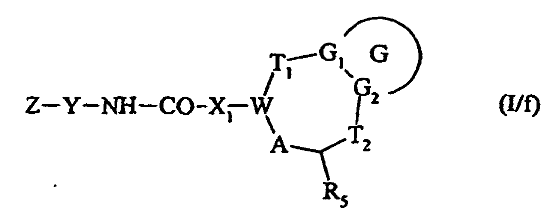 cas particulier des composés de formule (I) pour lequel G, G₁, G₂, T₁, T₂, R₅, A, W, X₁, Z et Y sont tels que définis dans la formule (I),
soit à l'action d'une amine de formule : 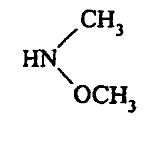 pour conduire au composé de formule (IX) : 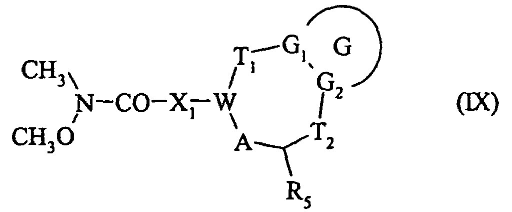 dans laquelle G, G₁, G₂, T₁, T₂, R₅, A, W et X₁ sont tels que définis précédemment,
qui est ensuite soumis à l'action d'un dérivé lithié de formule Z-Y-Li dans laquelle Y et Z sont tels que définis dans la formule (I), pour conduire au composé de formule (I/g) : 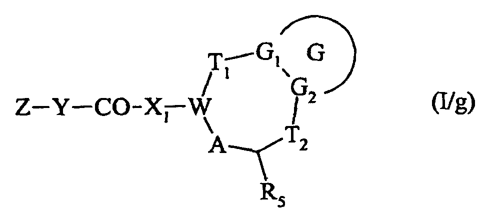 cas particulier des composés de formule (I) pour lequel G, G₁, G₂, T₁, T₂, X₁, W, A, Z et Y sont tels que définis dans la formule (I),
composés (I/a) à (I/g) formant la totalité des composés de formule (I),
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

Une autre variante avantageuse du procédé précédemment décrit, lorsque dans les composés de formule (I) que l'on souhaite obtenir, Z représente un groupement Z₁₀NR₆, consiste à utiliser comme produit de départ un composé de formule (X) : 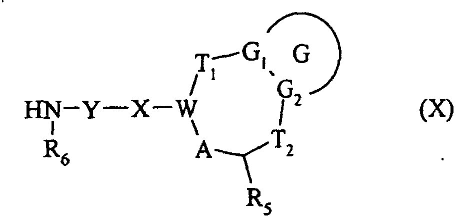 dans laquelle Y, X, W, T₁, G, G₁, G₂, T₂, A, R₅ et R₆ sont tels que définis dans la formule (I),
que l'on condense, en milieu basique ou en présence d'un catalyseur, sur un composé de formule (XI) : 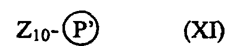 dans laquelle Z₁₀ est tel que défini dans la formule (I) et 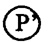 représente un groupe partant (par exemple un atome d'halogène, un groupement tosyle ou un groupement méthylthio ou thioxo)
pour conduire au composé de formule (I/i), cas particulier des composés de formule (I) : 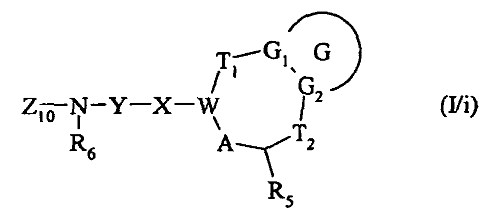 dans laquelle Z₁₀, R₆ Y, X, W, A, T₁, T₂, G₁, G₂, R₅ et G sont tels que définis précédemment,
- qui peut être, le cas échéant, purifié selon une technique classique de purification,
- dont on sépare, le cas échéant, les stéréoisomères selon une technique classique de séparation,
- que l'on transforme, si on le souhaite, en ses sels d'addition à un acide ou à une base pharmaceutiquement acceptable.

Les produits de départ utilisés sont soit connus, soit préparés selon des modes opératoires connus.

En particulier, les composés de formule (II/a) pour lesquels G, G₁ et G₂ forment ensemble un groupement phényle peuvent être préparés de la façon suivante, à partir de la 5,6,8,9-tétrahydro-7*H*-benzocycloheptèn-7-one (2) :

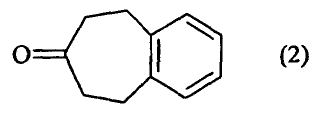

Le composé (2) peut subir une réaction de dihalogénation en position α du carbonyle, pour conduire après une réaction d'élimination en milieu basique au composé insaturé (3) : 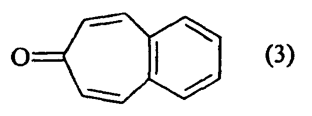 qui est traité en présence de sels de mercure par un cétène acétal de formule (4) : 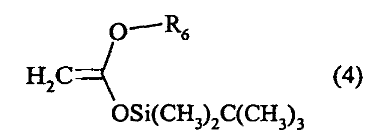 dans laquelle R₆ est tel que défini dans la formule (I),
pour conduire à un composé de formule (5) : 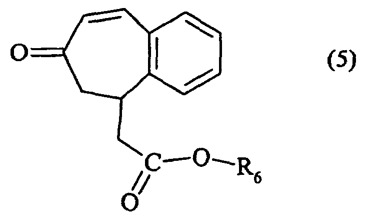 dans laquelle R₆ est tel que défini précédemment,
qui est traité :
- soit par le chlorométhyltriméthylsilane en milieu basique, pour conduire après un réarrangement en présence de silice à un composé de formule (6-II/a) : 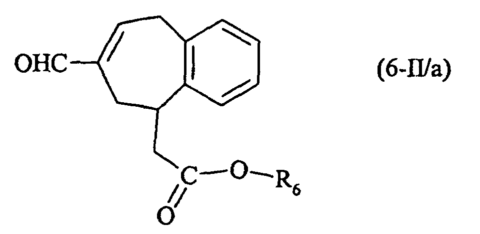 cas particulier des composés de formule (II/a) pour lequel R₆ est tel que défini dans formule (I),
- soit par le méthyllithium, pour conduire après déshydratation au composé (7) : 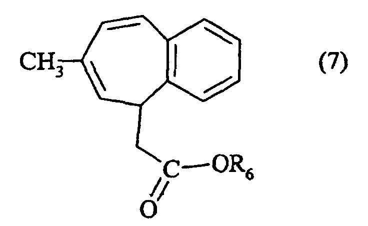 dans laquelle R₆ est tel que défini précédemment,
dont le méthyle est oxydé par un réactif approprié pour conduire à l'aldéhyde correspondant (8-II/a) : 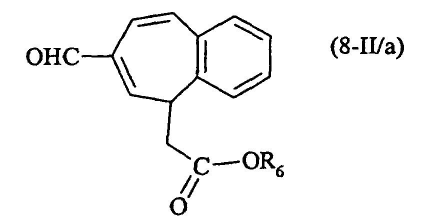 cas particulier des composés de formule (II/a) pour lequel R₆ est tel que défini dans la formule (I).

La cétone (2) telle que précédemment définie peut aussi être traitée par le chlorure de triméthylsilyle en milieu basique fort, pour conduire après oxydation conduit au composé insaturé (9) : 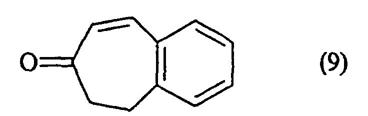 qui est condensé en milieu basique sur un dérivé de formule Hal-(CH₂)ₘ-COOR₆ dans laquelle m et R₆ sont tels que définis dans la formule (I) et Hal représente un atome d'halogène, pour conduire au composé (10) : 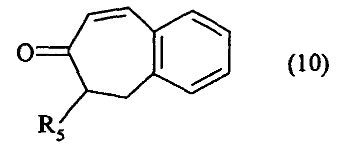 dans laquelle R₅ est tel que défini dans la formule (I),
composé (10) qui est soumis à l'action du monoxyde de carbone en présence de base dans un milieu THF/eau pour conduire au composé (11-II/a) : 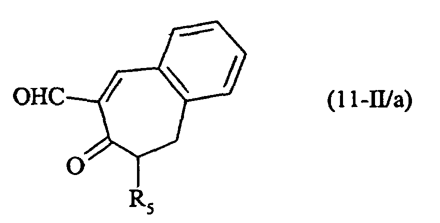 cas particulier des composés de formule (II/a) pour lequel R₅ est tel que défini dans la formule (**I**).

La cétone symétrique (2) peut également être transformée en cétone disymétrique (12) : 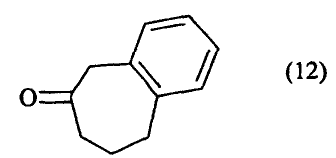 selon le mode opératoire décrit dans J.O.C., 1980, 45, 3028,
composé (12) qui peut subir une réaction de dihalogénation, pour conduire après élimination au composé de formule (13) : 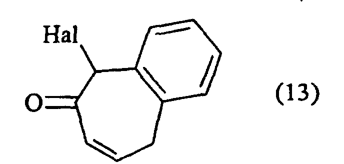 dans laquelle Hal représente un atome d'halogène,
qui est traité :
- soit par un cétène acétal de formule (4) telle que définie précédemment, en présence de sels de mercure, pour conduire à un composé de formule (14) : 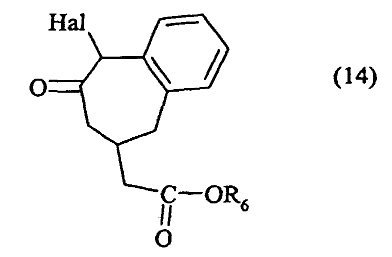 dans laquelle R₆ est tel que défini dans la formule (I) et Hal représente un atome d'halogène,
qui est soumis à une réaction de type Wittig en utilisant le chlorure de (méthoxyméthyl) triphénylphosphonium, pour conduire après traitement en milieu acide au composé de formule (15) : 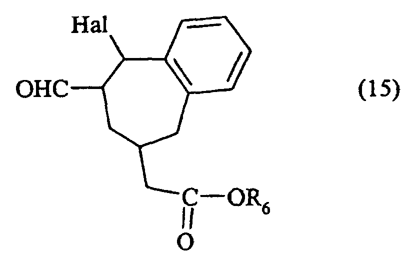 qui après traitement en milieu basique conduit à l'aldéhyde insaturé (16-II/a) : 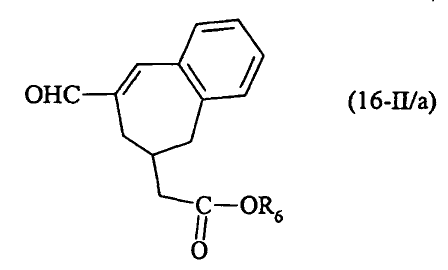 cas particulier des composés de formule (II/a) pour lequel R₆ est tel que défini dans la formule (I).
- soit par un malonate de dialkyle, en milieu basique, pour conduire après chauffage au composé de formule (17) : 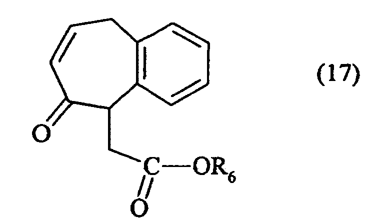 dans laquelle R₆ représente un groupement alkyle,
qui est soumis à l'action du monoxyde de carbone en milieu basique dans un milieu THF/eau, pour conduire à l'aldéhyde de formule (18-II/a) : 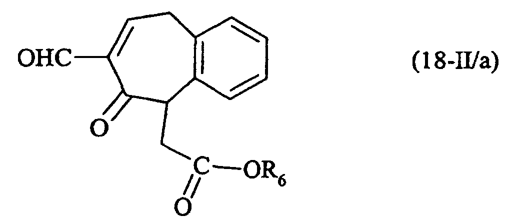 cas particulier des composés de formule (II/a) pour lequel R₆ représente un groupement alkyle.

Le composé (5) tel que défini précédemment, peut être soumis à une réaction de type Wittig à l'aide d'un alkoxyméthylène-triphénylphosphorane pour conduire à un composé de formule (19-II/a): 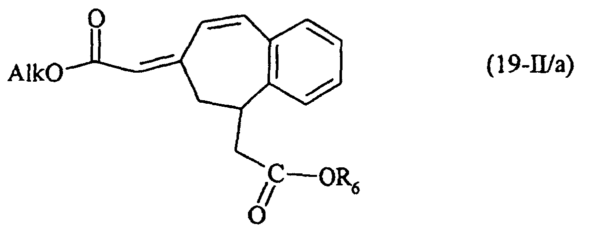 cas particulier des composés de formule (II) pour lequel R₆ est tel que défini dans la formule (I) et Alk représente un groupement alkyle.

La présente invention a également pour objet les compositions pharmaceutiques renfermant comme principe actif au moins un composé de formule (I), seul ou en combinaison avec un ou plusieurs excipients ou véhicules inertes non toxiques, pharmaceutiquement acceptable.

Parmi les compositions pharmaceutiques selon l'invention, on pourra citer plus particulièrement celles qui conviennent pour l'administration orale, parentérale, nasale, transdermique, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques usuelles.

### Préparation 1 : (7-Formyl-6,9-dihydro-5H-benzocycloheptèn-5-yl)-acétate de tert-butyle

### Stade a : 6,8-Dibromo-5,6,8,9-tétrahydro-7H-benzocycloheptèn-7-one

Une solution de brome (17,19 ml ; 333,7 mmol) dans 300 ml de 1,2-dichloroéthane, est ajoutée goutte à goutte à une solution de 5,6,8,9-tétrahydro-7*H*-benzocycloheptèn-7-one dans 500 ml de 1,2-dichloroéthane, à température ambiante. Après l'addition, le milieu réactionnel est porté pendant 3 heures à reflux. Après retour à température ambiante, le mélange est concentré pour conduire au composé attendu.

### Stade b : Benzocycloheptèn-7-one

Le composé obtenu au stade précédent (166,8 mmol) est dissout dans 1 litre de diméthylformamide. On ajoute alors le bromure de lithium (86,93 g ; 1 mol) et le carbonate de lithium (73,96 g ; 1 mol) au milieu réactionnel et on porte à reflux pendant une nuit. Après concentration le résidu est repris dans le dichlorométhane et lavé à l'eau. Les phases organiques sont rassemblées, séchées sur sulfate de magnésium et concentrées. Le résidu obtenu est purifié par chromatographie sur gel de silice (éluant toluène/acétate d'éthyl 95/5) pour conduire au produit attendu.

### Stade c : (7-Oxo-6,7-dihydro-5H-benzocycloheptèn-5-yl)-acétate de tert-butyle

A une solution du composé décrit au stade précédent (14,19 g ; 90,9 mmol) dans 120 ml de dichlorométhane sous agitation à -78°C, on ajoute l'iodure de mercure (10,32 g ; 22,7 mmol). Après 5 minutes d'agitation, le (1-tert-butoxy-vinyloxy)-tert-butyl-diméthylsilane (0,38 ml par mmol de composé ) est ajouté goutte à goutte, en une vingtaine de minutes. L'agitation est maintenue pendant 2 heures à -78°C. Après hydrolyse à l'aide de 150 ml d'une solution aqueuse de chlorure d'ammonium et extraction au dichlorométhane, la phase organique est séchée sur sulfate de magnésium, et concentrée. Le résidu obtenu est purifié par chromatographie sur gel de silice (éther de pétrole/acétate d'éthyle 95/5) pour conduire au composé attendu.

### Stade d : (7-Formyl-6,9-dihydro-5H-benzocycloheptèn-5-yl)-acétate de tert-butyle

A une solution de chlorométhyltrirnétylsilane (14,6 g ; 119 mmol ; 16,6 ml) dans 180 ml de tétrahydrofurane sous agitation à -78°C, sous atmosphère anhydre, sont additionnés goutte à goutte successivement 100 ml d'une solution de sec-butyllithium dans le cyclohexane (1,3 M), et la tétraméthyléthylènediamine (14,41 g ; 124 mmol ; 18,8 ml). Après une heure d'agitation à -78°C, une solution du composé décrit au stade précédent (15,4 g ; 56 mmol) dans 30 ml de tétrahydrofurane est ajoutée goutte à goutte. Après 1 heure 30 à froid, on laisse la température revenir à l'ambiante, pendant 1 heure 30. Le milieu est ensuite hydrolysé à l'aide de 800 ml d'une solution aqueuse saturée en chlorure d'ammonium et extrait au diéthyléther. Après séchage sur sulfate de magnésium et concentration de la phase organique, le résidu obtenu est repris dans 250 ml de dichlorométhane et agité en présence de 60 g de gel de silice. Après filtration le filtrat est concentré pour conduire au produit du titre.

### Préparation 2 : [5-(2-tert-Butoxy-2-oxoéthyl)-5,6-dihydro-7H-benzocycloheptèn-7-ylidène]acétate d'éthyle

Le composé décrit au stade c de la préparation 1 (8,45 g ; 31 mmol) est chauffé à reflux dans le tetrahydrofurane en présence de carbéthoxymétnylène triphénylphosphorane (21,7 g ; 62 mmol), sous atmosphère inerte pendant 10 jours. Le milieu réactionnel est concentré et le résidu obtenu est repris dans le minimum de dichlorométhane, puis on y ajoute du pentane. Le précipité formé est filtré et le filtrat est concentré, puis purifié par chromatographie sur gel de silice (éluant dichlorométhane/éther de pétrole 1/1) pour conduire au composé attendu sous forme de mélange d'isomères cis et trans.

### Préparation 3 : [1-(2-tert-Butoxy-2-oxoéthyl)-2-oxo-1,2,4,5-tétrabydro-3H-3-benzazépin-3-yl]acétate de méthyle

### Stade a : 1,3,4,5-Tétrahydro-2H-3-benzazépin-2-one

A une solution de β-tétralone (25 g ; 171 mmol) dans un mélange de 150 ml d'acide acétique glacial, et d'acide sulfurique concentré (33,39g; 342 mmol ; 18,25 ml) préalablement chauffée à 55°C, on ajoute NaN₃ (13,35 g ; 205,2 mmol) par petites portions en maintenant la température du milieu réactionnel inférieur à 65°C. L'agitation est ensuite poursuivie pendant 8 heures, à 70°C. Après retour à température ambiante le milieu réactionnel est versé sur de la glace, et dilué avec l'acétate d'éthyle. Après extraction de la phase organique, séchage sur sulfate de magnésium et concentration, le résidu obtenu est purifié par chromatographie sur gel de silice (éluant acétate d'éthyle) pour conduire au composé attendu.

### Stade b : 3-(Triméthylsilyl)-1,3,4,5-tétrahydro-2H-3-benzazépin-2-one

Le composé décrit au stade précédent (2 g ; 12,4 mmol) est mis en suspension dans 30 ml de pentane anhydre, sous atmosphère inerte à température ambiante. On ajoute la triéthylamine (2,5 g ; 24,8 mmol ; 3,45 ml) puis le chlorotriméthylsilane (2,69 g ; 24,8 mmol; 3,15 ml). Après 4 heures d'agitation à température ambiante, le milieu réactionnel est filtré sous atmosphère inerte, et le filtrat est concentré sous atmosphère inerte pour conduire au produit attendu.

### Stade c : (2-Oxo-2,3,4,5-tétrahydro-1H-3-benzazépin-1-yl)acétate de tert-butyle

Le diisopropylamidure de lithium est généré de façon classique (20 minutes à 0°C) à partir de diisopropylamine (18,6 mmol ; 2,6 ml) et de n-butyllithium (1,6 M dans hexane) (18,6 mmol ; 11,6 ml) dans 30 ml de tétrahydrofurane et le milieu réactionnel est refroidi ensuite à -78°C. Le composé décrit au stade précédent, dilué dans 30 ml de tétrahydrofurane est ajouté goutte à goutte. L'anion est formé en 15 minutes à -20°C, puis le milieu réactionnel est refroidi à nouveau à -78°C. Après 5 minutes d'agitation, on ajoute le bromoacétate de tert-butyle (2,4 g ; 12,4 mmol ; 2 ml), goutte à goutte. Après 30 minutes d'agitation, le milieu réactionnel est hydrolysé avec une solution aqueuse saturée en chlorure de sodium et extrait au diéthyléther. Après filtration, concentration et purification par chromatographie sur gel de silice, on obtient le produit attendu.

### Stade d : [1-(2-tert-Butoxy-2-oxoéthyl)-2-oxo-1,2,4,5-tétrahydro-3H-3-benzazépin-3-yl]acétate de méthyle

A une solution du composé décrit au stade précédent (100 mg ; 0,416 mmol), dans 6 ml tétrahydrofurane anhydre et 300 µl de diméthylformamide, refroidie à 0°C, on ajoute l'hydrure de sodium en dispersion dans l'huile à 60 % (22 mg ; 0,55 mmol). Après 30 minutes d'agitation, on ajoute successivement l'iodure de tétrabutylammonium (16 mg ; 0,04 mmol) et le bromométhylacétate de méthyle (69 mg ; 0,455 mmol; 43 µl). L'agitation est poursuivie 1 heure à 0°C puis le milieu réactionnel est hydrolysé avec une solution aqueuse saturée en chlorure d'ammonium. Après extraction au diéthyléther, la phase organique est séchée sur sulfate de magnésium et concentrée pour conduire au produit du titre.

### Préparation 4 : (7-Formyl-5H-benzocyclobeptèn-5-yl)acétate de tert-butyle

### Stade a : (7-Hydroxy-7-méthyl-6,7-dihydro-5H-benzocycloheptèn-5-yl)acétate de tert-butyle

A une solution du composé décrit au stade c de la préparation 1 (22,7 g ; 83,32 mmol), dans 225 ml de tétrahydrofurane, à -78°C, on ajoute goutte à goutte le méthyllithium (100 ml, 100 mmol) en solution 1M dans le tétrahydrofurane. La réaction est maintenue sous agitation à -78°C pendant 3 heures. Le milieu réactionnel est hydrolysé par 160 ml d'une solution aqueuse saturée en chlorure d'ammonium, et extrait au diéthyléther. La phase organique est séchée sur sulfate de magnésium, concentrée et purifiée par chromatographie sur gel de silice (éluant éther de pétrole / acétate d'éthyle 80/20) pour conduire au produit attendu.

### Stade b : (7-Méthyl-5H-benzocycloheptèn-5-yl)acétate de tert-butyle

A une solution du composé décrit au stade précédent (26,7 g ; 92,6 mmol), dans 165 ml de dichlorométhane refroidie à 0°C, on ajoute successivement la triéthylamine (18,74 g ; 185,2 mmol) et le chlorure de thionyle (11,01 g ; 92,6 mmol). L'agitation est maintenue à 0°C pendant 10 minutes. Le milieu réactionnel est versé sur de la glace, et extrait au dichlorométhane. La phase organique est séchée sur sulfate de magnésium et concentrée pour conduire au produit attendu.

### Stade c : (7-Formyl-5H-benzocycloheptèn-5-yl)acétate de tert-butyle

Le composé obtenu au stade précédent (23,7 g ; 90,25 mmol) est dissout dans un mélange dioxane/eau (250/4) à température ambiante. On ajoute ensuite le dioxyde de sélénium (28,4 g ; 256 mmol) et le milieu réactionnel est porté à reflux pendant 1 heure. Après refroidissement et filtration sur célite, le filtrat est concentré et purifié par chromatographie sur gel de silice (éluant : éther de pétrole / dichlorométhane 50/50) pour conduire au produit du titre.

### Préparation 5 : (7-{[(4-Aminobutanoyl)amino]méthyl}-6,9-dihydro-5H-benzo[a] cycloheptèn-5-yl)acétate de tert-butyle

### Stade a : (7-{[(4-Bromobutanoyl)amino]méthyl}-6,9-dihydro-5H-benzo[a] cycloheptèn-5-yl)acétate de tert-butyle

A une solution du composé obtenu au stade b de l'exemple 23 (4 g ; 13,9 mmol) en solution dans 45 ml de CH₂Cl₂ sous atmosphère d'argon, sous agitation à 0°C, on additionne successivement goutte à goutte Et₃N (1,4 g; 13,9 mmol; 1,93 ml) puis le chlorure de 4-bromobutanoyle (2,58 g ; 13,9 mmol ; 1,61 ml). Le milieu réactionnel est agité pendant 45 minutes à 0°C, puis est hydrolysé. Après extraction au dichlorométhane, séchage et concentration, on obtient le produit du titre sous forme d'une huile visqueuse jaune utilisée telle quelle pour la suite.

### Stade b : (7-{[(4-Azidobutanoyl)amino]méthyl}-6,9-dihydro-5H-benzo[a] cycloheptèn-5-yl)acétate de tert-butyle

A une solution du composé obtenu au stade a, dans 190 ml de DMF, sous agitation sous atmosphère d'argon, à température ambiante, on ajoute l'azoture de sodium (1,8 g ; 27,8 mol) en une portion. Le milieu réactionnel est ensuite chauffé à 80°C pendant 6 heures puis laissé reposer la nuit à température ambiante. Après concentration, on obtient un liquide brun correspondant au produit du titre, utilisé tel quel pour la suite.

### Stade c : (7-{[(4-Aminobutanoyl)amino]méthyl}-6,9-dihydro-5H-benzo[a] cycloheptèn-5-yl)acétate de tert-butyle

A une solution du composé obtenu au stade b (13,9 mmol) sous agitation à température ambiante dans 50 ml de THF, on ajoute la triphénylphosphine (5,47 g ; 20,85 mmol) en une portion. Après 3 heures d'agitation, on ajoute H₂O au milieu réactionnel (3,3 ml) et l'agitation est poursuivie 10 heures à température ambiante. Après concentration et purification sur gel de silice du résidu obtenu (éluant : CH₂Cl₂/EtOH/NH₄OH 95/5/0,5), on obtient le produit du titre sous forme d'une huile jaune.

### Préparation 6 : [4-(Aminométhyl)-2,3-dihydro-1H-1-benzazépin-2-yl]acétate de méthyle

### Stade a : 3-[2-(Hydroxyméthyl)anilino]pentanedioate de diméthyle

A une solution de 3-oxopentanedioate de diméthyle (5 g ; 28,71 mmol ; 4,2 ml) dans le dichloroéthane (125 ml) à 0°C, on ajoute successivement le (2-aminophényl)rnéthanol (2,95 g ; 23,93 mol), puis NaBH(OAc)₃ (6 g ; 28,71 mol) par petites portions. Le milieu réactionnel est ensuite agité pendant 12 heures à température ambiante puis concentré à sec. Le résidu est ensuite chromatographié sur silice (éluant : heptane/EtOAc) pour conduire au produit du titre.

### Stade b : 3-[2-(Bromométhyl)(trifluoroacétyl)anilino]pentanedioate de diméthyle

Une solution du composé obtenu au stade a (3,15 g ; 11,22 mmol), de Et₃N (16,73 g ; 16,53 mol ; 23 ml) et d'Et₂O (41 ml) est refroidie à 0°C. On y ajoute goutte à goutte une solution d'anhydride trifluoroacétique (2,7 g : 13 mol; 1,8 ml) dans 2 ml d'Et₂O et le milieu réactionnel est agité une heure à 0°C.
Le milieu réactionnel est alors lavé avec une solution d'H₂SO₄ (0,1 N), puis H₂O. Après séchage et concentration sous pression réduite, le composé obtenu est remis en solution dans un mélange CH₂Cl₂ (15,5 ml)/Et₂O (21,5 ml), puis on ajoute PBr₃ (9 g ; 3,36 mol) à 0°C. Le milieu réactionnel est ensuite agité quelques minutes à température ambiante puis à reflux pendant 30 minutes puis versé sur un mélange Et₂O/glace. La phase Et₂O est extraite et lavée avec une solution aqueuse saturée en NaCl. Après concentration et séchage ; on obtient un résidu que l'on chromatographie sur gel de silice, (éluant : heptane/Et₂O) pour obtenir le produit du titre.

### Stade c : 3-[2-(Bromométhyl)(trifluoroacétyl)anilino]-5-chloro-5-oxopentanoate de méthyle

Le composé obtenu au stade b (3,34 g ; 76 mmol) est dilué dans 5,5 ml d'un mélange 1/1 dioxane/eau. On ajoute l'hydroxyde de lithium (0,319 g ; 7,6 mmol), et la réaction est agitée à température ambiante pendant quelques heures. Le milieu réactionnel est alors extrait une fois au diéthylether. La phase aqueuse est alors acidifiée avec HCl (0,1 N) et extraite au dichlorométhane. La phase organique est concentrée et le résidu obtenu est dilué dans 10 ml de toluène et traité avec SOCl₂ (0,97 g ; 8,16 mol, 0,595 ml). Le milieu réactionnel est agité à 70°C pendant 2 heures puis concentré à sec et évaporé 3 fois avec du cyclohexane. Le résidu obtenu, correspondant au produit du titre, est utilisé tel quel par la suite.

### Stade d : [4-Oxo-1-(trifluoroacétyl)-2,3,4,5-tétrahydro-1H-1-benzazépin-2-yl] acétate de méthyle

Une solution du composé obtenu au stade c (2,72 g ; 6,12 mmol), dans 50 ml de 1,2-dimétoxyéthane est ajoutée sous atmosphère d'argon, à une suspension de Pd(PPh₃)₂Cl₂ (0,25 g ; 0,306 mmol) (5 mol %) et de poudre de zinc (0,8 g ; 12,24 mmol), et agitée dans 50 ml de DME à température ambiante. Après une heure d'agitation, le milieu réactionnel est filtré sur célite, concentré et chromatographié sur gel de silice pour conduire au produit du titre.

### Stade e : (1-(Trifluoroacétyl)-4-{[(trifluorométhyl)sulfonyl]oxy}-2,3-dihydro-1H-1-benzazépin-2-yl)acétate de méthyle

A une solution de diisopropylamine (0,602 g ; 4,6 mmol ; 811 µl) dans Et₂O (50 ml) à 0°C, on ajoute EtMgBr (4,66 ml), (1 ,0 N dans Et₂O) et le milieu réactionnel est agité à température ambiante pendant 12 heures. Après refroidissement du milieu réactionnel à 0°C, 10,34 mmoles de HMPA sont ajoutées, suivi du composé obtenu au stade d (1,61 g, 4,89 mmol). Le milieu réactionnel est alors agité pendant 6 heures à température ambiante, puis la N-phényltriflimide (1,66 g ; 4,66 mmol) est ajoutée en une portion. Le milieu réactionnel est ensuite agité 15 heures à température ambiante, puis porté à reflux pendant 6 heures. Le milieu réactionnel, après refroidissement, est lavé avec HCl (10 % aq), (2 x 80 ml) puis H₂O (2 x 80 ml), NaOH (10 % aq) (2 x 80 ml) et enfin NaCl (2 x 80 ml). Après séchage, concentration, et purification sur gel de silice (éluant : heptane/EtOAc), on obtient le composé du titre.

### Stade f : [4-Cyano-1-(trifluoroacétyl)-2,3-dihydro-1H-1-benzazépin-2-yl]acétate de méthyle

A un mélange de LiCN (0,5 M dans le DMF) (11,72 ml ; 5,86 mmol), (Ph₃P)₄Pd (237 mg ; 0,205 mmol), 12-crown-4 (36 mg ; 0,205 mmol) sous atmosphère d'argon, on ajoute une solution du composé obtenu au stade e (1,35 g ; 2,93 mmol) dans 16 ml de toluène anhydre. Le milieu réactionnel est agité à température ambiante pendant 6 heures. L'eau (10 ml) est ajoutée au milieu réactionnel, et la phase organique est extraite au diéthyléther. Après séchage et concentration, le résidu obtenu est purifié sur gel de silice (éluant : heptane/EtOAc) pour conduire au produit du titre.

### Stade g : [4-(Aminométhyl)-2,3-dihydro-1H-1-benzazépin-2-yl]acétate de méthyle

A une solution du composé obtenu au stade f (0,792 g ; 2,34 mmol) dans du dichloroéthane (10 ml), on ajoute nBu₄NBH₄ (0,602 g ; 2,34 mmol) à température ambiante. Le milieu réactionnel est ensuite chauffé à 45°C pendant 8 heures. On ajoute ensuite 10 ml de HCl (10 %) et on chauffe pendant 1 heure à 50°C. Après concentration, le résidu est chromatographié sur silice (CH₂Cl₂/EtOH/NH₄OH) pour conduire au composé du titre.

### Préparation A : 2-[(3-Aminopropyl)amino]pyridine, dichlorhydrate

### Stade a : N-tert-Butoxycarbonyl-1,3-propanediamine

A une solution de 1,3-propanediamine (78,13 g ; 1 mol ; 85 ml) dans 500 ml de dichlorométhane sous agitation à 0°C, on additionne goutte à goutte une solution de BOC₂O (21,825 g ; 0,1 mol ; 23 ml) dans 100 ml de dichlorométhane. Le milieu réactionnel est ensuite agité 30 minutes à température ambiante puis concentré. Le résidu obtenu est repris dans l'eau, filtré, et le filtrat est extrait par 2 x 200 ml de dichlorométhane. Les phases organiques sont séchées sur sulfate de magnésium et concentrées pour conduire au produit attendu.

### Stade b : 2{[3-(tert-Butoxycarbonylamino)propyl]amino}pyridine-N-oxyde

Le composé obtenu au stade précédent (9,4 g ; 53,9 mmol) est repris dans 52 ml d'alcool tertamylique et agité en présence d'hydrogénocarbonate de sodium (22,64 g ; 269,5 mmol). La 2-chloropyridine-N-oxyde (10,75 g ; 64,72 mmol) est alors ajoutée lentement au milieu réactionnel et le mélange est porté à reflux pendant 48 heures. Après refroidissement, le milieu réactionnel est dilué dans 100 ml de dichlorométhane puis filtré. Le filtrat est concentré et purifié par chromatographie sur gel de silice (éluant dichlorométhane/éthanol/ammoniac, 90/10/1) pour conduire au produit attendu.

### Stade c : 2-{[(3-tert-Butoxycarbonylamino)propyl]amino}pyridine

A une solution du composé obtenu stade précédent (9 g ; 34 mmol) dans 400 ml d'éthanol, on ajoute sous argon du palladium sur charbon 10 % (4,0 g), ainsi que (32,44 g ; 394 mmol , 40 ml) de cyclohexène. Le mélange est porté à reflux pendant 8 heures. Le milieu réactionnel est alors refroidi, filtré, concentré et purifié par chromatographie sur gel de silice (éluant dichlorométhane/éthanol, 95/5) pour conduire au produit attendu.

### Stade d : 2-[(3-Aminopropyl)amino]pyridine, dichlorhydrate

Le composé décrit au stade précédent (7,4 g ; 27,7 mmol) est dissout dans 275 ml de dichlorométhane et la solution est agitée à 0°C. On y fait buller de l'acide chlorhydrique gazeux pendant 30 min, puis le mélange réactionnel est agité à température ambiante pendant une heure. Le milieu est ensuite dilué à l'aide de 2 litres de diéthyléther et le précipité formé filtré et séché pour conduire au produit du titre.

### Préparation B : 2[(4-Aminobutyl)amino]pyridine, dichlorhydrate

Le produit attendu est obtenu selon le procédé décrit dans la préparation A en remplaçant la 1,3-propanediamine par la 1,4-butanediamine.

### Préparation C : 2-[(2-Aminoéthyl)amino]pyridine, dichlorhydrate

Le produit attendu est obtenu selon le procédé décrit dans la préparation A en remplaçant la 1,3-propanediamine par la 1,2-éthylènediamine.

### Préparation D : 2-[(3-Aminométhyl)benzylamino]pyridine, dichlorhydrate

Le produit attendu est obtenu selon le procédé décrit dans la préparation A en remplaçant la 1,3-propanediamine par la métaxylènediamine.

### Préparation E : 3-[(tert-Butoxycarbonyl)(2-pyridinyl)amino]propanoylimidodicarbonate de di(tert-butyle)

### Stade a : N-oxyde de l'acide 3-(2-pyridinylamino)propionique

Une solution de chlorhydrate de β-alanine (27 g ; 300 mmol) dans 250 ml d'eau est agitée en présence d'hydrogénocarbonate de sodium (65 g ; 770 mmol) à température ambiante. On ajoute ensuite la 2-chloropyridine-N-oxyde (25 g, 151 mol) et le mélange réactionnel est porté à reflux pendant 72 heures. Après refroidissement, le milieu réactionnel est lavé au dichlorométhane. La phase aqueuse est acidifiée par de l'acide chlorhydrique concentré. Le précipité formé est filtré, et le filtrat est concentré puis purifié par chromatographie sur gel de silice (éluant dichlorométhane/éthanol/ammoniac, 90/8/2) pour conduire au produit attendu.

### Stade b : Acide 3-(2-pyridinylamino)propionique

Le composé décrit au stade précédent (2,1 g ; 11,53 mmol) est dilué dans un mélange dioxane (40 ml) et eau (10 ml) et agité en présence de cyclohexène (17,25 g ; 210 mmol ; 14 ml) et de palladium sur charbon 10 % (2 g). Le milieu réactionnel est porté à reflux pendant 3 heures. Après refroidissement, le mélange est filtré, et le filtrat est concentré pour conduire au produit attendu.

### Stade c : 3-(2-Pyridinylamino)propionamide

Le composé décrit au stade précédent (3,9 g ; 23,32 mmol) est dilué dans 400 ml de méthanol et le chlorure d'acétyle (3,66 g ; 46,6 mmol ; 3,3 ml) est ajouté goutte à goutte. Le mélange est chauffé à reflux pendant 2 heures. Le milieu réactionnel est concentré et le résidu est repris dans une solution de méthanol ammoniacal saturée dans un appareil de Parr, que l'on chauffe à 130°C pendant 72 heures. Le mélange est refroidi et concentré pour conduire au produit attendu.

### Stade d : 3-Amino-3-oxopropyl(2-pyridinyl)carbamate de tert-butyle

Le composé obtenu au stade précédent (2,6 g; 15,74 mmol) est agité à température ambiante sous argon dans 15 ml de tert-butanol. Le dicarbonate de di(tert-butyle) (6,87 g; 31,48 mol ; 6,6 ml) est ajouté goutte à goutte et le milieu réactionnel est agité pendant 48 heures. Le mélange est concentré et le résidu obtenu est purifié par chromatographie sur gel de silice (éluant dichlorométhane/éthanol, 95/5) pour conduire au produit attendu.

### Stade e : 3-[(tert-Butoxycarbonyl)(2-pyridinyl)amino]propanoylimidodicarbonate de di(tert-butyle)

A une solution du composé obtenu au stade précédent (500 mg ; 1,88 mmol) dans 5 ml d'acétonitrile agitée sous argon, à température ambiante, le dicarbonate de di(terbutyle) (862 mg ; 395 mmol ; 0,910 ml), et la diméthylaminopyridine (23 mg ; 0,188 mmol) sont successivement ajoutés. Le milieu réactionnel est agité pendant 16 heures à température ambiante, puis concentré. Le résidu obtenu est purifié par chromatographie sur gel de silice (éluant dichlorométhane/acétate d'éthyle, 30/1) pour conduire au produit attendu.

### Préparation F : 4-[(tert-Butoxycarbonyl)(2-pyridinyl)amino]butanoylimidodicarbonate de di(tert-butyle)

Le produit attendu est obtenu selon le procédé décrit dans la préparation E en remplaçant le chlorhydrate de β-alanine par l'acide 4-aminobutyrique.

### Préparation G : 5-[(tert-Butoxycarbouyl)(2-pyridinyl)amino]pentanoylimidodicarbonate de di(tert-butyle)

Le produit attendu est obtenu selon le procédé décrit dans la préparation E en remplaçant le chlorhydrate de β-alanine par l'acide 5-aminovalérique.

### Préparation H : 3-[(2-Pyridinylamino)méthyl]benzoylimidocarbonate de di(tert-butyte)

### Stade a : 3-[(2-Pyridinylamino)méthyl]benzonitrile

A une solution de 3-cyanobenzaldéhyde (10 g ; 63,7 mmol) et de 2-aminopyridine (6 g ; 63,7 mmol) dans 300 ml de 1,2-dichloroéthane, agitée sous atmosphère d'argon, le triacétoxyborohydrure de sodium (23 g ; 108 mmol) est ajouté par petites portions. Après 12 heures d'agitation à température ambiante, le milieu réactionnel est hydrolysé par du méthanol et concentré. Le résidu obtenu est purifié par chromatographie sur gel de silice (éluant éther de pétrole/acétate d'éthyle, 3/1 puis 2/1 et 1/1), pour conduire au produit attendu.

### Stade b : 3-[(2-Pyridinylamino)méthyl]benzamide

Le composé obtenu au stade précédent (2 g ; 9,56 mmol) est dilué dans 6,6 ml d'eau. On y ajoute le chlorure de triméthylsilane (34,3 g ; 315,5 mmol ; 40 ml) goutte à goutte. Le milieu réactionnel est agité 7 jours à température ambiante, puis concentré. Le résidu est repris dans l'acétate d'éthyle et lavé avec une solution aqueuse saturée en hydrogénocarbonate de sodium. Les phases organiques sont séchées sur sulfate de magnésium et concentrées pour conduire au produit attendu.

### Stade c : 3-[(2-Pyridinylamino)méthyl]benzoylimidocarbonate de di(tert-butyle)

Le produit attendu est obtenu selon le procédé décrit dans au stade e de la préparation E en utilisant comme produit de départ le composé décrit au stade précédent.

### Préparation I : 2-[(5-Hydroxy-1-pentyl)amino]pyridine

Le produit attendu est obtenu selon le procédé décrit aux stades b et c de la préparation A en utilisant comme produit de départ le 5-amino-1-pentanol.

### Préparation J : 2-[(4-Hydroxy-1-butyl)amino]pyridine

Le produit attendu est obtenu selon le procédé décrit aux stades b et c de la préparation A en utilisant comme produit de départ le 4-amino-1-butanol.

### Préparation K : 2-[(3-Hydroxy-1-propyl)amino]pyridine

Le produit attendu est obtenu selon le procédé décrit aux stades b et c de la préparation A en utilisant comme produit de départ le 3-amino-1-propanol.

### Préparation L : {3-[(2-Pyridinylamino)méthyl]phényl}méthanol

### Stade a : [3-({[tert-Butyl(diméthyl)silyl]oxy}méthyl)phényl]méthanol

Une solution de 1,3-benzènediméthanot (2 g ; 14,47 mmol) dans 50 ml de diméthylformamide est agitée à température ambiante. L'imidazole (1,97 g ; 28,94 mmol), puis le chlorure de tert-butyl(diméthyl)silane (2,18 g ; 14,47 mmol) sont ajoutés successivement. Après 12 heures d'agitation, le milieu réactionnel est dilué dans l'éther et lavé par une solution aqueuse saturée en chlorure de sodium. Après extraction, la phase éthérée est séchée sur sulfate de magnésium et concentrée pour conduire au composé attendu.

### Stade b : 3-({[tert-Butyl(diméthyl)silyl]oxy}méthyl)benzaldéhyde

Une solution du composé décrit au stade précédent (1,73 g ; 6,85 mmol) dans 20 ml de dichlorométhane est agitée en présence de dioxyde de manganèse activé (6 g ; 69 mmol). Après 24 heures d'agitation à température ambiante, le milieu réactionnel est filtré sur célite, et le filtrat concentré pour conduire au produit attendu.

### Stade c : N-[3-({[tert-Butyl(diméthyl)silyl]oxy}méthyl)benzyl]-2-pyridinamine

Une solution du composé décrit au stade précédent (1,3 g ; 5,23 mmol) dans 21 ml de 1,2-dichloroéthane est agitée à température ambiante. La 2-aminopyridine (492 mg ; 5,23 mmol) puis le tri-acétoxyborohydrure de sodium (1,88 g ; 8,89 mmol) sont ajoutés successivement. Après 12 heures d'agitation à température ambiante, le milieu réactionnel est concentré et le résidu purifié par chromatographie sur gel de silice (éluant : éther de pétrole/acétate d'éthyle 4/1) pour conduire au produit attendu.

### Stade d : {3-[(2-Pyridinylamino)méthyl]phényl}méthanol

Une solution du composé décrit au stade précédent (1,8 g ; 5,49 mmol) dans 27 ml de tétrahydrofurane anhydre est agitée à 0°C. On ajoute du fluorure de tétrabutylammonium (1 M dans le tétrahydrofurane, 3,3 ml; 3,29 mmol) au mélange réactionnel et l'agitation est poursuivie de 0°C à température ambiante pendant 10 heures. Le milieu réactionnel est alors dilué dans l'éther, et lavé avec une solution aqueuse saturée en chlorure de sodium. Après extraction, la phase éthérée est séchée sur sulfate de magnésium, concentrée, et purifiée par chromatographie sur gel de silice (éluant : éther de pétrole/acétate d'éthyle 1/1), pour conduire au produit attendu.

### Préparation M : N1-(4,5,6,7-Tétrahydro-3H-azépin-2-yl)-1,3-propanediamine

### Stade a : 2-(4,5,6,7-Tétrahydro-3H-azépin-2-yl)éthylcarbamate de tert-butyle

A une solution de 2-méthoxy-4,5,6,7-tétrahydro-3*H*-azépine (1,46 g ; 11,48 mmol ; 1,7 ml) dans 4 ml d'éthanol absolu, on ajoute une solution de N-tert-Butoxycarbonyl-1,3-propanediamine (2 g; 11,48 mmol) décrit au stade a de la préparation A, dans 1 ml d'éthanol. Après 8 heures d'agitation à température ambiante, le milieu réactionnel est concentré pour conduire au produit attendu.

### Stade b : N1-(4,5,6,7-Tétrahydro-3H-azépin-2-yl)-1,3-propanediamine

Le produit attendu est obtenu selon le procédé décrit au stade d de la péparation A à partir du composé décrit au stade précédent.

### Préparation N : N1-(4,5-Dihydro-1H-imidazo-2-yl)-1,3-propanediamine

### Stade a : 3-(4,5-Dihydro-1H-imidazo-2-ylamino)propylcarbamate de tert-butyle

A une solution agitée de N-tert-butoxycarbonyl-1,3-propanediamine (8,9 g ; 51 mmol) dans 150 ml de diméthylacétamide, sous atmosphère d'argon, on ajoute l'hydroiodure de 2-méthylthioimidazoline (25 g ; 102 mmol), puis la diisopropylamine (13,18 g ; 102 mmol ; 18 ml), à température ambiante. Le milieu réactionnel est chauffé à 100°C pendant 12 heures. Après refroidissement et concentration, le résidu obtenu est purifié par chromatographie sur gel de silice (éluant acétate d'éthyle /éther de pétrole 4/1) pour conduire au produit désiré.

### Stade b : N1-(4,5-Dihydro-1H-imidazo-2-yl)-1,3-propanediamine

Le produit attendu est obtenu selon le procédé décrit au stade d, de la préparation A, à partir du composé décrit au stade précédent.

### Préparation O : N-[3-(Aminométhyl)benzyl]-4,5-dihydro-1H-imidazol-2-amine

### Stade a : 3-(Aminométhyl)benzylcarbamate de tert-butyle

Le produit attendu est obtenu selon le procédé décrit au stade a, de la préparation A, en utilisant comme produit de départ la métaxylènediamine.

### Stade b : 3-[(4,5-Dihydro-1H-imidazol-2-ylamino)méthyl]benzylcarbamate de tert-butyle

Le produit attendu est obtenu selon le procédé décrit au stade a, de la préparation N, en utilisant comme produit de départ le composé décrit au stade précédent.

### Stade c : N-[3-(Aminométhyl)benzyl]-4,5-dihydro-1H-imidazol-2-amine

Le produit attendu est obtenu selon le procédé décrit au stade d, de la préparation A, en utilisant comme produit de départ le composé décrit au stade précédent.

### Préparation P : Acide N-(4,5,6,7-tétrahydro-3H-azépin-2-yl)aminobutyrique

Le produit attendu est obtenu selon le procédé décrit au stade a, de la préparation M, en remplaçant la N-tert-butoxycarbonyl-1,3-propanediamine par l'acide γ-aminobutyrique.

### Préparation Q : 2-[{4-[bis(tert-Butoxycarbonyl)amino]-4-oxobutyl}(tert-butoxycarbonyl)amino]-1H-benzimidazole-1-carboxylate de tert-butyle

### Stade a : 2-[(tert-Butoxycarbonyl)amino]-1H-benzimidazole-1-carboxylate de tert-butyle

A une solution de aminobenzimidazole (10,66 g ; 80 mmol) dans 200 ml de tert-butanol, sous agitation à température ambiante, on ajoute le BOC₂O (34,8 g ; 160 mmol). L'agitation est maintenue pendant 10 heures. Le précipité formé est filtré, et rincé au pentane pour conduire au produit attendu.

### Stade b : 2-[(4-Amino-4-oxobutyl)(tert-butoxycarbonyl)amino]-1H-benzimidazole-1-carboxylate de tert-butyle

Le composé obtenu au stade précédent (6 g ; 18 mmol) est dissout dans 60 ml de diméthylformamide. Le γ-hydroxybutyramide (930 mg ; 9 mmol) est ajouté à la solution, et le milieu réactionnel est refroidi à 0°C. La triphénylphosphine (4,18 g ;13,5 mmol) puis le DEAD (2,35 g ; 13,5 mmol) sont ajoutés. La réaction est agitée pendant 15 heures, de 0°C à température ambiante. Après concentration, le résidu obtenu est purifié par chromatographie sur gel de silice (éluant dichlorométhane/éthanol 40/1) pour conduire au produit attendu.

### Stade c : 2-[{4-[bis(tert-Butoxycarbonyl)amino]-4-oxobutyl}(tert-butoxycarbonyl)amino]-1H-benzimidazole-1-carboxylate de tert-butyle

Le produit attendu est obtenu selon le procédé décrit dans la préparation E, stade e, à partir du composé décrit au stade précédent.

### Préparation R : Acide 4-[(4,5-dihydro-1H-imidazol-2-yl)amino]butyrique

Le produit attendu est obtenu selon le procédé décrit dans la préparation N en remplaçant la N-tert-butoxycarbonyl-1,3-propanediamine par le 4-aminobutanoate de tert-butyle, suivi d'une hydrolyse selon le procédé décrit au stade d de la préparation A.

### Préparation S : Acide 3-(1,4,5,6-tétrahydro-2-pyrimidinylamino)benzoïque

### Stade a : Acide 3-{[imino(méthylsulfanyl)méthyl]amino}benzoïque

A une solution d'acide 3-[(aminocarbothioyl)amino]benzoïque (25 g ; 127,4 mmol) dans 375 ml de tétrahydrofurane anhydre, sous atmosphère d'argon, on ajoute l'iodure de méthyle (18 g ; 127,4 mmol ; 7,93 ml), à température ambiante, puis le milieu réactionnel est chauffé à 75°C pendant 2 heures. Après refroidissement et concentration le résidu est repris dans l'éther, et le précipité formé est filtré pour conduire au produit attendu.

### Stade b : Acide 3-(1,4,5,6-tétrahydro-2-pyrimidinylamino)benzoïque

A une solution du composé obtenu au stade précédent (10,1 g ; 30 mmol) dans 15 ml de diméthylformamide anhydre, on ajoute la triéthylamine (3,03 g ; 30 mmol ; 4,17 ml), la 4-diméthylaminopyridine (420mg; 3,4 mmol), et le 1,3-diaminopropane (2,22 g ; 30 mmol), et le mélange réactionnel est chauffé à 145°C pendant 5 heures. Après refroidissement à température ambiante, le milieu réactionnel est repris dans l'eau. Le précipité formé est filtré, resolubilisé dans l'eau, puis acidifié par l'acide chlorhydrique à 36 %. Le mélange est concentré pour conduire au produit attendu.

### Préparation T : Acide N-(4,5,6,7-tétrahydro-3H-azépin-2-yl)aminopentanoïque

Le produit attendu est obtenu selon le procédé décrit au stade a de la préparation M en remplaçant la N-tert-butoxycarbonyl-1,3-propanediamine par l'acide 5-aminopentanoïque.

### Préparation U : 2-[{5-[bis(tert-Butoxycarbonyl)amino]-5-oxopentyl}(tert-butoxycarbonyl)amino]-1H-benzimidazole-1-carboxylate de tert butyle

Le produit attendu est obtenu selon le procédé décrit dans la préparation Q, en remplaçant au stade b le γ-hydroxybutyramide par le δ-hydroxypentanamide.

### Préparation V : Acide 5-[(4,5-dibydro-1H-imidazol-2-yl)amino]pentanoïque

Le produit attendu est obtenu selon le procédé décrit dans la préparation N en remplaçant la N-tert-butoxycarbonyl-1,3-propanediamine par le 5-aminopentanoate de tert-butyle, suivi d'une hydrolyse selon le procédé décrit au stade d de la préparation A.

### Préparation W : 2-(Méthylthio)-5,6-dihydro-1(4H)-pyrimidine carboxylate de tert-butyle

A une solution de 2-méthylthio-2-tétrahydropyrimidine iodhydrate (10 g ; 38,7 mmol), sous agitation à température ambiante dans 38 ml de dichlorométhane, on ajoute la triéthylamine (3,92 g ; 38,7 mmol ; 5,54 ml), puis le BOC₂O (9,3 g ; 42,57 mmol). L'agitation est maintenue pendant 10 heures. Le milieu réactionnel est ensuite évaporé à sec puis repris dans du pentane. Le filtrat évaporé conduit au produit du titre sous forme d'une huile incolore.

### Préparation X : 2-(Méthylthio)-4,5-dihydro-1H-imidazole-1-carboxylate de tert-butyle

On procède comme dans la préparation W en remplaçant le 2-méthylthio-2-tétrahydropyrimidine iodidrate par le 2-méthylthio-2-imidazoline iodidrate.

### Préparation Y : 5-Méthoxy-2-thioxo-1H-benzimidazole-1,3(2H)-dicarboxylate de di (tert-butyle)

A une solution de 2-mercapto-5-méthoxybenzimidazole (10 g ; 55,5 mmoles) en solution sous argon dans 400 ml de tétrahydrofurane sous agitation à 10°C, on ajoute, par petites fractions, NaH (60 % dans l'huile) (5,55 g ; 138 mmol). Le milieu réactionnel est ensuite agité 30 minutes à 0°C, puis le BOC₂O (26,64 g ; 122 mmol) est ajouté en une seule fois. L'agitation du milieu réactionnel est alors poursuivie à température ambiante pendant 18 heures. Le milieu réactionnel est dilué avec du méthanol, filtré sur célite puis concentré à sec. Le résidu obtenu est alors repris avec un mélange 1/1 d'éther de pétrole/éther isopropylique. Le solide qui précipite est alors essoré pour conduire au produit du titre sous forme d'une poudre blanche.

### Préparation Z : 3-[(Tert-butoxycarbonyl)(2-pyridinylméthyl)amino]propanoyl imidodicarbonate de di (tert-butyle)

### Stade a : 3-[(2-Pyridinylméthyl)amino]propanamide

A une solution de 2-(aminométhyl)-pyridine (5 g ; 46,24 mmol ; 4,77 ml) dans 19 ml de diméthyl-formamide, sous agitation sous atmosphère d'argon, à température ambiante, on ajoute successivement goutte à goutte l'acrylamide (3,29 g; 46,24 mmol) et la triéthylamine (0,468 g ; 4,624 mmoles ; 662 µl). Le milieu réactionnel est alors porté à 40°C, et agité à cette température pendant 72 heures. En fin de réaction, le milieu réactionnel est concentré et le résidu est purifié par chromatographie sur gel de silice (éluant : CH₂Cl₂/EtOH/NH₄OH 80/20/2) pour conduire au produit du titre sous forme d'une huile jaune.

### Stade b : 3-Amino-3-oxopropyl(2-pyridinylméthyl)carbamate de tert-butyle

Au composé obtenu au stade a (7,6 g ; 42,4 mmol) agité à température ambiante dans 50 ml de tert-butanol, on ajoute BOC₂O (18,5 g ; 84,8 mmol) en une portion. Après 72 heures d'agitation à température ambiante, le milieu réactionnel est concentré et le résidu obtenu purifié via chromatographie sur gel de silice (éluant : CH₂Cl₂/EtOH 95/5) pour conduire au produit du titre.

### Stade c : 3-[(Tert-butoxycarbonyl)(2-pyridinylméthyl)amino]propanoyl imidodicarbonate de di (tert-butyle)

On procède comme dans le stade e de la préparation E à partir du composé obtenu au stade b.

### Préparation A₁ : 2-{[{3-[Bis(tert-butoxycarbonyl)amino]-3-oxopropyl}(tert-butoxycarbonyl)amino]méthyl}-1H-benzimidazole-1-carboaylate de tert-butyle

On procède comme dans la préparation Z en remplaçant la 2-(aminométhyl)-pyridine par le 2-(aminométhyl)benzimidazole.

### Préparation A₂ : Acide 4-(5,6,7,8-tétrahydro[1,8]naphtyridin-2-yl)butanoïque

### Stade a : 4-[1,8]Naphtyridin-2-yl butanoate d'éthyle

Une solution composée de 2-aminonicotinaldéhyde (0,7 g ; 5,8 mmol), de 5-oxohexanoate d'éthyle (1,83 g ; 11,6 mmol ; 1,85 ml), de L-proline (0,166 g ; 1,45 mmol) et de 50 ml d'EtOH est chauffée à 90°C sous agitation pendant 6 heures. Le milieu réactionnel est alors concentré et le résidu est purifié via chromatographie sur gel de silice (éluant : EtOAc) pour conduire au produit du titre sous forme d'un solide beige.

### Stade b : 4-(5,6,7,8-Tétrahydro[1,8]naphtyridin-2yl)butanoate d'éthyle

Le composé obtenu au stade a (0,57 g ; 2,33 mmol) est mis en solution dans 15 ml d'AcOEt et soumis sous agitation, à une atmosphère d'hydrogène, en présence de 57 mg de Pd/C (10 %) pendant 18 heures. Le milieu réactionnel est ensuite filtré sur célite et concentré à sec pour conduire au produit du titre sous forme d'une huile jaune.

### Stade c : Acide 4-(5,6,7,8-tétrahydro[1,8]naphtyridin-2-yl)butanoïque

L'ester obtenu au stade b (0,36 g ; 1,45 mmol) est mis en solution dans 10 ml d'HCl 6N. Le milieu réactionnel est chauffé à 55°C pendant 4 heures. Le milieu réactionnel est ensuite concentré, et le résidu est repris dans AcOEt. Le précipité formé correspondant au produit du titre est essoré sur fritté, et isolé sous forme d'un solide jaune pâle.

### Préparation A₃ : Acide 4-oxo-4(2-pyridinylamino)butanoïque

Le composé 2-aminopyridine (5 g ; 53,02 mmol) est dissout dans 80 ml de THF et chauffé pendant 15 heures à 80°C en présence d'anhydride succinique (5,3 g ; 53,08 mmol) et une quantité catalytique de Et₃N (0,7 ml). On observe un solide blanc qui précipite dans le milieu réactionnel, que l'on essore, correspondant au produit du titre.

### Préparation A₄ : 4-(1,3-Thiazol-2-ylamino)butanoyl imidodicarbonate de di (tert-butyle)

On procède comme dans la préparation Q en remplaçant l'aminobenzimidazole par la 1,3-thiazol-2-ylamine.

### EXEMPLE 1 : [7-({[5-(2-Pyridinylamino)pentyl]oxy}méthyl)-6,9-dihydro-5H-benzo[a]cycloheptèn-5-yl]acétate de tert-butyle

### Stade a : (7-Hydroxyméthyl-6,9-dihydro-5H-benzo[a]cycloheptèn-5-yl)acétate de tert-butyle

A une solution du composé décrit dans la préparation 1 (8,26 g ; 28,84 mmol) dans 1,6 1 d'un mélange méthanol/dichlorométhane (1/1), sous agitation à 0°C, on ajoute le borohydrure de sodium (1,1 g; 28,84 mol) par petites portions. Après 30 minutes d'agitation à 0°C, le milieu réactionnel est concentré, repris dans du dichlorométhane et lavé avec une solution aqueuse saturée en chlorure de sodium. La phase organique est séchée sur sulfate de magnésium, concentrée pour conduire au produit désiré.

### Stade b : (7-Bromométhyl-6,9-dihydro-5H-benzo[a]cycloheptèn-5-yl)acétate de tert-butyle

A une solution de composé décrit au stade précédent (7,5 g, 26 mmol) dans 30 ml de dichloroéthane, agité à 0°C, on ajoute le tétrabromure de carbone (9,05 g ; 27,3 mmol), puis la triphénylphosphine (7,16 g ; 27,3 mmol) en solution dans 35 ml de dichloroéthane. Le milieu réactionnel est agité à 0°C, pendant 30 min puis concentré. Le résidu obtenu est purifié par chromatographie sur gel de silice (éluant éther de pétrole/dichlorométhane, 1/1) pour conduire au composé attendu.

### Stade c : [7-({[5-(2-Pyridinylamino)pentyl]oxy}méthyl)-6,9-dihydro-5H-benzo[a] cycloheptèn-5-yl]acétate de tert-butyle

A une solution du composé décrit au stade précédent (940 mg ; 2,68 mmol) dans 4 ml d'acétonitrile agitée à température ambiante, on ajoute le composé décrit dans la préparation 1 (484 mg ; 2,68 mmol) en solution dans 4 ml d'acétonitrile, puis le carbonate de césium (873 mg ; 2,68 mmol). Le mélange réactionnel est alors chauffé à 40°C pendant 12 heures. L'insoluble est filtré, et le filtrat concentré, puis purifié par chromatographie sur gel de silice (éluant éther de pétrole/diéthyléther 1/1) pour conduire au produit attendu.

### EXEMPLE 2 : Acide [7-({[5-(2-pyridinylamino)pentyl]oxy}méthyl)-6,9-dihydro-5H-benzo[a]cycloheptèn-5-yl]acétique, chlorhydrate

Le composé décrit dans l'exemple 1 (315 mg ; 0,65 mmol) est dilué dans 8 ml de dichlorométhane, et agité à température ambiante. 16 ml d'une solution d'éther chlorhydrique (4N) sont alors ajoutés. Le milieu réactionnel est agité jusqu'à disparition totale de produit de départ vérifiée par chromatographie sur couche mince (éluant dichlorométhane/éthanol 95/5). En fin de réaction, 50 ml d'éther de pétrole sont ajoutés au milieu réactionnel, et le mélange est agité plusieurs heures. Le surnageant est enlevé, et on réitère 3 fois le lavage de la gomme au pentane. La gomme est ensuite reprise dans l'eau et lavée au dichlorométhane. La phase aqueuse est ensuite lyophilisée, et la poudre obtenue est purifiée par chromatographie sur gel de silice (éluant dichlorométhane/éthanol/acide acétique, 98/2/0,7) pour conduire au produit du titre.

| Microanalyse élémentaire : C₂₄H₃₀N₂O₉, HCl | | | |
|---|---|---|---|
| | C | H | N |
| % Calculé | 66,88 | 7,25 | 6,50 |
| % Trouvé | 66,77 | 7,22 | 7,15 |

### EXEMPLE 3: [7-({[4-(2-Pyridinylamino)butyl]oxy}méthyl)-6,9-dihydro-5H-benzo [a]cycloheptèn-5-yl]acétate de tert-butyle

Le produit attendu est obtenu selon le procédé décrit dans l'exemple 1 en remplaçant, au stade c, le produit décrit dans la préparation I par le produit décrit dans la préparation J.

### EXEMPLE 4 : Acide [7-({[4-(2-pyridinylamino)butyl]oxy}méthyl)-6,9-dihydro-5H-benzo[a]cycloheptèn-5-yl]acétique, chlorhydrate

Le produit attendu est obtenu selon le procédé décrit dans l'exemple 2 en utilisant comme produit de départ le composé décrit dans l'exemple 3.

| Microanalyse élémentaire : C₂₃H₂₈N₂O₃, HCl (0,8 mol) | | | |
|---|---|---|---|
| | C | H | N |
| % Calculé | 67,43 | 7,09 | 6,84 |
| % Trouvé | 67,53 | 6,92 | 6,88 |

### EXEMPLE 5 : [7-({[3-(2-Pyridinylamino)propyl]oxy}méthyl)-6,9-dihydro-5H-benzo [a]cycloheptèn-5-yl]acétate de tert-butyle

Le produit attendu est obtenu selon le procédé décrit dans l'exemple 1 en remplaçant, au stade c, le produit décrit dans la préparation I par le produit décrit dans la préparation K.

### EXEMPLE 6: Acide [7-({[3-(2-pyridinylamino)propyl]oxy}méthyl)-6,9-dihydro-5H-benzo[a]cycloheptèn-5-yl]acétique, chlorhydrate

Le produit attendu est obtenu selon le procédé décrit dans l'exemple 2 en utilisant comme produit de départ le composé décrit dans l'exemple 5.

| Microanalyse élémentaire : C₂₂H₂₆N₂O₃, HCl (1,3 mol) | | | |
|---|---|---|---|
| | C | H | N |
| % Calculé | 63,84 | 6,65 | 6,77 |
| % Trouvé | 63,81 | 6,66 | 6,78 |

### EXEMPLE 7 : Acide {7-[({3-[(2-pyridinylamino)méthyl]benzyl}oxy)méthyl]-6,9-dihydro-5H-benzo[a]cycloheptèn-5-yl}acétique, chlorhydrate

Le produit attendu est obtenu selon le procédé décrit dans les exemples 1 et 2 en remplaçant, au stade c de l'exemple 1, le produit décrit dans la préparation I par le produit décrit dans la préparation L.

| Microanalyse élémentaire : C₂₇H₂₈N₂O₃, HCl (1,2 mol) | | | | |
|---|---|---|---|---|
| | C | H | N | Cl |
| % Calculé | 68,66 | 6,23 | 5,93 | 9,0 |
| % Trouvé | 68,51 | 6,04 | 6,11 | 7,73 |

Spectre de masse (ESI) : M+H¹⁺=429

### EXEMPLE 8 : Acide [7-(2-oxo-2-{[3-(2-pyridinylamino)propyl]amino}éthyl)-5H-benzo[a]cycloheptèn-5-yl]acétique, trifluoroacétate

### Stade a : Acide [5-(2-tert-butoxy-2-oxoéthyl)-5,6-dihydro-7H-benzo[a]cycloheptèn-5-ylidène]acétique

Le composé décrit dans la préparation 2 (3 g ; 8,76 mmol) est agité vigoureusement dans 6,4 ml d'un mélange 1/1 dioxane/eau. On ajoute l'hydroxyde de lithium (0,368 g ; 8,76 mmol), et le milieu réactionnel est agité à 40°C pendant 24 heures. Après refroidissement le mélange réactionnel est extrait une fois au diéthyléther. La phase aqueuse est traitée par une solution aqueuse saturée en chlorure d'ammonium et extraite au dichlorométhane. Les phases organiques sont regroupées et concentrées pour conduire au composé attendu.

### Stade b : [7-(2-Oxo-2-{[3-(2-pyridinylamino)propyl]amino}éthyl)-5H-benzo[a] cycloheptèn-5-yl]acétate de tert-butyle

A une solution de composé décrit au stade précédent (1 g ; 3,82 mmol) dans 25 ml de diméthylformamide anhydre, on ajoute successivement le produit décrit dans la préparation A (0,856 g; 3,82 mmol), l'hydroxybenzotriazole monohydrate (0,516 g; 3,82 mmol), la diisopropylamine (2,06 g; 15,9 mmol ; 2,77 ml) et enfin le chlorhydrate de N-ethyl-N'-3-diméthylaminopropylcarbodiimide (0,732 g ; 3,82 mmol), à température ambiante, le milieu réactionnel est agité pendant 24 heures. Après concentration, le résidu est repris dans le dichlorométhane et lavé avec une solution aqueuse saturée en hydrogénocarbonate de sodium. La phase organique est séchée sur sulfate de magnésium, concentrée et purifiée par chromatographie sur gel de silice (éluant dichlorométhane/éthanol 40/1) pour conduire au produit attendu.

### Stade c : Acide [7-(2-oxo-2-{[3-(2-pyridinylamino)propyl]amino}éthyl)-5H-benzo [a]cycloheptèn-5-yl]acétique, trifluoroacétate

Le composé décrit au stade précédent (190 mg ; 0,43 mmol) est agité dans 8 ml de dichlorométhane à température ambiante. On ajoute goutte à goutte l'acide trifluoroacétique (979 mg ; 8,59 mmol ; 660 µl) et l'agitation est maintenue pendant 24 heures. Le milieu réactionnel est dilué par un mélange de diéthyléther et de pentane. Le précipité formé est filtré et lavé au pentane pour conduire au produit du titre.

| Microanalyse élémentaire : C₂₃H₂₅N₃O₃, C₂HF₃O₂ (1,3 mol) | | | |
|---|---|---|---|
| | C | H | N |
| % Calculé | 56,97 | 4,91 | 7,79 |
| % Trouvé | 56,82 | 4,87 | 7,60 |

### EXEMPLE 9 : Acide [7-(2-oxo-2-{[4-(2-pyridinylamino)butyl]amino}éthyl)-5H-benzo[a]cycloheptèn-5-yl]acétique, trifluoroacétate

Le produit attendu est obtenu selon le procédé décrit dans l'exemple 8 en remplaçant, au stade b, le composé de la préparation A par le composé décrit dans la préparation B.

| Microanalyse élémentaire : C₂₄H₂₇N₃O₃, C₂HF₃O₂ (1,3 mol) | | | |
|---|---|---|---|
| | C | H | N |
| % Calculé | 57,7 | 5,15 | 7,59 |
| % Trouvé | 57,36 | 4,99 | 7,57 |

### EXEMPLE 10 : Acide [7-(2-oxo-2-{[2-(2-pyridinylamino)éthyl]amino}éthyl)-5H-benzo[a]cycloheptèn-5-yl]acétique, trifluoroacétate

Le produit attendu est obtenu selon le procédé décrit dans l'exemple 8 en remplaçant, au stade b, le composé de la préparation A par le composé décrit dans la préparation C.

| Microanalyse élémentaire : C₂₂H₂₃N₃O₃, C₂HF₃O₂ (1,1mol) | | | |
|---|---|---|---|
| | C | H | N |
| % Calculé | 57,80 | 4,84 | 8,36 |
| % Trouvé | 57,41 | 4,89 | 8,25 |

### EXEMPLE 11 : Acide {7-[2-oxo-2-({3-[(2-pyridinylamino)méthyl]benzyl}amino) éthyl]-6,9-dihydro-5H-benzo[a]cycloheptèn-5-yl}acétique, trifluoroacétate

Le produit attendu est obtenu selon le procédé décrit dans l'exemple 8 en remplaçant, au stade b, le composé de la préparation A par le composé décrit dans la préparation D.

| Microanalyse élémentaire : C₂₈H₂₇N₃O₃, C₂HF₃O₂ | | | |
|---|---|---|---|
| | C | H | N |
| % Calculé | 59,61 | 4,61 | 6,73 |
| % Trouvé | 59,75 | 5,12 | 6,29 |

### EXEMPLE 12 : Acide [7-(2-oxo-2-{[3-(N1-4,5,6,7-tétrahydro-3H-azépin-2-yl) propyl]amino}éthyl)-5H-benzo[a]cycloheptèn-5-yl]acétique, trifluoroacétate

Le produit attendu est obtenu selon le procédé décrit dans l'exemple 8 en remplaçant, au stade b, le composé de la préparation A par le composé décrit dans la préparation M.

### EXEMPLE 13 : Acide [7-(2-oxo-2-{[3-(4,5-dihydro-1H-imidazol-2-yl)propyl] amino}éthyl)-5H-benzo[a]cycloheptèn-5-yl]acétique, trifluoroacétate

Le produit attendu est obtenu selon le procédé décrit dans l'exemple 8 en remplaçant, au stade b, le composé de la préparation A par le composé décrit dans la préparation N.

### EXEMPLE 14 : Acide {7-[2-oxo-2-({3-[(4,5-dihydro-1H imidazol-2-ylamino) méthyl]benzyl}amino)éthyl]-6,9-dihydro-5H-benzo[a] cycloheptèn-5-yl}acétique, trifluoroacétate

Le produit attendu est obtenu selon le procédé décrit dans l'exemple 8 en remplaçant, au stade b, le composé de la préparation A par le composé décrit dans la préparation O.

### EXEMPLE 15 : Acide [7-({[4-(2-pyridinylamino)butanoyl]amino}méthyl)-5H-benzo[a]cycloheptèn-5-yl]acétique, chlorhydrate

### Stade a : [7-(Hydroxyméthyl)-5H-benzo[a]cycloheptèn-5-yl]acétate de tert-butyle

Le produit attendu est obtenu selon le procédé décrit dans l'exemple 1, au stade a, en remplaçant le composé décrit dans la préparation 1 par le produit décrit dans la préparation 4.

### Stade b : {7-[(1,3-Dioxo-1,3-dihydro-2H-isoindol-2-yl)méthyl]-5H-benzo[a] cycloheptèn-5-yl}acétate de tert-butyle

A une solution du composé décrit au stade précédent (2,5 g ; 8,7 mmol) dans 12 ml de dichlorométhane, refroidie à 0°C, on ajoute successivement le phtalimide (1,67 g ; 11,3 mmol), la triphénylphosphine (2,98 g ; 11,3 mmol) et le DEAD (1,97 g ; 11,3 mmol). L'agitation est maintenue de 0°C jusqu'à température ambiante pendant 5 heures. Après concentration, le résidu est purifié par chromatographie sur gel de silice (éluant : dichlorométhane) pour conduire au produit désiré.

### Stade c : (7-Aminométhyl-5H-benzo[a]cycloheptèn-5-yl)acétate de tert-butyle

Le composé décrit au stade précédent (5,72 g ; 13,8 mmol) est dilué dans 60 ml de dichlorométhane. Le monohydrate d'hydrazine (2,07 g ; 41,3 mmol) en solution dans 60 ml de méthanol est ensuite ajouté. Le milieu réactionnel est agité pendant 24 heures à température ambiante. La réaction est filtrée, et le filtrat concentré. Le résidu est repris dans l'éther et lavé par une solution aqueuse de carbonate de sodium à 5 %. La phase organique est séchée sur sulfate de magnésium, concentrée et purifiée par chromatographie sur gel de silice (éluant : dichlorométhane/éthanol/ammoniac, 95/5/0,5) pour conduire au produit attendu.

### Stade d : {7-[({4-[(tert-butoxycarbonyl)(2-pyridinyl)amino]butanoyl}amino)méthyl] -5H-benzo[a]cycloheptèn-5-yl}acétate de tert-butyle

A une solution du composé décrit au stade précédent (500 mg ; 1,76 mmol) dans 10 ml de dichlorométhane, on ajoute goutte à goutte une solution du composé décrit dans la préparation F (1,26 g ; 2,63 mmol) dans 5 ml de dichlorométhane. Le milieu réactionnel est agité pendant 24 heures à température ambiante. Après concentration, le résidu obtenu est purifié par chromatographie sur gel de silice (éluant : éther de pétrole / diéthyléther 1/2) pour conduire au produit attendu.

### Stade e : Acide [7-({[4-(2-pyridinylamino)butanoyl]amino}méthyl)-5H-benzo[a] cycloheptèn-5-yl]acétique, chlorhydrate

Le produit attendu est obtenu selon le procédé décrit dans l'exemple 8, stade c, en utilisant comme produit de départ le composé décrit au stade précédent, et en remplaçant l'acide trifluoroacétique par une solution d'éther chlorhydrique.

| Microanalyse élémentaire : C₂₃H₂₅N₃O₃, HCl | | | | |
|---|---|---|---|---|
| | C | H | N | Cl |
| % Calculé | 64,01 | 6,10 | 9,74 | 9,04 |
| % Trouvé | 63,67 | 6,02 | 9,43 | 8,42 |

### EXEMPLE 16 : Acide [7-({[5-(2-pyridinylamino)pentanoyl]amino}méthyl)-5H-benzo[a]cycloheptèn-5-yl]acétique, trifluoroacétate

Le produit attendu est obtenu selon le procédé décrit dans l'exemple 15, en remplaçant, au stade d, le produit de la préparation F par le produit de la préparation G.

### EXEMPLE 17 : Acide [7-({[4-(3,4,5,6-tétrahydro-2H-azépin-7-ylamino)butanoyl] amino}méthyl)-5H-benzo[a]cycloheptèn-5-yl]acétique, trifluoroacétate

Le produit attendu est obtenu selon le procédé décrit au stade b de l'exemple 8, en utilisant comme produit de départ le composé décrit au stade c de l'exemple 15 et en remplaçant le produit de la préparation A par le composé décrit dans la préparation P, suivi d'une déprotection selon le stade c de l'exemple 8.

### EXEMPLE 18 : Acide [7-({[5-(3,4,5,6-tétrahydro-2H-azépin-7-ylamino) pentanoyl]amino}méthyl)-5H-benzo[a]cycloheptén-5-yl]acétique, trifluoroacétate

Le produit attendu est obtenu selon le procédé décrit au stade b de l'exemple 8, en utilisant comme produit de départ le composé décrit au stade c de l'exemple 15 et en remplaçant le produit de la préparation A par le composé décrit dans la préparation T, suivi d'une déprotection selon le stade c de l'exemple 8.

### EXEMPLE 19 : Acide [7-({[4-(4,5-dihydro-1H-imidazol-2-ylamino)butanoyl] amino}méthyl)-5H-benzo[a]cycloheptèn-5-yl]acétique, trifluoroacétate

Le produit attendu est obtenu selon le procédé décrit au stade b de l'exemple 8, en utilisant comme produit de départ le composé décrit au stade c de l'exemple 15 et en remplaçant le produit de la préparation A par le composé décrit dans la préparation R, suivi d'une déprotection selon le stade c de l'exemple 8.

### EXEMPLE 20 : Acide [7-({[5-(4,5-dihydro-1H-imidazol-2-ylamino)pentanoyl] amino}méthyl)-5H-benzo[a]cycloheptèn-5-yl]acétique, trifluoroacétate

Le produit attendu est obtenu selon le procédé décrit au stade b de l'exemple 8, en utilisant comme produit de départ le composé décrit au stade c de l'exemple 15 et en remplaçant le produit de la préparation A par le composé décrit dans la préparation V, suivi d'une déprotection selon le stade c de l'exemple 8.

### EXEMPLE 21 : Acide [7-({[4-(1H-benzimidazol-2-ylamino)butanoyl]amino} méthyl)-5H-benzo[a]cycloheptèn-5-yl]acétique, chlorhydrate

Le produit attendu est obtenu selon le procédé décrit dans l'exemple 15, en remplaçant, au stade d, le produit de la préparation F par le produit de la préparation Q.

| Microanalyse élémentaire : C₂₅H₂₆N₄O₃, HCl | | | | |
|---|---|---|---|---|
| | C | H | N | Cl |
| % Calculé | 64,30 | 5,83 | 12,00 | 7,59 |
| % Trouvé | 64,34 | 5,81 | 11,90 | 7,38 |

### EXEMPLE 22 : Acide [7-({[5-(1H-benzimidazol-2-ylamino)pentanoyl]amino} méthyl)-5H-benzo[a]cycloheptèn-5-yl]acétique, trifluoroacétate

Le produit attendu est obtenu selon le procédé décrit dans l'exemple 15, en remplaçant, au stade d, le produit de la préparation F par le produit de la préparation U.

### EXEMPLE 23 : Acide [7-({[3-(2-pyridinylamino)propanoyl]amino}méthyl)-6,9-dihydro-5H-benzo[a]cycloheptèn-5-yl]acétique, trifluoroacétate

### Stade a : {7-[(1,3-Dioxo-1,3-dihydro-2H-isoindol-2-yl)méthyl]-6,9-dihydro-5H-benzo[a]cycloheptèn-5-yl}acétate de tert-butyle

A une solution du composé décrit au stade b de l'exemple 1 (2 g ; 5,69 mmol) dans 15 ml de diméthylformamide, on ajoute le phtalimidate de potassium (2 g ; 11,38 mmol) à température ambiante. Après 24 heures d'agitation, le milieu réactionnel est dilué avec 250 ml de dichlorométhane et lavé avec une solution aqueuse saturée en chlorure de sodium. Après extraction, la phase organique est séchée sur sulfate de magnésium, concentrée et purifiée par chromatographie sur gel de silice (éluant : toluène/acétate d'éthyle, 95/5) pour conduire au produit attendu.

### Stade b : [7-(Aminométhyl)-6,9-dihydro-5H-benzo[a]cycloheptèn-5-yl]acétate de tert-butyle

Le composé décrit au stade précédent (1,45 g ; 3,47 mmol) est dilué, sous atmosphère d'argon, dans 35 ml de dichlorométhane. On ajoute goutte à goutte le monohydrate d'hydrazine (520 mg ; 10,41 mmol ; 510 µl) en solution dans 35 ml de méthanol. Le milieu réactionnel est agité pendant 24 heures à température ambiante. Le précipité formé est filtré, et le filtrat concentré. Le résidu obtenu est repris dans l'éther et lavé avec une solution aqueuse de carbonate de sodium à 5 %. La phase organique est séchée sur sulfate de magnésium concentrée et purifiée par chromatographie sur gel de silice (éluant : dichlorométhane/éthanol/ammoniac 95/5/0,5) pour conduire au produit attendu.

### Stade c : {7-[({3-((tert-Butoxycarbonyl)(2-pyridinyl)amino]propanoyl}amino) méthyl]-6,9-dihydro-5H-benzo[a]cycloheptèn-5-yl}acétate de tert-butyle

A une solution du composé décrit au stade précédent (160 mg ; 0,53 mmol) dans 1 ml de dichlorométhane, on additionne goutte à goutte une solution du composé décrit dans la préparation E (260 mg ; 0,56 mmol) dans 3 ml de dichlorométhane. Le milieu réactionnel est agité pendant 24 heures à température ambiante. Après concentration, le résidu obtenu est purifié par chromatographie sur gel de silice (éluant éther de pétrole / diéthyléther 1/2), pour conduire au produit du titre.

### Stade d : Acide [7-({[3-(2-pyridinylamino)propanoyl]amino}méthyl)-6,9-dihydro-5H-benzo[a]cycloheptèn-5-yl]acétique, trifluoroacétate

Le produit attendu est obtenu selon le procédé décrit dans l'exemple 8, stade c, en utilisant comme produit de départ le composé décrit au stade précédent.

| Microanalyse élémentaire : C₂₂H₂₅N₃O₃, C₂HF₃O₂ | | | |
|---|---|---|---|
| | C | H | N |
| % Calculé | 58,41 | 5,31 | 8,51 |
| % Trouvé | 58,45 | 5,31 | 8,24 |

### EXEMPLE 24 : Acide [7-({[4-(2-pyridinylamino)butanoyl]amino}méthyl)-6,9-dihydro-5H-benzo[a]cycloheptèn-5-yl]acétique, chlorhydrate

Le produit attendu est obtenu selon le procédé décrit dans l'exemple 23 en remplaçant, au stade c, le composé décrit dans la préparation E par le composé de la préparation F, et au stade d, en remplaçant l'acide trifluoroacétique par une solution d'éther chlorhydrique. Une résolution sur HPLC chirale (éluant : n-heptane/EtOH/Et₃N 750/250/1) sur phase chirale Whelk 01 permet d'obtenir les 2 énantiomères.

| 24a) (+) Microanalyse élémentaire: C₂₃H₂₇N₃O₃, 1,5 HCl | | | | |
|---|---|---|---|---|
| | C | H | N | Cl |
| % Calculé | 61,63 | 6,42 | 9,38 | 11,86 |
| % Trouvé | 61,60 | 6,33 | 9,23 | 11,04 |

| 24b) (-) Microanalyse élémentaire : C₂₃H₂₇N₃O₃, HCl | | | | |
|---|---|---|---|---|
| | C | H | N | Cl |
| % Calculé | 64,25 | 6,56 | 9,77 | 8,25 |
| % Trouvé | 64,14 | 6,17 | 9,55 | 9,18 |

### EXEMPLE 25 : Acide [7-({[5-(2-pyridinylamino)pentanoyl]amino}méthyl)-6,9-dihydro-5H-benzo[a]cycloheptèn-5-yl]acétique, chlorhydrate

Le produit attendu est obtenu selon le procédé décrit dans l'exemple 23 en remplaçant, au stade c, le composé décrit dans la préparation E par le composé de la préparation G, et au stade d, en remplaçant l'acide trifluoroacétique par une solution d'éther chlorhydrique

| Microanalyse élémentaire : C₂₄H₂₉N₃O₃, 2 HCl | | | | |
|---|---|---|---|---|
| | C | H | N | Cl |
| % Calculé | 60,00 | 6,50 | 8,75 | 14,76 |
| % Trouvé | 59,68 | 6,51 | 8,61 | 14,15 |

### EXEMPLE 26 : {7-[({3-[(2-Pyridinylamino)méthyl)benzoyl}amino)méthyl]-6,9-dihydro-5H-benzo[a]cycloheptèn-5-yl}acétate de tert-butyle.

Le produit attendu est obtenu selon le procédé décrit dans l'exemple 23, stades a, b et c, en remplaçant, au stade c, le composé décrit dans la préparation E par le composé de la préparation H.

### EXEMPLE 27 : Acide {7-[({3-[(2-pyridinylamino)méthyl]benzoyl}amino)méthyl]-6,9-dihydro-5H-benzo[a]cycloheptèn-5-yl}acétique, trifluoroacétate

Le produit attendu est obtenu selon le procédé décrit dans l'exemple 8, stade c, en utilisant comme produit de départ le composé décrit dans l'exemple 26.

| Microanalyse élémentaire : C₂₇H₂₇N₃O₃, C₂HF₃O₂ (1,3 mol) | | | |
|---|---|---|---|
| | C | H | N |
| % Calculé | 60,28 | 4,85 | 7,13 |
| % Trouvé | 61,53 | 5,07 | 7,15 |

### EXEMPLE 28 : Acide [7-({[4-(1H-benzoimidazol-2-ylamino)butanoyl]amino} méthyl)-6,9-dihydro-5H-benzo[a]cycloheptèn-5-yl]acétique, chlorhydrate

Le produit attendu est obtenu selon le procédé décrit dans l'exemple 23 en remplaçant, au stade c, le composé décrit dans la préparation E par le composé de la préparation Q, et au stade d, en remplaçant l'acide trifluoroacétique par une solution d'éther chlorhydrique.

| Microanalyse élémentaire : C₂₅H₂₈N₄O₃, HCl | | | | |
|---|---|---|---|---|
| | C | H | N | Cl |
| % Calculé | 64,03 | 6,23 | 11,95 | 7,56 |
| % Trouvé | 63,66 | 6,29 | 11,82 | 7,72 |

### EXEMPLE 29 : [7-({[4-(N1-4,5,6,7-Tétrahydo-3H-azépin-2-ylamino)butanoyl] amino}méthyl)-6,9-dihydro-5H-benzo[a]cycloheptèn-5-yl]acétate de tert-butyle

Le produit attendu est obtenu selon le procédé décrit dans le stade b de l'exemple 8, en remplaçant le composé décrit dans la préparation A par le composé de la préparation P, et en remplaçant le composé du stade a par le composé du stade b de l'exemple 23.

### EXEMPLE 30 : Acide [7-({[4-(N1-4,5,6,7-tétrahydo-3H-azépin-2-ylamino) butanoyl]amino}méthyl)-6,9-dihydro-5H-benzo[a]cycloheptèn-5-yl]acétique, chlorhydrate

Le produit attendu est obtenu selon le procédé décrit dans l'exemple 8, stade c, en utilisant comme produit de départ le composé décrit dans l'exemple 29, et en remplaçant l'acide trifluoroacétique par une solution d'éther chlorhydrique.

### EXEMPLE 31 : [7-({[4-(4,5-Dihydro-1H-imidazol-2-ylamino)butanoyl)amino} méthyl)-6,9-dihydro-5H-benzo[a]cycloheptèn-5-yl]acétate de tert-butyle

A une solution du composé obtenu dans la préparation 5 (0,6 g ; 1,61 mmol) en solution dans 9 ml de CH₂Cl₂ sous agitation, à température ambiante, on ajoute successivement le composé de la préparation X (9,66 mmol), puis Et₃N (1,14 g ; 11,27 mmol ; 1,61 ml). Le milieu réactionnel est ensuite chauffé pendant 72 heures à 35°C. Après retour à température ambiante, le milieu réactionnel est hydrolysé, extrait au dichlorométhane. Après concentration de la phase organique, le résidu obtenu est chromatographié sur gel de silice (éluant : CH₂Cl₂/EtOH/NH₄OH 98/2/0,2), pour conduire au produit du titre.

### EXEMPLE 32 : Acide [7-({[4-(4,5-dihydro-1H-imidazol-2-ylamino)butanoyl] amino}méthyl)-6,9-dihydro-5H-benzo[a]cycloheptèn-5 yl]acétique, chlorhydrate

On procède comme au stade d de l'exemple 23 en utilisant l'éther chlorhydrique au lieu de l'acide trifluoroacétique.

| Microanalyse élémentaire : C₂₁H₂₈N₄O₃, 1,25HCl | | | | |
|---|---|---|---|---|
| | C | H | N | Cl |
| % Calculé | 54,75 | 7,40 | 12,17 | 9,69 |
| % Trouvé | 55,80 | 6,87 | 12,04 | 9,27 |

### EXEMPLE 33 : {7-[({3-[(1,4,5,6-Tétrahydro-2-pyrimidinylamino)méthyl) benzoyl}amino)méthyl]-6,9-dihydro-5H-benzo[a]cycloheptèn-5-yl}acétate de tert-butyle

Le produit attendu est obtenu selon le procédé décrit dans l'exemple 8, stade b en remplaçant, le composé décrit dans la préparation A par le composé de la préparation S.

### EXEMPLE 34 : Acide {7-[({3-[(1,4,5,6-tétrahydro-2-pyrimidinylamino)méthyl) benzoyl}amino)méthyl]-6,9-dihydro-5H-benzo[a]cycloheptèn-5-yl}acétique, trifluoroacétate

Le produit attendu est obtenu selon le procédé décrit dans l'exemple 8, stade c, en utilisant comme produit de départ le composé décrit dans l'exemple 33.

### EXEMPLE 35 : [2-Oxo-3-(2-oxo-2-{[3-(2-pyridinylamino)propyl]amino}éthyl)-2,3,4,5-tétrahydro-1H-3-benzazépin-1-yl]acétate de tert-butyle

Le produit attendu est obtenu selon le procédé décrit dans l'exemple 8, en utilisant, au stade a comme produit de départ le composé décrit dans la préparation 3.

### EXEMPLE 36 : Acide [2-oxo-3-(2-oxo-2-{[3-(2-pyridinylamino)propyl]amino} éthyl)-2,3,4,5-tétrahydro-1H-3-benzazépin-1-yl]acétique, trifluoroacétate

Le produit attendu est obtenu selon le procédé décrit dans l'exemple 8, stade c, en utilisant comme produit de départ le composé décrit dans l'exemple 35.

### EXEMPLE 37 : [2-Oxo-3-(2-oxo-2-{[4-(2-pyridinylamino)butyl]amino}éthyl)-2,3,4,5-tétrahydro-1H-3-benzazépin-1-yl]acétate de tert-butyle

Le produit attendu est obtenu selon le procédé décrit dans l'exemple 8, en utilisant, au stade a comme produit de départ le composé décrit dans la préparation 3, et en remplaçant, au stade b le produit de la préparation A par le produit décrit dans la préparation B.

### EXEMPLE 38 : Acide [2-oxo-3-(2-oxo-2-{[4-(2-pyridinylamino)butyl]amino} éthyl)-2,3,4,5-tétrahydro-1H-3-benzazépin-1-yl]acétique, trifluoroacétate

Le produit attendu est obtenu selon le procédé décrit dans l'exemple 8, stade c, en utilisant comme produit de départ le composé décrit dans l'exemple 37.

### EXEMPLE 39 : [2-Oxo-3-(2-oxo-2-{[2-(2-pyridinylamino)éthyl]amino}éthyl)-2,3,4,5-tétrahydro-1H-3-benzazépin-1-yl]acétate de tert-butyle

Le produit attendu est obtenu selon le procédé décrit dans l'exemple 8, en utilisant, au stade a comme produit de départ le composé décrit dans la préparation 3, et en remplaçant, au stade b le produit de la préparation A par le produit décrit dans la préparation C.

### EXEMPLE 40 : Acide [2-oxo-3-(2-oxo-2-{[2-(2-pyridinylamino)éthyl]amino}éthyl) - 2,3,4,5-tétrahydro-1H-3-benzazépin-1-yl]acétique, trifluoroacétate.

Le produit attendu est obtenu selon le procédé décrit dans l'exemple 8, stade c, en utilisant comme produit de départ le composé décrit dans l'exemple 39.

### EXEMPLE 41 : {2-Oxo-3-[2-oxo-2-({3-[(2-pyridinylamino)méthyl]benzyl}amino) éthyl]-2,3,4,5-tétrahydro-1H-3-benzazépin-1-yl}acétate de tert-butyle

Le produit attendu est obtenu selon le procédé décrit dans l'exemple 8, en utilisant, au stade a comme produit de départ le composé décrit dans la préparation 3, et en remplaçant, au stade b le produit de la préparation A par le produit décrit dans la préparation D.

### EXEMPLE 42 : Acide {2-oxo-3-[2-oxo-2-({3-[(2-pyridinylamino)méthyl]benzyl} amino)éthyl]-2,3,4,5-tétrahydro-1H-3-benzazépin-1-yl}acétique, trifluoroacétate

Le produit attendu est obtenu selon le procédé décrit dans l'exemple 8, stade c, en utilisant comme produit de départ le composé décrit dans l'exemple 41.

### EXEMPLE 43 : [2-Oxo-3-(2-oxo-2-{[3-(N1-4,5,6,7-tétrabydro-3H-azépin-2-yl) propyl]amino}éthyl}-2,3,4,5-tétrahydro-1H-3-benzazépin-1-yl] acétate de tert-butyle

Le produit attendu est obtenu selon le procédé décrit dans l'exemple 8, en utilisant, au stade a comme produit de départ le composé décrit dans la préparation 3, et en remplaçant, au stade b, le produit de la préparation A par le produit décrit dans la préparation M.

### EXEMPLE 44 : Acide [2-oxo-3-(2-oxo-2-{[3-(N1-4,5,6,7-tétrahydro-3H-azépin-2-yl)propyl]amino}éthyl)-2,3,4,5-tétrahydro-1H-3-benzazépin-1-yl} acétique, trifluoroacétate

Le produit attendu est obtenu selon le procédé décrit dans l'exemple 8, stade c, en utilisant comme produit de départ le composé décrit dans l'exemple 43.

### EXEMPLE 45 : [2-Oxo-3-(2-oxo-2-{[3-(4,5-dihydro-1H-imidazol-2-yl)propyl] amino}éthyl)-2,3,4,5-tétrahydro-1H-3-benzazépin-1-yl]acétate de tert-butyle

Le produit attendu est obtenu selon le procédé décrit dans l'exemple 8, en utilisant, au stade a comme produit de départ le composé décrit dans la préparation 3, et en remplaçant, au stade b, le produit de la préparation A par le produit décrit dans la préparation N.

### EXEMPLE 46 : Acide [2-oxo-3-(2-oxo-2-{[3-(4,5-dihydro-1H-imidazol-2-yl)propyl] amino}éthyl)-2,3,4,5-tétrahydro-1H-3-benzazépin-1-yl]acétique, trifluoroacétate

Le produit attendu est obtenu selon le procédé décrit dans l'exemple 8, stade c, en utilisant comme produit de départ le composé décrit dans l'exemple 45.

### EXEMPLE 47 : {2-Oxo-3-[2-oxo-2-({3-[(4,5-dihydro-1H-imidazol-2-ylamino) méthyl]benzyl}amino)éthyl]-2,3,4,5-tétrahydro-1H-3-benzazépin-1-yl}acétate de tert-butyle

Le produit attendu est obtenu selon le procédé décrit dans l'exemple 8, en utilisant, au stade a comme produit de départ le composé décrit dans la préparation 3, et en remplaçant, au stade b, le produit de la préparation A par le produit décrit dans la préparation O.

### EXEMPLE 48 : Acide {2-oxo-3-[2-oxo-2-({3-[(4,5-dihydro-1H-imidazol-2-ylamino)méthyl]benzyl}amino)éthyl]-2,3,4,5-tétrahydro-1H-3-benzazépin-1 -yl}acétique, trifluoroacétate

Le produit attendu est obtenu selon le procédé décrit dans l'exemple 8, stade c, en utilisant comme produit de départ le composé décrit dans l'exemple 47.

### EXEMPLE 49 : [7-({[4-(1,4,5,6-Tétrahydro-2-pyrimidinylamino)butanoyl]amino} méthyl)-6,9-dihydro-5H-benzo[a]cycloheptèn-5-yl]acétate de tert-butyle

A une solution du composé obtenu dans la préparation 5 (0,6 g ; 1,61 mmol) dans 9 ml de CH₂Cl₂ à température ambiante, on ajoute sous agitation successivement le composé de la préparation W (2,22 g ; 9,66 mmol), puis Et₃N (1,14 g ; 11,27 mmol ; 1,61 ml). Le milieu réactionnel est ensuite chauffé pendant 72 heures à 35°C. Après retour à température ambiante, le milieu réactionnel est hydrolysé, extrait au dichlorométhane. Après concentration de la phase organique, le résidu obtenu est chromatographié sur gel de silice (éluant : CH₂Cl₂/EtOH /NH₄OH 98/2/0,2), pour conduire au produit attendu sous forme d'une mousse blanchâtre.

### EXEMPLE 50 : Acide [7-({[4-(1,4,5,6-tétrahydro-2-pyrimidinylamino)butanoyl] amino}méthyl)-6,9-dihydro-5H-benzo[a]cycloheptèn-5-yl] acétique, chlorhydrate

On procède comme au stade d de l'exemple 23 en utilisant une solution d'éther chlorhydrique au lieu de l'acide trifluoroacétique.

| Microanalyse élémentaire : C₂₂H₃₀N₄O₃, HCl | | | | |
|---|---|---|---|---|
| | C | H | N | Cl |
| % Calculé | 60,75 | 7,18 | 12,88 | 8,15 |
| % Trouvé | 61,02 | 7,07 | 12,65 | 8,38 |

### EXEMPLE 51 : {7-[({4-[(5-Méthoxy-1H-benzimidazol-2-yl)amino]butanoyl} amino)méthyl]-6,9-dihydro-5H-benzo[a]cycloheptèn-5-yl}acétate de tert-butyle

A une solution du composé obtenu dans la préparation 5 (0,6 g ; 16,1 mmol) dans 10 ml de CH₂Cl₂ sous agitation sous atmosphère d'argon à -78°C, on ajoute successivement le composé de la préparation Y (1,274 g ; 33,5 mmol), la triéthylamine (0,814 g ; 8,05 mmol ; 1,15 ml) et HgCl₂ (0,914 g ; 3,36 mmol). Après 10 minutes d'agitation à -78°C, le milieu réactionnel est filtré sur célite, lavé avec une solution aqueuse saturée en NaCl et extrait au dichlorométhane. Après séchage et concentration de la phase organique, le résidu obtenu est chromatographie sur gel de silice (éluant : CH₂Cl₂/EtOH/NH₄OH 98/2/0,2) pour conduire au composé désiré sous forme d'une mousse beige.

### EXEMPLE 52 : Acide {7-[({4-[(5-méthoxy-1H-benzimidazol-2-yl)amino]butanoyl} amino)méthyl]-6,9-dihydro-5H-benzo[a]cycloheptèn-5-yl} acétique, chlorhydrate

On procède comme au stade d de l'exemple 23 en remplaçant l'acide trifluoroacétique par l'éther chlorhydrique.

| Microanalyse élémentaire : C₂₆H₃₀N₄O₄, HCl | | | | |
|---|---|---|---|---|
| | C | H | N | Cl |
| % Calculé | 62,58 | 6,29 | 11,23 | 7,10 |
| % Trouvé | 61,96 | 6,09 | 11,03 | 7,69 |

### EXEMPLE 53 : {7-[({3-[(2-Pyridinylméthyl)amino]propanoyl}amino)méthyl]-6,9-dibydro-5H-benzo[a]cycloheptèn-5-yl}acétate de tert-butyle

On procède comme au stade c de l'exemple 23 en remplaçant le composé obtenu dans la préparation E par le composé obtenu dans la préparation Z.

### EXEMPLE 54 : Acide {7-[({3-[(2-pyridinylméthyl)amino]propanoyl}amino) méthyl]-6,9-dibydro-5H-benzo[a]cycloheptèn-5-yl}acétique, chlorhydrate

On procède comme au stade c de l'exemple 8 à partir du composé obtenu dans l'exemple 53.

### EXEMPLE 55 : {7-[({3-[(1H-Benzimidazol-2-ylméthyl)amino]propanoyl}amino) méthyl]-6,9-dihydro-5H-benzo[a]cycloheptèn-5-yl}acétate de tert-butyle

On procède comme au stade c de l'exemple 23 en remplaçant le composé obtenu dans la préparation E par le composé obtenu dans la préparation A₁.

### EXEMPLE 56 : Acide {7-[({3-[(1H-benzimidazol-2-ylméthyl)amino]propanoyl} amino)méthyl]-6,9-dihydro-5H-benzo[a]cycloheptèn-5-yl} acétique, chlorhydrate

On procède comme au stade d de l'exemple 23 à partir du composé obtenu dans l'exemple 55, en remplaçant l'acide trifluoroacétique par l'éther chlorhydrique.

| Microanalyse élémentaire : C₂₅H₂₈N₄O₃, 2HCl | | | | |
|---|---|---|---|---|
| | C | H | N | Cl |
| % Calculé | 59,41 | 5,98 | 11,08 | 14,03 |
| % Trouvé | 59,64 | 5,93 | 10,95 | 14,27 |

### EXEMPLE 57 : [7-({[4-(5,6,7,8-Tétrahydro[1,8]naphtyridin-2-yl)butanoyl]amino} méthyl)-6,9-dihydro-5H-benzo[a]cycloheptèn-5-yl]acétate de tert-butyle

On procède comme au stade b de l'exemple 8 à partir du composé obtenu au stade b de l'exemple 23 et du composé obtenu dans la préparation A₂.

### EXEMPLE 58 : Acide [7-({[4-(5,6,7,8-tétrahydro[1,8]naphtyridin-2-yl)butanoyl] amino}méthyl)-6,9-dihydro-5H-benzo[a]cycloheptèn-5-yl] acétique, chlorhydrate

Le composé obtenu dans l'exemple 57 est mis en solution dans un mélange CH₂Cl₂/éther chlorhydrique et agité à température ambiante pendant 72 heures. Le précipité obtenu est essoré pour conduire au produit du titre.

| Microanalyse élémentaire : C₂₆H₃₁N₃O₃, 1,2 HCl | | | | |
|---|---|---|---|---|
| | C | H | N | Cl |
| % Calculé | 65,42 | 6,81 | 8,81 | 8,91 |
| % Trouvé | 64,93 | 6,40 | 8,76 | 7,83 |

### EXEMPLE 59 : [7-({[4-Oxo-4-(2-pyridinylamino)butanoyl]amino}méthyl)-6,9-dihydro-5H-benzo[a]cycloheptèn-5-yl]acétate de tert-butyle

A une solution du composé obtenu au stade b de l'exemple 23 (0,322 g ; 1,12 mmol) dans 25 ml de CH₂Cl₂ sous agitation à température ambiante, on ajoute successivement le composé obtenu dans la préparation A₃ (0,2 g ; 1,12 mmol), la diisopropyléthylamine (0,724 g ; 5,6 mmol ; 0,97 ml), le HOBT (1,34 mmol) et finalement l'EDC (0,257 g ; 1,34 mmol). Le milieu réactionnel est agité 10 heures à température ambiante puis est concentré à sec, repris à l'acétate d'éthyle, lavé avec une solution aqueuse saturée en NH₄Cl et extrait à l'acétate d'éthyle. Après séchage et concentration de la phase organique, le résidu obtenu est purifié par chromatographie sur gel de silice (éluant : CH₂Cl₂/MeOH 9872) pour conduire au composé du titre sous la forme d'une huile visqueuse.

### EXEMPLE 60 : Acide [7-({[4-oxo-4-(2-pyridinylamino)butanoyl]amino}méthyl)-6,9-dihydro-5H-benzo[a]cycloheptèn-5-yl]acétique, chlorhydrate

Le composé obtenu dans l'exemple précédent est dissout dans 10 ml de CH₂Cl₂ et agité en présence de 25 ml d'éther chlorhydrique à température ambiante pendant 72 heures. Le précipité obtenu est essoré pour conduire au produit du titre sous forme d'une poudre blanche.

### EXEMPLE 61 : [7-({[4-(1,3-Thiazol-2-ylamino)butanoyl]amino}méthyl)-6,9-dihydro-5H-benzo[a]cyclopentèn-5-yl}acétate de tert-butyle

On procède comme dans l'exemple 23 stade c en remplaçant le composé obtenu dans la préparation E par le composé obtenu dans la préparation A₄.

### EXEMPLE 62 : Acide [7-({[4-(1,3-thiazol-2-ylamino)butanoyl]amino}méthyl)-6,9-dihydro-5H-benzo[a]cyclopentèn-5-yl]acétique, chlorhydrate

On procède comme au stade d de l'exemple 23 à partir du composé obtenu dans l'exemple 61, en remplaçant l'acide trifluoroacétique par l'éther chlorhydrique.

| Microanalyse élémentaire : C₂₁H₂₅N₃O₃S, HCl | | | | | |
|---|---|---|---|---|---|
| | C | H | N | S | Cl |
| % Calculé | 56,90 | 5,97 | 9,48 | 7,23 | 9,60 |
| % Trouvé | 56,85 | 5,90 | 9,26 | 7,13 | 9,19 |

### EXEMPLE 63 : [4-({[4-(2-Pyridinylamino)butanoyl]amino}méthyl)-2,3-dihydro-1H-1-benzazépin-2-yl]acétate de méthyle

Le produit du titre est obtenu selon le procédé décrit dans les stades c et d de l'exemple 23, en remplaçant le composé décrit dans la préparation E par le composé de la préparation F et en remplaçant le composé du stade b par le composé obtenu dans la préparation 6, puis en procédant à l'hydrolyse du NBoc en milieu éther chlorhydrique, au lieu de l'acide trifluoroacétique du stade d.

### EXEMPLE 64 : Acide [4-({[4-(2-pyridinylamino)butanoyl]amino}méthyl)-2,3-dihydro-1H-1-benzazépin-2-yl]acétique

Le composé obtenu dans l'exemple 63 est soumis aux conditions d'hydrolyse LiOH/dioxane/H₂O puis HCl (1 N), décrites dans le stade a de l'exemple 8.

### EXEMPLE 65 : [4-({[4-(4,5-Dihydro-1H-imidazol-2-ylamino)butanoyl]amino} méthyl)-2,3-dihydro-1H-1-benzazépin-2-yl]acétate de méthyle

Le produit attendu est obtenu selon le procédé décrit dans les stades c et d de l'exemple 23 en remplaçant le composé décrit dans la préparation E par le composé de la préparation Q et le composé de départ du stade d par le composé obtenu dans la préparation 6, et en effectuant l'hydrolyse du NBoc en milieu éther chlorhydrique, au lieu de l'acide trifluoroacétique du stade d.

### EXEMPLE 66 : Acide [4-({[4-(4,5-dihydro-1H-imidazol-2-ylamino)butanoyl] amino}méthyl)-2,3-dihydro-1H-1-benzazépin-2-yl]acétique

Le produit du titre est obtenu en soumettant le composé de l'exemple 65 aux conditions d'hydrolyse LiOH/dioxane/H₂O puis HCl (1 N), décrites dans le stade a de l'exemple 8.

### ETUDE PHARMACOLOGIQUE

### EXEMPLE A : Mesure de l'affinité in vitro aux récepteurs αvβ₃ et αvβ₅ de la vitronectine et au récepteur α_{IIb}β₃ du fibrogène

Les intégrines purifiées par chromatographie d'affinité à partir de placenta humain (αᵥβ₃ et αᵥβ₅) sont diluées à une concentration de 500 ng/ml dans un tampon Tris contenant 2 mM de CaCl₂ et 1 mM de MgCl₂ et MnCl₂, pH 7,5 (tampon de binding), puis transférées pour permettre leur adsorption à raison de 100 µl par puits dans des plaques 96 puits Costar pendant une heure à température ambiante. Après lavage et blocage des sites d'adhérence non spécifiques avec de l'albumine sérique bovine, les intégrines adsorbées sont incubées, dans le tampon de binding contenant 0, 1 % d'albumine, avec de la vitronectine (50 ng par puits) ou du fibrinogène (1 µg par puits) marqués à la biotine en présence ou non des composés testés. Après lavage des puits, la quantité de vitronectine liée à l'intégrine αᵥβ₃ ou αᵥβ₅ ou la quantité de fibrinogène lié à l'intégrine α_{IIb}β₃ est évaluée indirectement par reconnaissance avec un anticorps dirigé contre la biotine et couplé à l'alcaline phosphatase qui permet une révélation par réaction colorimétrique à 405 nm avec du para-nitrophényl phosphate. La concentration de composé conduisant à 50% d'inhibition de la liaison du ligand biotinylé à l'intégrine est alors calculée (IC₅₀).

*Résultats :* Il apparaît que les composés de l'invention possèdent des IC₅₀ de l'ordre du nanomolaire pour les récepteurs αᵥβ₃ et αᵥβ₅, et de l'ordre du micromolaire pour l'intégrine des plaquettes α_{IIb}β₃.

### EXEMPLE B : Test de l'adhérence cellulaire dépendant des intégrines αvβ₃ et αvβ₅

Des cellules endothéliales dérivées de la veine ombilicale de placenta humain sont utilisées pour étudier l'adhérence dépendant de l'intégrine αᵥβ₃. Ces cellules sont utilisées en culture primaire ou bien en lignée établie après immortalisation par transfection avec l'antigène T de SV40 (Biol. Cell, 1991, 73, 7-14, J. Cell Physiol. 1993, 157, 41-51). La lignée de carcinome humain ovarien IGROV1 est utilisée pour l'adhérence dépendant de l'intégrine αᵥβ₅.

La vitronectine humaine est diluée dans un tampon phosphate à une concentration finale de 5 µg/ml et transférée pour permettre son adsorption à raison de 10 µl par puits dans une plaque Terasaki de 60 puits pendant 90 minutes à 37°C. Après lavage et blocage des sites d'adhérence non spécifiques avec de l'albumine sérique bovine, les puits reçoivent les cellules sous forme de suspension. Celles-ci ont préalablement été récupérées par trypsinisation dans du milieu de culture RPMI sans sérum contenant 0.5% d'albumine sérique bovine et préincubées pendant 30 minutes sur glace avec les composés testés. Après adhérence à 37°C en plaque Terasaki pour une durée de 20 minutes (cellules endothéliales) ou une heure (lignée IGROV1), les puits sont lavés. Les cellules ayant adhéré sont fixées au formaldéhyde, colorées au cristal violet et dénombrées par analyse d'image sur l'ensemble du puits. La concentration de composé conduisant à 50% d'inhibition de l'adhérence des cellules à la vitronectine est alors calculée (IC₅₀).

*Résultats :* Les composés de l'invention présentent des activités (IC₅₀) de l'ordre de quelques dizaines de nM et quelques centaines de nM pour l'adhérence dépendant des intégrines αᵥβ₅ et αᵥβ₃, respectivement.

### EXEMPLE C : Test d'agrégation des plaquettes

Le sang veineux provenant de donneurs humains n'ayant pas absorbé d'aspirine pendant les quinze jours précédents est prélevé sur citrate de sodium à 3.8% (un volume pour neuf volumes de sang). Les échantillons sanguins sont centrifugés pendant 15 minutes à 160 g. Le PRP (plasma riche en plaquettes) est recueilli et les plaquettes sont dénombrées. Le PPP (plasma pauvre en plaquettes) est ensuite obtenu par centrifugation du sang restant pendant quinze minutes à 3000 g. Les échantillons sont testés sur un agrégomètre 4 canaux, le PPP étant utilisé comme blanc (100% transmission). 250 µl de PRP (0% de transmission) sont introduits dans chaque microtube. L'agrégation est induite par l'ADP (2.5 µl par tube) à 10 µM et la courbe d'agrégation est alors obtenue (% transmission par rapport au temps). Les produits à tester (2.5 µl) sont ajoutés au PRP à différentes concentrations trois minutes avant l'addition de l'agent d'agrégation. Les résultats sont exprimés en pourcentage d'inhibition de l'agrégation des plaquettes.

*Résultats :* Il apparaît que les composés de l'invention n'ont pas entraîné d'effet sur l'agrégation des plaquettes pour des doses allant jusqu'à 100 µM.

### EXEMPLE D : Test de prolifération des cellules endothéliales

Les cellules endothéliales de la veine ombilicale de placenta humain sont utilisées en culture primaire et ensemencées en plaques de culture de 96 puits. Après 24h de pré-culture en milieu complet contenant du sérum de veau foetal, les cellules sont confrontées aux composés pendant 96h dans le même milieu. Le dénombrement cellulaire est alors réalisé par une méthode indirecte colorimétrique. La concentration de composé conduisant à 50% d'inhibition de prolifération est alors calculée (IC₅₀).

*Résultats :* Il apparaît que les composés de l'invention présentent des IC₅₀ de l'ordre de 10 à 100 nM sur les cellules endothéliales.

### EXEMPLE E : Composition Pharmaceutique

| Formule de préparation pour 1000 comprimés dosés à 10 mg | |
|---|---|
| Composé de l'exemple 24 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 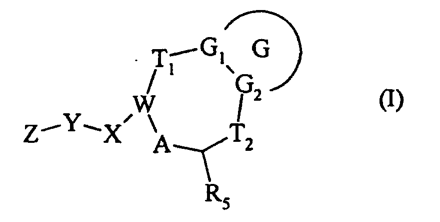 dans laquelle :
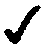 G représente un groupement phényle,
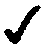 G₁ et G₂ représentent un atome de carbone,
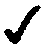 -T₁- représente un groupement choisi parmi -CH₂-CH₂-, -CH=CH-, =CH-CH₂-, et -T₂- représente une liaison,
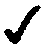 R₅ représente un groupement -(CH₂)ₘ-COOR₆,
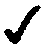 R₆ représente un atome d'hydrogène, un groupement alkyle, aryle éventuellement substitué, ou arylalkyle éventuellement substitué,
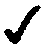 -W- représente un groupement -CH-, =C- ou -C= et -A- représente un groupement -CH₂- ou =CH-,
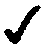 -X- représente un groupement choisi parmi -CO-X₁-, -CO-NR₆-X₁-, -NR₆-CO-X₁-, - O-X₁-, -SO₂-NR₆-X₁-, et -S(O)ₙ-X₁- dans lesquels n est compris inclusivement entre 0 et 2, et X₁ représente un groupement alkylène,
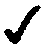 -Y- représente un groupement choisi parmi -Y₁-, -Y₂-Y₁- et -Y₁-Y₂-Y₁-, dans lesquels Y₁ représente un groupement alkylène, alkénylène ou alkynylène, et Y₂ représente un groupement arylène, hétéroarylène, cycloalkylène, ou hétérocycloalkylène,
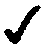 Z- représente un groupement choisi parmi Z₁-, Z₁₀-NR₆-, et Z₁₀-NR₆-CO- dans lesquels Z₁₀ représente un groupement alkyle ou Z₁, et Z, représente un groupement choisi parmi Z₂-, 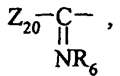 Z₂₀-NR₆-, et Z₂₀-NR₆-CO-, dans lesquels Z₂₀ représente un groupement alkyle, hétéroalkyle, ou Z₂, et Z₂ représente un groupement hétéroaryle éventuellement substitué, hétérocycloalkyle éventuellement substitué, hétéroarylalkyle éventuellement substitué, hétérocycloalkylalkyle éventuellement substitué, arylhétéroaryle fusionné éventuellement substitué, arylhétérocycloalkyle fusionné éventuellement substitué, hétéroarylhétérocycloalkyle fusionné éventuellement substitué, hétérocycloalkylhétéroaryle fusionné éventuellement substitué, hétéroarylhétéroaryle fusionné éventuellement substitué, ou cycloalkylhétérocycloalkyle fusionné,
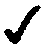 m est un entier compris inclusivement entre 1 et 6,
étant entendu que :
- le terme alkyle désigne un groupement linéaire ou ramifié, contenant de 1 à 6 atomes de carbone,
- le terme hétéroalkyle désigne un groupement alkyle dont un atome de carbone a été remplacé par un hétéroatome choisi parmi azote, oxygène et soufre,
- le terme alkylène désigne un groupement bivalent linéaire ou ramifié, contenant de 1 à 6 atomes de carbone,
- le terme alkénylène désigne un groupement bivalent linéaire ou ramifié, contenant de 2 à 6 atomes de carbone et de 1 à 3 doubles liaisons,
- le terme alkynylène désigne un groupement bivalent linéaire ou ramifié, contenant de 2 à 6 atomes de carbone et de 1 à 3 triples liaisons,
- le terme cycloalkyle désigne un groupement cyclique saturé contenant de 3 à 8 atomes de carbone,
- le terme cycloalkylène désigne un groupement bivalent cyclique saturé contenant de 3 à 8 atomes de carbone,
- le terme hétérocycloalkyle désigne un groupement cyclique saturé de 5 à 7 chaînons contenant de 1 à 3 hétéroatomes choisis parmi azote, oxygène et soufre,
- le terme aryle désigne un groupement phényle ou naphtyle,
- le terme hétéroaryle désigne un groupement mono ou bicyclique, insaturé ou partiellement insaturé, de 5 à 11 chaînons, contenant de 1 à 5 hétéroatomes choisis parmi azote, oxygène et soufre,
- le terme arylhétéroaryle fusionné représente un groupement polycyclique constitué d'un groupement aryle et d'un groupement hétéroaryle tels que définis précédemment et accolés par l'une quelconque de leur liaison,
- le terme arylhétérocycloalkyle fusionné représente un groupement bi- ou tricyclique constitué d'un groupement aryle et d'un groupement hétérocycloalkyle tels que définis précédemment et accolés par l'une quelconque de leur liaison,
- le terme hétéroarylhétérocycloalkyle fusionné représente un groupement bi- ou tricyclique constitué d'un groupement hétéroaryle et d'un groupement hétérocycloalkyle tels que définis précédemment et accolés par l'une quelconque de leur liaison,
- le terme hétérocycloalkylhétéroaryle fusionné représente un groupement bi- ou tricyclique constitué d'un groupement hétéroaryle et d'un groupement hétérocycloalkyle tels que définis précédemment et accolés par l'une quelconque de leur liaison,
- le terme hétéroarylhétéroaryle fusionné représente un groupement polycyclique constitué de deux groupements hétéroaryles tels que définis précédemment et accolés par l'une quelconque de leur liaison,
- le terme cycloalkylhétérocycloalkyle fusionné représente un groupement bicyclique constitué d'un groupement cycloalkyle et du groupement hétérocycloalkyle tels que définis précédemment et accolés par l'une quelconque de leur liaison,
- la terminaison "-ène" signifie que le groupement concerné est un radical bivalent possédant la même définition que le radical de base,
- l'expression "éventuellement substitué" associée aux groupements hétérocycloalkyle, aryle, arylalkyle, hétéroaryle, arylhétéroaryle fusionné, hétéroarylhétérocycloalkyle fusionné, hétéroarylhétéroaryle fusionné, et arylhétérocycloalkyle fusionné, signifie que ces groupements sont non substitués ou substitués par un ou plusieurs atomes d'halogène ou groupements alkyle, alkoxy, hydroxy, mercapto, cyano, amino (éventuellement substitué par un ou deux groupements alkyle), nitro, carboxy, alkoxycarbonyle, aminocarbonyle (éventuellement substitué par un ou deux groupements alkyle), étant entendu que les groupements hétéroaryle et hétérocycloalkyle peuvent en plus être substitués par un groupement oxo,
leurs énantiomères, diastéréoisomères, ainsi que leurs sels d'addition à un acide ou à une base pharmaceutiquement acceptable.

2. Composés de formule (I) selon la revendication 1 pour lesquels R₅ représente un groupement -CH₂-COOR₆, leurs énantiomères, diastéréoisomères ainsi que leurs sels d'addition à un acide ou à une base pharmaceutiquement acceptable.

3. Composés de formule (I) selon la revendication 1 pour lesquels X est choisi parmi -CO-NR₆-X₁, -NR₆-CO-X₁ et -O-X₁, leurs énantiomères, diastéréoisomères ainsi que leurs sels d'addition à un acide ou à une base pharmaceutiquement acceptable.

4. Composés de formule (I) selon la revendication 1 pour lesquels Y représente un groupement Y₁ ou Y₁-Y₂-Y₁, leurs énantiomères, diastéréoisomères ainsi que leurs sels d'addition à un acide ou à une base pharmaceutiquement acceptable.

5. Composés de formule (I) selon la revendication 1 pour lesquels Z représente un groupement hétéroaryle, hétérocycloalkyle, arylhétéroaryle fusionné ou hétérocycloalkylhétéroaryle fusionné, leurs énantiomères, diastéréoisomères ainsi que leurs sels d'addition à un acide ou à une base pharmaceutiquement acceptable.

6. Composés de formule (I) selon la revendication 1 pour lesquels Z représente un groupement Z₁₀-NR₆, leurs énantiomères, diastéréoisomères ainsi que leurs sels d'addition à un acide ou à une base pharmaceutiquement acceptable.

7. Composés de formule (I) selon la revendication 1 pour lesquels G représente un groupement phényle, G₁ et G₂ étant chacun un atome de carbone, R₅ représente un groupement -CH₂-COOR₆, R₆ étant choisi parmi un atome d'hydrogène et un groupement alkyle, T₂ représente une liaison, T₁ représente un groupement choisi parmi -CH=CH-, et =CH-CH₂-, A représente un groupement -CH₂-, ou =CH- et W représente un groupement -CH- ou -C=, leurs énantiomères, diastéréoisomères ainsi que leurs sels d'addition à un acide ou à une base pharmaceutiquement acceptable.

8. Composés de formule (I) selon la revendication 7 pour lesquels X est choisi parmi les groupements -CO-NR₆-X₁-, -NR₆-CO-X₁-, et -O-X₁-, X₁ étant un groupement méthylène, Y représente un groupement -Y₁- ou -Y₁-Y₂-Y₁ dans lesquels Y₁ est un groupement alkylène et Y₂ représente un groupement arylène, et Z représente un groupement hétéroaryle, hétérocycloalkyle, arylhétéroaryle fusionné, hétérocycloalkylhétéroaryle fusionné ou Z₁₀-NR₆ dans lequel Z₁₀ représente un groupement choisi parmi les groupements hétéroaryle, hétérocycloalkyle, arylhétéroaryle fusionné et hétérocycloalkylhétéroaryle fusionné, R₆ étant un atome d'hydrogène, leurs énantiomères, diastéréoisomères ainsi que leurs sels d'addition à un acide ou à une base pharmaceutiquement acceptable.

9. Composé de formule (I) qui est l'acide [7-({[4-(2-pyridinylamino)butanoyl]amino} méthyl)-6,9-dihydro-5*H*-benzo[a]cycloheptèn-5-yl]acétique.

10. Composé de formule (I) qui est l'acide [7-({[5-(2-pyridinylamino)pentanoyl]amino} méthyl)-6,9-dihydro-5*H*-benzo[a]cycloheptèn-5-yl]acétique, chlorhydrate.

11. Composé de formule (I) qui est l'acide [7-(2-oxo-2-{[3-(2-pyridinylamino)propyl] amino}éthyl)-5*H*-benzo[a]cycloheptèn-5-yl]acétique.

12. Composé de formule (I) qui est l'acide [7-({[4-(4,5-dihydro-1*H*-imidazol-2-ylamino) butanoyl]amino}méthyl)-6,9-dihydro-5*H*-benzo[a]cycloheptèn-5-yl]acétique, chlorhydrate.

13. Composé de formule (I) qui est l'acide [7-({[4-(2-pyridinylamino)butanoyl]amino} méthyl)-5*H*-benzo[a]cycloheptèn-5-yl]acétique.

14. Composé de formule (I) qui est l'acide [7-({[4-(1*H*-benzimidazol-2-ylamino)butanoyl] amino}méthyl)-5*H*-benzo[a]cycloheptèn-5-yl]acétique.

15. Composé de formule (I) qui est l'acide [7-({[4-(1*H*-benzimidazol-2-ylamino)butanoyl] amino}méthyl)-6,9-dihydro-5*H*-benzo[a]cycloheptèn-5-yl]acétique.

16. Composé de formule (I) qui est l'acide [7-({[4-(1,4,5,6-tétrahydro-2-pyrimidinylamino) butanoyl]amino}méthyl)-6,9-dihydro-5*H*-benzo[a]cycloheptèn-5-yl]acétique, chlorhydrate.

17. Composé de formule (I) qui est l'acide [7-({[4-(5,6,7,8-tétrahydro[1,8]naphtyridin-2-yl) butanoyl]amino}méthyl)-6,9-dihydro-5*H*-benzo[a]cycloheptén-5-yl]acétique, chlorhydrate.

18. Procédé de préparation des composés de formule (I) selon la revendication 1 **caractérisé en ce que** l'on utilise comme produit de départ un composé de formule (II/a): 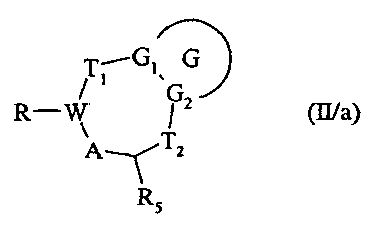 dans laquelle G, G₁, G₂, T₁, T₂ et R₅ ont la même signification que dans la formule (I), W et A sont tels que définis précédemment, et R représente un groupement CHO, CN ou AlkOOC-CH=,
qui lorsque R représente un groupement formyle, est soumis à une réaction de Wittig ou d'Homer Emons de façon à homologuer la chaîne portant la fonction aldéhyde, en utilisant le réactif approprié, pour conduire au composé de formule (III/a) : 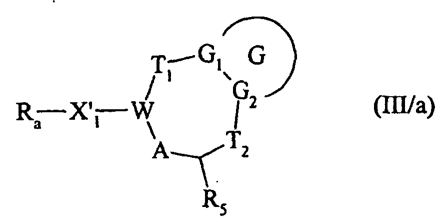 dans laquelle G, G₁, G₂, T₁, T₂, R₅, A et W sont tels que définis précédemment, X'₁ représente un groupement alkylène ou alkénylène de deux à six atomes de carbone et Rₐ représente un groupement cyano, formyle, ou alkoxycarbonyle en fonction du réactif choisi,
composé (III/a) qui, avec les composés de formule (III/b) : 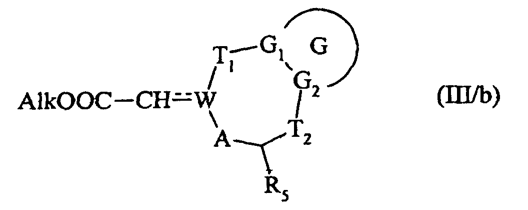 cas particulier des composés de formule (II/a) pour lequel G, G₁, G₂, T₁, T₂, R₅, W et A sont tels que définis précédemment, et Alk représente un groupement alkyle, les pointillés indiquant la présence éventuelle d'une double liaison,
forment l'ensemble des composés de formule (III) : 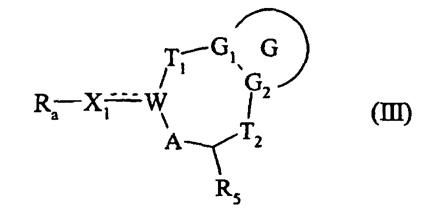 dans laquelle G, G₁, G₂, T₁, T₂, W, A et Rₐ sont tels que définis précédemment, et X₁ a la même signification que dans la formule (I),
composé (III) dont le groupement Rₐ est soit réduit en alcool, ou déprotégé lorsqu'il représente un groupement hydroxy masqué, pour conduire au composé de formule (IV) : 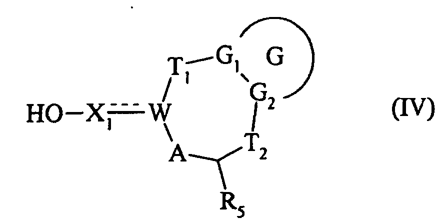 dans laquelle G, G₁, G₂, T₁, T₂, R₅, W, A et X₁ sont tels que définis dans la formule (I),
qui est soumis à une réaction d'halogénation, pour conduire au composé de formule (V) : 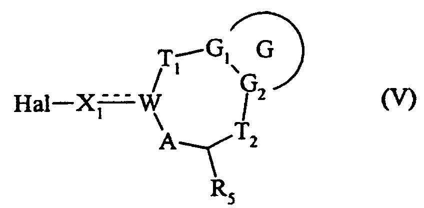 dans laquelle G, G₁, G₂, T₁, T₂, R₅, A, W et X₁ sont tels que définis précédemment, et Hal représente un atome d'halogène,
qui est traité, en milieu basique, par un composé de formule Z-Y-OH dans laquelle Z et Y ont la même signification que dans la formule (I), pour conduire au composé de formule (I/a) : 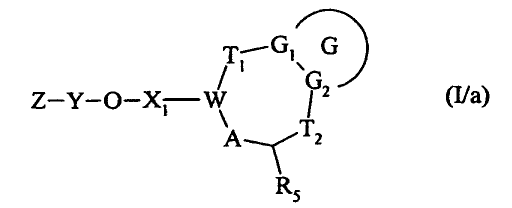 cas particulier des composés de formule (I) pour lequel G, G₁, G₂, T₁, T₂, R₅, X₁, W, A, Y et Z sont tels que définis dans la formule (I),
ou bien qui est traité, en milieu basique, par un composé de formule Z-Y-SH, dans laquelle Z et Y ont la même signification que dans la formule (I), pour conduire au composé de formule (I/b) : 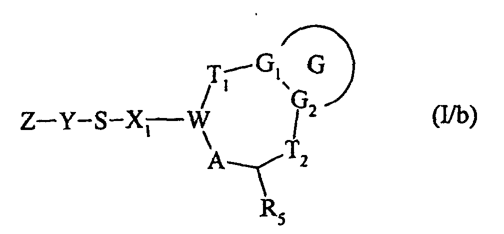 cas particulier des composés de formule (I) pour lequel G, G₁, G₂, T₁, T₂, R₅, X₁, W, A, Y et Z sont tels que définis dans la formule (I),
dont l'atome de soufre peut être oxydé pour conduire un composé de formule (I/c) : 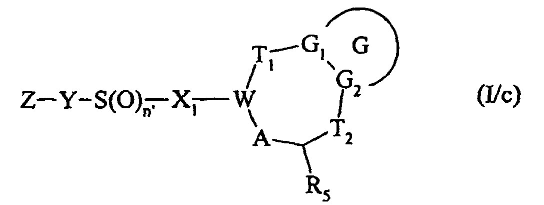 cas particulier des composés de formule (I) pour lequel G, G₁, G₂, T₁, T₂, R₅, X₁, W, A, Y et Z sont tels que définis dans la formule (I), et n' représente un entier égal à 1 ou 2,
ou bien,
composé de formule (V), qui soumis à l'action d'une imide cyclique en milieu basique, suivi d'un traitement par l'hydrazine en milieu alcoolique, conduit à l'amine de formule (VI) : 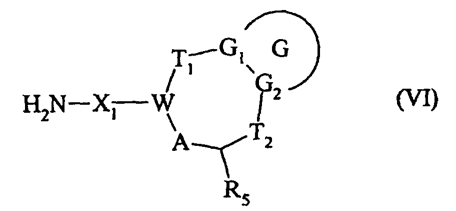 dans laquelle G, G₁, G₂, T₁, T₂, R₅, X₁, A et W sont tels que définis précédemment,
composé de formule (VI) pouvant par ailleurs être obtenu directement lorsque X₁ représente un groupement CH₂, par réduction du composé de formule (II/a) dans laquelle R représente un groupemnet CN,
composé de formule (VI) qui est traité par un composé de formule (VII) : 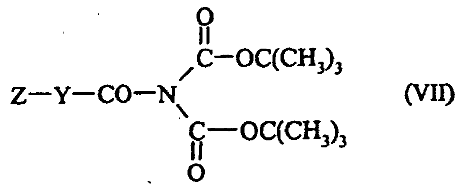 dans laquelle Z et Y sont tels que définis dans la formule (I),
pour conduire au composé de formula (I/d) : 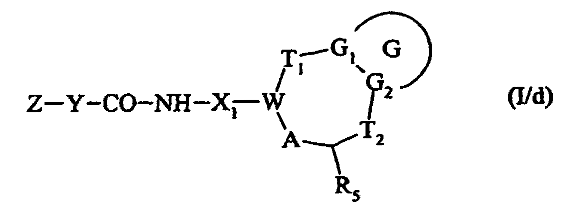 cas particulier des composés de formule (I) pour lequel G, G₁, G₂, T₁, T₂, R₅, X₁, A, W, Z et Y sont tels que définis dans la formule (I),
ou bien qui est traité par un chlorure de sulfonyle de formule Z-Y-SO₂-Cl, dans laquelle Z et Y sont tels que définis dans la formule (I), pour conduire à un composé de formule (I/e) : 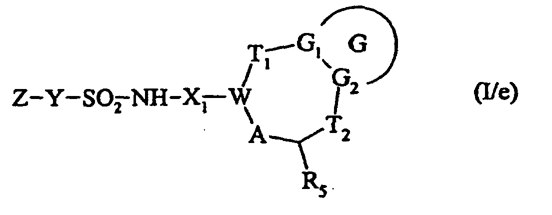 cas particulier des composés de formule (I) pour lequel G, G₁, G₂, T₁, T₂, R₅, X₁, A, W, Z et Y sont tels que définis précédemment dans la formule (I),
- ou bien, composé de formule (III), dont le groupement Rₐ est transformé en acide correspondant pour conduire au composé de formule (VIII) : 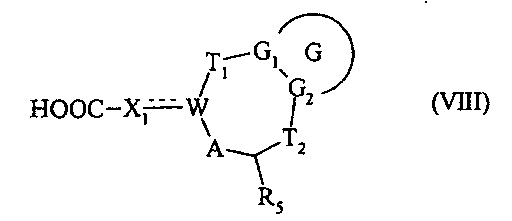 dans laquelle G, G₁, G₂, T₁, T₂, R₅, W, A et X₁ sont tels que définis dans la formule (I),
qui est soit soumis à l'action d'une amine de formule Z-Y-NH₂ ou du sel correspondant, pour conduire à un composé de formule (I/f) : 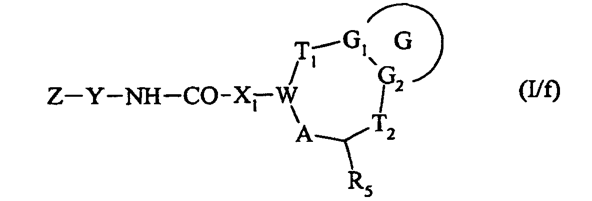 cas particulier des composés de formule (I) pour lequel G, G₁, G₂, T₁, T₂, R₅, A, W, X₁, Z et Y sont tels que définis dans la formule (I),
soit à l'action d'une amine de formule : 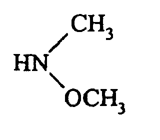 pour conduire au composé de formule (IX) : 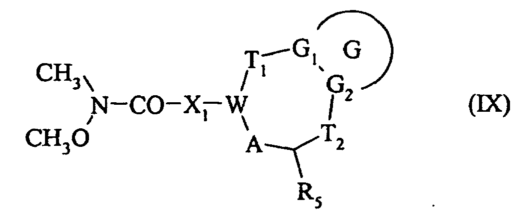 dans laquelle G, G₁, G₂, T₁, T₂, R₅, A, W et X₁ sont tels que définis précédemment,
qui est ensuite soumis à l'action d'un dérivé lithié de formule Z-Y-Li dans laquelle Y et Z sont tels que définis dans la formule (I), pour conduire au composé de formule (I/g) : 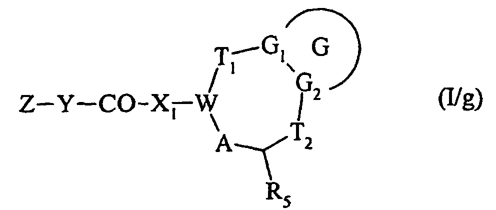 cas particulier des composés de formule (I) pour lequel G, G₁, G₂, T₁, T₂, X₁, W, A, Z et Y sont tels que définis dans la formule (I),
composés (I/a) à (I/g) formant la totalité des composés de formule (I),
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

19. Procédé de préparation des composés de formule (I) selon la revendication 1 pour lesquels Z représente un groupement Z₁₀NR₆, **caractérisé en ce que** l'on utilise comme produit de départ un composé de formule (X) : 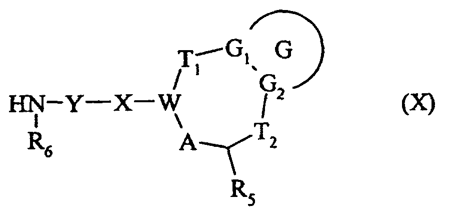 dans laquelle Y, X, W, T₁, G, G₁, G₂, T₂, A, R₅ et R₆ sont tels que définis dans la formule (I),
que l'on condense, en milieu basique ou en présence d'un catalyseur, sur un composé de formule (XI) : 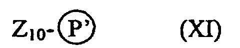 dans laquelle Z₁₀ est tel que défini dans la formule (I) et 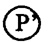 représente un groupe partant (par exemple un atome d'halogène, un groupement tosyle ou un groupement méthylthio ou thioxo),
pour conduire au composé de formule (I/i), cas particulier des composés de formule (I) : 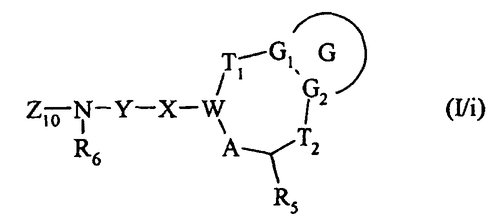 dans laquelle Z₁₀, R₆ Y, X, W, A, T₁, T₂, G₁, G₂, R₅ et G sont tels que définis précédemment,
- qui peut être, le cas échéant, purifié selon une technique classique de purification,
- dont on sépare, le cas échéant, les stéréoisomères selon une technique classique de séparation,
- que l'on transforme, si on le souhaite, en ses sels d'addition à un acide ou à une base pharmaceutiquement acceptable.

20. Compositions pharmaceutiques contenant comme principe actif au moins un composé selon l'une quelconque des revendications 1 à 17 seul ou en combinaison avec un ou plusieurs excipients ou véhicules inertes, non toxiques, pharmaceutiquement acceptables.

21. Compositions pharmaceutiques selon la revendication 20 contenant au moins un principe actif selon l'une quelconque des revendications 1 à 17 utiles dans la préparation de médicaments utiles comme antagonistes des récepteurs de la vitronectine, dans le traitement des maladies cardiovasculaires, des maladies inflammatoires, du cancer, de l'ostéoporose, de l'arthrite rhumatoïde, du psoriasis et des rétinopathies.

22. Compositions pharmaceutiques selon la revendication 20 contenant au moins un principe actif selon l'une quelconque des revendications 1 à 17 utiles dans la préparation de médicaments utiles comme inhibiteurs de croissance tumorale et des formations de métastases dans le traitement du cancer.

## Patentansprüche

1. Verbindungen der Formel (I): 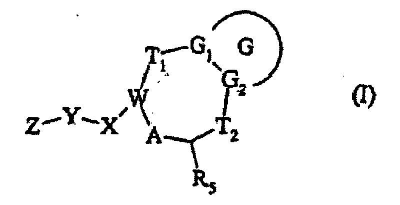 in der:
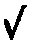 G eine Phenylgruppe bedeutet,
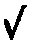 G₁ und G₂ ein Kohlenstoffatom darstellen,
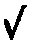 -T₁- eine Gruppe ausgewählt aus -CH₂-CH₂-, -CH=CH-, =CH-CH₂- und -T₂eine Bindung bedeuten,
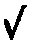 R₅ eine Gruppe -(CH₂)ₘ-COOR₆ darstellt,
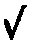 R₆ ein Wasserstoffatom, eine Alkylgruppe, eine gegebenenfalls substituierte Arylgruppe oder eine gegebenenfalls substituierte Arylalkylgruppe bedeutet,
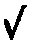 -W- eine Gruppe -CH-, =C- oder -C= und -A- eine Gruppe -CH₂- oder =CHbedeuten,
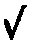 -X- eine Gruppe bedeutet ausgewählt aus -CO-X₁-, -CO-NR₆-X₁-, -NR₆-CO-X₁-, -O-X₁-, -SO₂-NR₆-X₁- und -S(O)ₙ-X₁-, worin n zwischen 0 und 2 einschließlich und X₁ eine Alkylengruppe bedeuten,
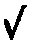 -Y- eine Gruppe bedeutet ausgewählt aus -Y₁-, -Y₂-Y₁- und -Y₁-Y₂-Y₁-, worin Y₁ eine Alkylengruppe, Alkenylengruppe oder Alkinylengruppe und Y₂ eine Arylengruppe, Heteroarylengruppe, Cycloalkylengruppe oder Heterocycloalkylengruppe darstellen,
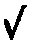 Z- eine Gruppe bedeutet ausgewählt aus Z₁-, Z₁₀-NR₆- und Z₁₀-NR₆-CO-, worin Z₁₀ eine Alkylgruppe oder Z₁ bedeutet, und Z₁ eine Gruppe bedeutet ausgewählt aus Z₂-, 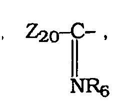 Z₂₀-NR₆- und Z₂₀-NR₆-CO-, in denen Z₂₀ eine Alkylgruppe, Heteroalkylgruppe oder Z₂ bedeutet, und worin Z₂ eine gegebenenfalls substituierte Heteroarylgruppe, gegebenenfalls substituierte Heterocycloalkylgruppe, gegebenenfalls substituierte Heteroarylalkylgruppe, gegebenenfalls substituierte Heterocycloalkylalkylgruppe, gegebenenfalls substituierte kondensierte Arylheteroarylgruppe, gegebenenfalls substituierte kondensierte Arylheterocycloalkylgruppe, gegebenenfalls substituierte kondensierte Heteroarylheterocycloalkylgruppe, gegebenenfalls substituierte kondensierte Heterocycloalkylheteroarylgruppe, gegebenenfalls substituierte kondensierte Heteroarylheteroarylgruppe oder kondensierte Cycloalkylheterocycloalkylgruppe darstellt,
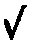 m eine ganze Zahl mit einem Wert zwischen 1 und 6 einschließlich bedeutet,
mit der Maßgabe, daß:
- der Begriff Alkyl für eine geradkettige oder verzweigte Gruppe steht, die 1 bis 6 Kohlenstoffatome enthält,
- der Begriff Heteroalkyl für eine Alkylgruppe steht, bei der ein Kohlenstoffatom durch ein Heteroatom ausgewählt aus Stickstoff, Sauerstoff und Schwefel ersetzt ist,
- der Begriff Alkylen für eine geradkettige oder verzweigte zweiwertige Gruppe steht, die 1 bis 6 Kohlenstoffatome enthält.
- der Begriff Alkenylen für eine geradkettige oder verzweigte zweiwertige Gruppe steht, die 2 bis 6 Kohlenstoffatome und 1 bis 3 Doppelbindungen enthält,
- der Begriff Alkinylen für eine geradkettige oder verzweigte zweiwertige Gruppe steht, die 2 bis 6 Kohlenstoffatome und 1 bis 3 Dreifachbindungen enthält,
- der Begriff Cycloalkyl für eine gesättigte cyclische Gruppe steht, die 3 bis 8 Kohlenstoffatome enthält,
- der Begriff Cycloalkylen für eine gesättigte zweiwertige cyclische Gruppe steht, die 3 bis 8 Kohlenstoffatome enthält,
- der Begriff Heterocycloalkyl für eine gesättigte cyclische Gruppe mit 5 bis 7 Kettengliedern steht, die 1 bis 3 Heteroatome ausgewählt aus Stickstoff, Sauerstoff und Schwefel enthält,
- der Begriff Aryl für eine Phenyl- oder Naphthylgruppe steht,
- der Begriff Heteroaryl für eine mono- oder bicyclische, gesättigte oder teilweise ungesättigte Gruppe mit 5 bis 11 Kettengliedern steht, die 1 bis 5 Heteroatome ausgewählt aus Stickstoff, Sauerstoff und Schwefel enthält,
- der Begriff kondensiertes Arylheteroaryl für eine polycyclische Gruppe steht, die aus einer Arylgruppe und einer Heteroarylgruppe gebildet ist, wie sie oben definiert worden sind, und die durch irgendeine ihrer Bindungen verknüpft sind,
- der Begriff kondensiertes Arylheterocycloalkyl für eine bi- oder tricyclische Gruppe steht, die aus einer Arylgruppe und einer Heterocycloalkylgruppe, wie sie oben definiet worden sind, gebildet ist und die durch irgendeine ihrer Bindungen verknüpft sind,
- der Begriff kondensiertes Heteroarylheterocycloalkyl für eine bi- oder tricyclische Gruppe steht, die aus einer Heteroarylgruppe und einer Heterocycloalkylgruppe, wie sie oben definiert worden sind, gebildet ist und die durch irgendeine ihrer Bindungen verknüpft sind,
- der Begriff kondensiertes Heterocycloalkylheteroaryl für eine bi- oder tricyclische Gruppe steht, die aus einer Heteroarylgruppe und einer Heterocycloalkylgruppe, wie sie oben definiert worden sind, gebildet ist und die durch irgendeine ihrer Bindungen verknüpft sind,
- der Begriff kondensiertes Heteroarylheteroaryl für eine polycyclische Gruppe steht, die aus zwei Heteroarylgruppen, wie sie oben definiet worden sind, gebildet ist und die durch irgendeine ihrer Bindungen verknüpft sind,
- der Begriff kondensiertes Cycloalkylheterocycloalkyl für eine bicyclische Gruppe steht, die aus einer Cycloalkylgruppe und einer Heterocycloalkylgruppe, wie sie oben definiert worden sind, gebildet ist und durch irgendeine ihrer Bindungen verknüpft sind,
- der Endung "-en" bedeutet, daß die betreffende Gruppe ein zweiwertiger Rest ist, der die gleiche Definition besitzt wie der Grundrest,
- der Begriff "gegebenenfalls substituiert" im Hinblick auf Heterocycloalkyl-, Aryl-, Arylalkyl-, Heteroaryl-, kondensierte Arylheteroaryl-, kondensierte Heteroarylheterocycloalkyl-, kondensierte Heteroarylheteroaryl- und kondensierte Arylheterocycloalkylgruppen bedeutet, daß diese Gruppen nicht substituiert sind oder durch ein oder mehrere Halogenatome oder Alkyl-, Alkoxy-, Hydroxy-, Mercapto-, Cyano-, (gegebenenfalls durch eine oder zwei Alkylgruppen substituierte) Amino-, Nitro-, Carboxy-, Alkoxycarbonyl-, (gegebenenfalls durch eine oder zwei Alkylgruppen substituierte) Aminocarbonylgruppen substituiert sind, mit der Maßgabe, daß die Heteroaryl- und Heterocycloalkylgruppen zusätzlich .durch eine Oxogruppe substituiert sein können,
deren Enantiomere, Diastereoisomere sowie deren Additionssalze mit einer pharmazeutisch annehmbaren Säure oder Base.

2. Verbindungen der Formel (I) nach Anspruch 1, worin R₅ eine Gruppe -CH₂-COOR₆ bedeutet, deren Enantiomere, Diastereoisomere sowie deren Additionssalze mit einer pharmazeutisch annehmbaren Säure oder Base.

3. Verbindungen der Formel (I) nach Anspruch 1, worin X ausgewählt ist aus -CO-NR₆-X₁, -NR₆-CO-X₁ und -O-X₁, deren Enantiomere, Diastereoisomere sowie deren Additionssalze mit einer pharmazeutisch annehmbaren Säure oder Base.

4. Verbindungen der Formel (I) nach Anspruch 1, worin Y eine Gruppe Y₁ oder Y₁-Y₂-Y₁ bedeutet, deren Enantiomere, Diastereoisomere sowie deren Additionssalze mit einer pharmazeutisch annehmbaren Säure oder Base.

5. Verbindungen der Formel (I) nach Anspruch 1, worin Z eine Heteroaryl-, Heterocycloalkyl-, kondensierte Arylheteroaryl- oder kondensierte Heterocycloalkylheteroarylgruppe bedeutet, deren Enantiomere, Diastereoisomere sowie deren Additionssalze mit einer pharmazeutisch annehmbaren Säure oder Base.

6. Verbindungen der Formel (I) nach Anspruch I, worin Z eine Gruppe Z₁₀-NR₆ bedeutet, deren Enantiomere, Diastereoisomere sowie deren Additionssalze mit einer pharmazeutisch annehmbaren Säure oder Base.

7. Verbindungen der Formel (I) nach Anspruch 1, worin G eine Phenylgruppe bedeutet, G₁ und G₂ jeweils ein Kohlenstoffatom darstellen, R₅ eine Gruppe -CH₂-COOR₆ bedeutet, R₆ ausgewählt ist aus einem Wasserstoffatom und einer Alkylgruppe, T₂ eine Bindung bedeutet, T₁ eine Gruppe bedeutet ausgewählt aus - CH=CH- und =CH-CH₂-, A eine Gruppe -CH₂- oder =CH- darstellt und W eine Gruppe -CH- oder -C= darstellt, deren Enantiomere, Diastereoisomere sowie deren Additionssalze mit einer pharmazeutisch annehmbaren Säure oder Base.

8. Verbindungen der Formel (I) nach Anspruch 7, worin X ausgewählt ist aus den Gruppen -CO-NR₆-X₁-, -NR₆-CO-X₁- und -O-X₁-, worin X₁ eine Methylengruppe darstellt, Y eine Gruppe -Y₁- oder -Y₁-Y₂-Y₁ darstellt, worinY₁ eine Alkylengruppe darstellt und Y₂ eine Arylengruppe bedeutet, und Z eine Heteroaryl-, Heterocycloalkyl-, kondensierte Arylheteroaryl-, kondensierte Heterocycloalkylheteroarylgruppe oder eine Gruppe Z₁₀-NR₆ darstellt, worin Z₁₀ eine Gruppe bedeutet ausgewählt aus Heteroaryl-, Heterocycloalkyl-, kondensierten Arylheteroarylund kondensierten Heterocycloalkylheteroarylgruppen und R₆ ein Wasserstoffatom darstellt, deren Enantiomere, Diastereoisomere sowie deren Additionssalze mit einer pharmazeutisch annehmbaren Säure oder Base.

9. Verbindung der Formel (I), nämlich [7-({[4-(2-Pyridinylamino)-butanoyl]-amino}-methyl)-6,9-dihydro-5*H*-benzo[a]cyclohepten-5-yl]-essigsäure.

10. Verbindung der Formel (I), nämlich [7-({[5-(2-Pyridinylamino]-pentanoyl]-amino}-methyl)-6,9-dihydro-5*H*-benzo[a]cyclohepten-5-yl]-essigsäure, Hydrochlorid.

11. Verbindung der Formel (I), nämlich [7-(2-Oxo-2-{[3-(2-pyridinylamino)-propyl]-amino}-ethyl)-5*H*-benzo[a]cyclohepten-5-yl]-essigsäure.

12. Verbindung der Formel (I), nämlich [7-({[4-(4,5-Dihydro-1*H*-imidazol-2-ylamino)-butanoyl]-amino}-methyl)-6,9-dihydro-5*H*-benzo[a]cyclohepten-5-yl]-essigsäure, Hydrochlorid.

13. Verbindung der Formel (I), nämlich [7-({[4-(2-Pyridinylamino)-butanoyl]-amino}-methyl)-5*H*-benzo[a]cyclohepten-5-yl]-essigsäure.

14. Verbindung der Formel (I), nämlich [7-({[4-(1H-Benzimidazol-2-ylamino)-butanoyl]-amino}-methyl)-5*H*-benzo[a]cyclohepten-5-yl]-essigsäure.

15. Verbindung der Formel (I), nämlich [7-({[4-(1*H*-Benzimidazol-2-ylamino)-butanoyl]-amino}-methyl)-6,9-dihydro-5*H*-benzo[a]cyclohepten-5-yl]-essigsäure.

16. Verbindung der Formel (I), nämlich [7-({[4-(1,4,5,6-Tetrahydro-2-pyrimidinylamino)-butanoyl]-amino}-methyl)-6,9-dihydro-5H-benzo[a]cyclohepten-5-yl]-essigsäure, Hydrochlorid.

17. Verbindung der Formel (I), nämlich [7-({[4-(5,6,7,8-Tetrahydro[1,8]naphthyridin-2-yl)-butanoyl]-amino}-methyl)-6,9-dihydro-5*H*-benzo[a]cyclohepten-5-yl]-essigsäure, Hydrochlorid.

18. Verfahren zur Herstellung der Verbindungen der Formel (I) nach Anspruch 1, **dadurch gekennzeichnet, daß** man aus Ausgangsprodukt eine Verbindung der Formel (II/a) verwendet: 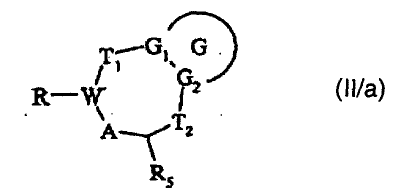 in der G, G₁, G₂, T₁, T₂ und R₅ die bezüglich der Formel (I) angegebenen Bedeutungen besitzen, W und A die oben angegebenen Bedeutungen besitzen und R eine Gruppe CHO, CN oder AlkOOC-CH= darstellt,
welche man unter Verwendung eines geeigneten Reagens einer Wittig- oder Horner Emons-Reaktion unterwirft, um die die Aldehydfunktion tragende Kette zu homologisieren, zur Bildung der Verbindung der Formel (III/a): 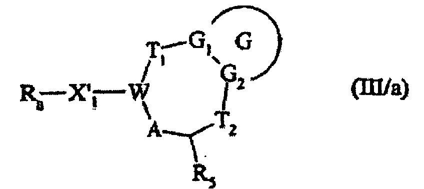 in der G, G₁, G₂, T₁, T₂, R₅, A und W die oben angegebenen Bedeutungen besitzen, X'₁ eine Alkylen- oder Alkenylengruppe mit zwei bis sechs Kohlenstoffatomen darstellt und Rₐ in Abhängigkeit von dem ausgewählten Reagens eine Cyano-, Formyl- oder Alkoxycarbonylgruppe bedeutet,
welche Verbindung (III/a) zusammen mit den Verbindungen der Formel (III/b): 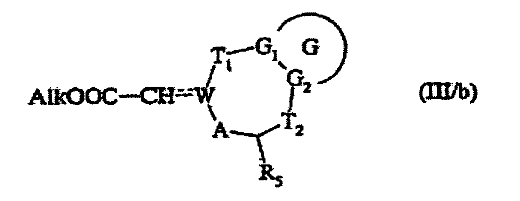 einem Sonderfall der Verbindungen der Formel (II/a), worin G, G₁, G₂, T₁, T₂, R₅, W und A die oben angegebenen Bedeutungen besitzen und Alk eine Alkylgruppe darstellt und die gepunktete Linie bedeutet, daß gegebenenfalls eine Doppelbindung vorliegt,
gemeinsam die Verbindungen der Formel (III) bildet: 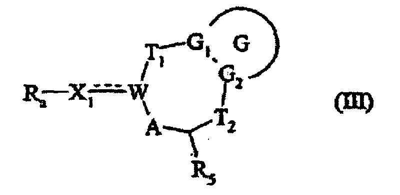 in der G, G₁, G₂, T₁, T₂, W, A und Rₐ die oben angegebenen Bedeutungen besitzen und X₁ die bezüglich der Formel (I) angegebene Bedeutung aufweist,
von welcher Verbindung (III) man die Gruppe Rₐ entweder zu einem Alkohol reduziert oder von der Schutzgruppe befreit, wenn es sich um eine maskierte Hydroxygruppe handelt, zur Bildung der Verbindung der Formel (IV): 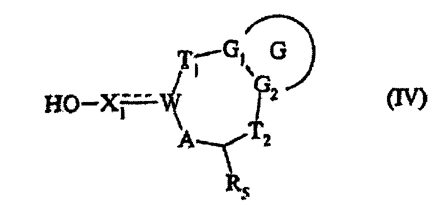 in der G, G₁, G₂, T₁, T₂, R₅, W, A und X₁ die bezüglich der Formel (I) angegebenen Bedeutungen besitzen,
welche man einer Halogenierungsreaktion unterwirft zur Bildung der Verbindung der Formel (V): 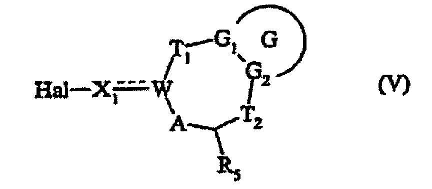 in der G, G₁, G₂, T₁, T₂, R₅, A, W und X₁ die oben angegebenen Bedeutungen besitzen und Hal für ein Halogenatom steht,
welche man in basischem Medium mit einer Verbindung der Formel Z-Y-OH, in der Z und Y die bezüglichd er Formel (I) angegebenen Bedeutungen besitzen, behandelt zur Bildung der Verbindung der Formel (I/a): 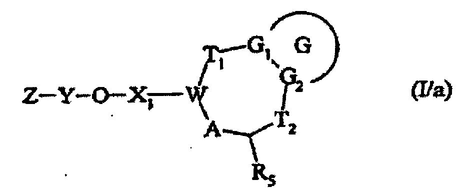 einem Sonderfall der Verbindungen der Formel (I), in der G, G₁, G₂, T₁, T₂, R₅, X₁, W, A, Y und Z die bezüglich der Formel (I) angegebenen Bedeutungen besitzen,
oder die man in basischem Medium mit einer Verbindung der Formel Z-Y-SH, in der Z und Y die bezüglich der Formel (I) angegebenen Bedeutungen besitzen, behandelt zur Bildung der Verbindung der Formel (I/b): 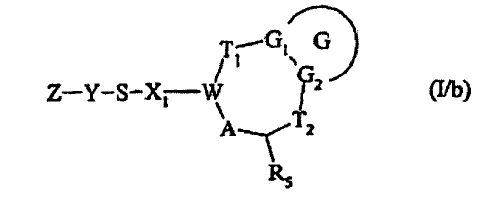 einem Sonderfall der Verbindungen der Formel (I), in der G, G₁, G₂, T₁, T₂, R₅, X₁, W, A, Y und Z die bezüglich der Formel (I) angegebenen Bedeutungen besitzen,
dessen Schwefelatom oxidiert werden kann zur Bildung einer Verbindung der Formel (I/c): 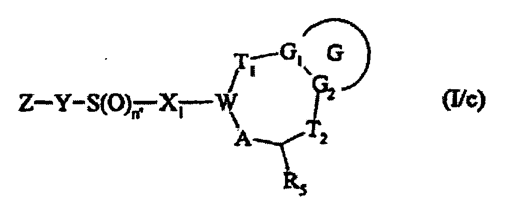 einem Sonderfall der Verbindungen der Formel (I), in der G, G₁, G₂, T₁, T₂, R₅, X₁, W, A, Y und Z die bezüglich der Formel (I) angegebenen Bedeutungen besitzen und n' eine ganze Zahl mit einem Wert von 1 oder 2 darstellt,
oder
man die Verbindung der Formel (V) der Einwirkung eines cyclischen Imids in basischem Medium gefolgt von einer Behandlung mit Piperazin in alkoholischem Medium unterzieht zur Bildung des Amins der Formel (VI): 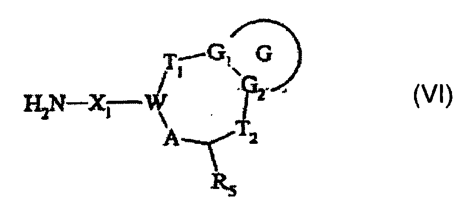 in der G, G₁, G₂, T₁, T₂, R₅, X₁, A und W die oben angegebenen Bedeutungen besitzen,
welche Verbindung der Formel (VI), wenn X₁ eine Gruppe CH₂ darstellt, auch direkt erhalten werden kann durch Reduktion der Verbindung der Formel (II/a), in der R eine Gruppe CN darstellt,
welche Verbindung der Formel (VI) mit einer Verbindung der Formel (VII): 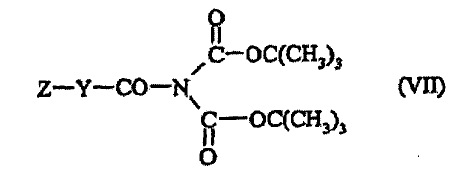 in der Z und Y die bezüglich der Formel (I) angegebenen Bedeutungen besitzen,
behandelt wird zur Bildung der Verbindung der Formel (I/d): 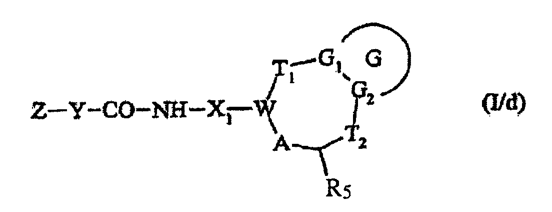 einem Sonderfall der Verbindungen der Formel (I), in der G, G₁, G₂, T₁, T₂, R₅, X₁, A, W, Z und Y die bezüglich der Formel (I) angegebenen Bedeutungen besitzen,
oder welche man mit einem Sulfonylchlorid der Formel Z-Y-SO₂-Cl, in der Z und Y die bezüglich der Formel (I) angegebenen Bedeutungen besitzen, behandelt zur Bildung einer Verbindung der Formel (I/e): 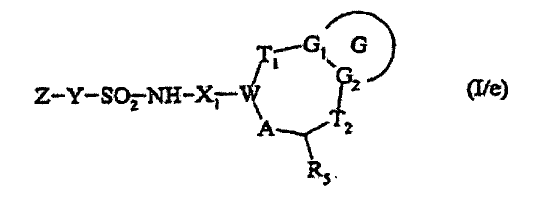 einem Sonderfall der Verbindungen der Formel (I), in der G, G₁, G₂, T₁, T₂, R₅, X₁, A, W, Z und Y die bezüglich der Formel (I) angegebenen Bedeutungen besitzen,
- oder man bei der Verbindung der Formel (III) die Gruppe Rₐ in die entsprechende Säure umwandelt zur Bildung der Verbindung der Formel (VIII): 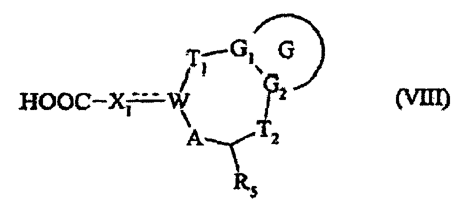 in der G, G₁, G₂, T₁, T₂, R₅, W, A und X₁ die bezüglich der Formel (I) angegebenen Bedeutungen besitzen.
welche man der Einwirkung eines Amins der Formel Z-Y-NH₂ oder des entsprechenden Salzes unterwirft zur Bildung einer Verbindung der Formel (I/f): 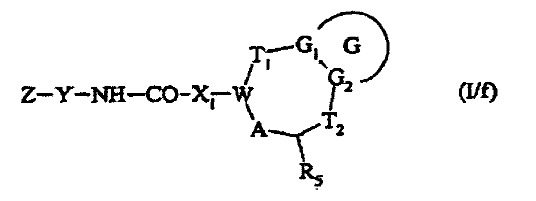 einem Sonderfall der Verbindungen der Formel (I), in der G, G₁, G₂, T₁, T₂, R₅, A, W, X₁, Z und Y die bezüglich der Formel (I) angegebenen Bedeutungen besitzen,
oder der Einwirkung eines Amins der Formel: 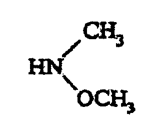 unterwirft zur Bildung der Verbindung der Formel (IX): 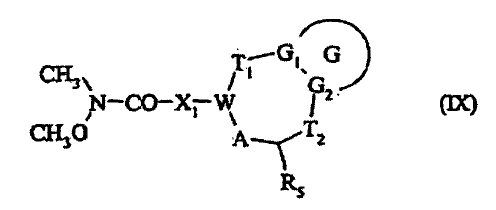 in der G, G₁, G₂, T₁, T₂, R₅, A, W und X₁ die oben angegebenen Bedeutungen besitzen,
welche anschließend der Einwirkung eines Lithiumderivats der Formel Z-Y-Li, in der Y und Z die bezüglich der Formel (I) angegebenen Bedeutungen besitzen, unterworfen wird, zur Bildung der Verbindung der Formel (I/g): 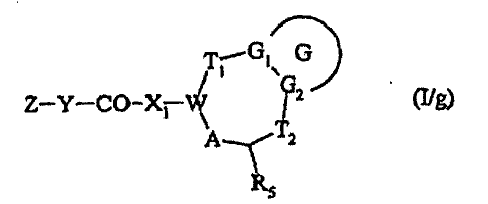 einem Sonderfall der Verbindungen der Formel (I), in der G, G₁, G₂, T₁, T₂, X₁, W, A, Z und Y die bezüglich der Formel (I) angegebenen Bedeutungen besitzen,
welche Verbindungen (I/a) bis (I/g) die Gesamtheit der Verbindungen der Formel (I) bilden,
- die gegebenenfalls mit Hilfe einer klassischen Reinigungsmethode gereinigt werden können,
- die man gegebenenfalls mit einer klassischen Trennmethode in ihre Stereoisomeren trennt,
- welche man gewünschtenfalls in ihre Additionssalze mit einer pharmazeutisch annehmbaren Säure oder Base überführt.

19. Verfahren zur Herstellung der Verbindungen der Formel (I) nach Anspruch 1, in der Z eine Gruppe Z₁₀NR₆ darstellt, **dadurch gekennzeichnet, daß** man als Ausgangsprodukt eine Verbindung der Formel (X) verwendet: 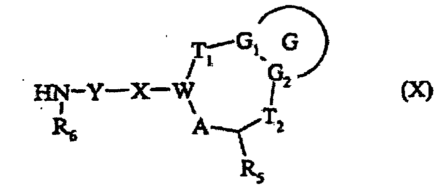 in der Y, X, W, T₁, G, G₁, G₂, T₂, A, R₅ und R₆ die bezüglich der Formel (I) angegebenen Bedeutungen besitzen,
welche man in basischem Medium oder in Gegenwart eines Katalysators mit einer Verbindung der Formel (XI) kondensiert: 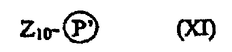 in der Z₁₀ die bezüglich der Formel (I) angegebenen Bedeutungen besitzt und 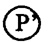 für eine austretende Gruppe steht (beispielsweise ein Halogenatom, eine Tosylgruppe oder eine Methylthio- oder Thioxogruppe),
zur Bildung der Verbindung der Formel (I/i), einem Sonderfall der Verbindungen der Formel (I): 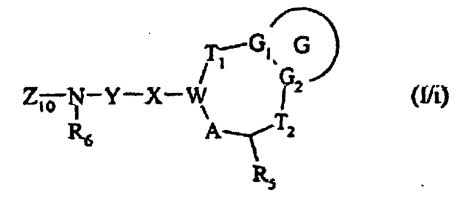 in der Z₁₀, R₆, Y, X, W, A, T₁, T₂, G₁, G₂, R₅ und G die oben angegebenen Bedeutungen besitzen,
- welche gegebenenfalls mit Hilfe einer klassischen Reinigungsmethode gereinigt werden können,
- welche man gegebenenfalls mit Hilfe einer klassischen Trennmethode in ihre Stereoisomeren trennt,
- welche man gewünschtenfalls in ihre Additionssalze mit einer pharmazeutisch annehmbaren Säure oder Base überführt.

20. Pharmazeutische Zubereitungen, enthaltend als Wirkstoff mindestens eine Verbindung nach einem der Ansprüche 1 bis 17 allein oder in Kombination mit einem oder mehreren inerten, nichttoxischen, pharmazeutisch annehmbaren Trägermaterialien oder Bindemitteln.

21. Pharmazeutische Zubereitungen nach Anspruch 20, enthaltend mindestens einen Wirkstoff nach einem der Ansprüche 1 bis 17 für die Herstellung von Arzneimitteln, die geeignet sind als Antagonisten der Rezeptoren von Vitronectin, für die Behandlung von kardiovaskulären Erkrankungen, von Entzündungserkrankungen, von Krebs, der Osteoporose, der rheumatoiden Arthritis, der Psoriasis und von Retinopathien.

22. Pharmazeutische Zubereitungen nach Anspruch 20, enthaltend mindestens einen Wirkstoff nach einem der Ansprüche 1 bis 17, für die Herstellung von Arzneimitteln, die geeignet sind als Inhibitoren des Wachstums von Tumoren und der Bildung von Metastasen bei der Behandlung von Krebs.

## Claims

1. Compounds of formula (I) : 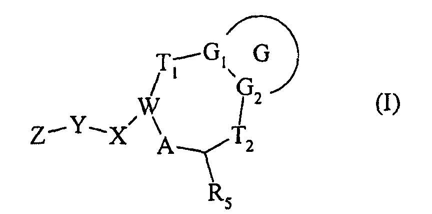 wherein :
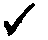 G represents a phenyl group,
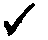 G₁ and G₂ represent a carbon atom,
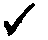 -T₁- represents a group selected from -CH₂-CH₂-, -CH=CH- and =CH-CH₂-, and -T₂- represents a bond,
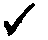 R₅ represents a -(CH₂)ₘ-COOR₆ group,
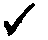 R₆ represents a hydrogen atom or an alkyl, optionally substituted aryl or optionally substituted arylalkyl group,
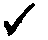 -W- represents a -CH-, =C- or -C= group and -A- represents a -CH₂- or =CH-group,
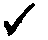 -X- represents a group selected from -CO-X₁-, -CO-NR₆-X₁-, -NR₆-CO-X₁-, -O-X₁-, -SO₂-NR₆-X₁- and -S(O)ₙ-X₁-, wherein n is from 0 to 2 inclusive and X₁ represents an alkylene group,
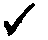 -Y- represents a group selected from -Y₁-, -Y₂-Y₁- and -Y₁-Y₂-Y₁-, wherein Y₁ represents an alkylene, alkenylene or alkynylene group and Y₂ represents an arylene, heteroarylene, cycloalkylene or heterocycloalkylene group,
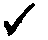 Z- represents a group selected from Z₁-, Z₁₀-NR₆- and Z₁₀-NR₆-CO- wherein Z₁₀ represents an alkyl group or Z₁, and Z₁ represents a group selected from Z₂-, 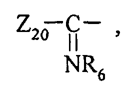 Z₂₀-NR₆- and Z₂₀-NR₆-CO- wherein Z₂₀ represents an alkyl or heteroalkyl group or Z₂, and Z₂ represents an optionally substituted heteroaryl group, an optionally substituted heterocycloalkyl group, an optionally substituted heteroarylalkyl group, an optionally substituted heterocycloalkylalkyl group, an optionally substituted fused arylheteroaryl group, an optionally substituted fused arylheterocycloalkyl group, an optionally substituted fused heteroarylheterocycloalkyl group, an optionally substituted fused heterocycloalkylheteroaryl group, an optionally substituted fused heteroarylheteroaryl group or a fused cycloalkylheterocycloalkyl group,
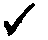 m is an integer of from 1 to 6 inclusive,
wherein :
- the term "alkyl" denotes a linear or branched group having from 1 to 6 carbon atoms,
- the term "heteroalkyl" denotes an alkyl group in which a carbon atom has been replaced by a hetero atom selected from nitrogen, oxygen and sulphur,
- the term "alkylene" denotes a linear or branched bivalent group having from 1 to 6 carbon atoms,
- the term "alkenylene" denotes a linear or branched bivalent group having from 2 to 6 carbon atoms and from 1 to 3 double bonds,
- the term "alkynylene" denotes a linear or branched bivalent group having from 2 to 6 carbon atoms and from 1 to 3 triple bonds,
- the term "cycloalkyl" denotes a saturated cyclic group having from 3 to 8 carbon atoms,
- the term "cycloalkylene" denotes a saturated cyclic bivalent group having from 3 to 8 carbon atoms,
- the term "heterocycloalkyl" denotes a saturated cyclic group having from 5 to 7 ring members and containing from I to 3 hetero atoms selected from nitrogen, oxygen and sulphur,
- the term "aryl" denotes a phenyl group or a naphthyl group,
- the term "heteroaryl" denotes an unsaturated or partially unsaturated mono- or bi-cyclic group having from 5 to 11 ring members and containing from 1 to 5 hetero atoms selected from nitrogen, oxygen and sulphur,
- the expression "fused arylheteroaryl" denotes a polycyclic group formed by an aryl group and a heteroaryl group each as defined hereinabove and conjoined by means of any one of their bonds,
- the expression "fused arylheterocycloalkyl" denotes a bi- or tri-cyclic group formed by an aryl group and a heterocycloalkyl group each as defined hereinabove and conjoined by means of any one of their bonds,
- the expression "fused heteroarylheterocycloalkyl" denotes a bi- or tri-cyclic group formed by a heteroaryl group and a heterocycloalkyl group each as defined hereinabove and conjoined by means of any one of their bonds,
- the expression "fused heterocycloalkylheteroaryl" denotes a bi- or tri-cyclic group formed by a heteroaryl group and a heterocycloalkyl group each as defined hereinabove and conjoined by means of any one of their bonds,
- the expression "fused heteroarylheteroaryl" denotes a polycyclic group formed by two heteroaryl groups as defined hereinabove and conjoined by means of any one of their bonds,
- the expression "fused cycloalkylheterocycloalkyl" denotes a bicyclic group formed by a cycloalkyl group and a heterocycloalkyl group each as defined hereinabove and conjoined by means of any one of their bonds,
- the ending "-ene" denotes that the group in question is a bivalent radical having the same meanings as the base radical,
- the expression "optionally substituted" in connection with the groups heterocycloalkyl, aryl, arylalkyl, heteroaryl, fused arylheteroaryl, fused heteroarylheterocycloalkyl, fused heteroarylheteroaryl and fused arylheterocycloalkyl denotes that those groups are unsubstituted or substituted by one or more halogen atoms or groups alkyl, alkoxy, hydroxy, mercapto, cyano, amino (optionally substituted by one or two alkyl groups), nitro, carboxy, alkoxycarbonyl, aminocarbonyl (optionally substituted by one or two alkyl groups), wherein the heteroaryl and heterocycloalkyl groups may also be substituted by an oxo group,
their enantiomers and diastereoisomers, and also addition salts thereof with a pharmaceutically acceptable acid or base.

2. Compounds of formula (I) according to claim 1, wherein R₅ represents a -CH₂-COOR₆ group, their enantiomers and diastereoisomers, and also addition salts thereof with a pharmaceutically acceptable acid or base.

3. Compounds of formula (I) according to claim 1, wherein X is selected from -CO-NR₆-X₁, -NR₆-CO-X₁ and -O-X₁, their enantiomers and diastereoisomers, and also addition salts thereof with a pharmaceutically acceptable acid or base.

4. Compounds of formula (I) according to claim 1, wherein Y represents a Y₁ or Y₁-Y₂-Y₁ group, their enantiomers and diastereoisomers, and also addition salts thereof with a pharmaceutically acceptable acid or base.

5. Compounds of formula (I) according to claim 1, wherein Z represents a heteroaryl, heterocycloalkyl, fused arylheteroaryl or fused heterocycloalkylheteroaryl group, their enantiomers and diastereoisomers, and also addition salts thereof with a pharmaceutically acceptable acid or base.

6. Compounds of formula (I) according to claim 1, wherein Z represents a Z₁₀-NR₆ group, their enantiomers and diastereoisomers, and also addition salts thereof with a pharmaceutically acceptable acid or base.

7. Compounds of formula (I) according to claim 1, wherein G represents a phenyl group, G₁ and G₂ each being a carbon atom, R₅ represents a -CH₂-COOR₆ group, R₆ being selected from a hydrogen atom and an alkyl group, T₂ represents a bond, T₁ represents a group selected from -CH=CH- and =CH-CH₂-, A represents a -CH₂- or =CH- group and W represents a -CH- or -C= group, their enantiomers and diastereoisomers, and also addition salts thereof with a pharmaceutically acceptable acid or base.

8. Compounds of formula (I) according to claim 7, wherein X is selected from the groups -CO-NR₆-X₁-, -NR₆-CO-X₁- and -O-X₁-, X₁ being a methylene group, Y represents a -Y₁- or -Y₁-Y₂-Y₁ group in which Y₁ is an alkylene group and Y₂ represents an arylene group, and Z represents a heteroaryl, heterocycloalkyl, fused arylheteroaryl or fused heterocycloalkylheteroaryl group or a Z₁₀-NR₆ group wherein Z₁₀ represents a group selected from the groups heteroaryl, heterocycloalkyl, fused arylheteroaryl and fused heterocycloalkylheteroaryl, R₆ being a hydrogen atom, their enantiomers and diastereoisomers, and also addition salts thereof with a pharmaceutically acceptable acid or base.

9. Compound of formula (I), which is [7-({[4-(2-pyridylamino)butanoyl]amino}methyl)-6,9-dihydro-5*H*-benzo[a]cyclohepten-5-yl]acetic acid.

10. Compound of formula (I), which is [7-({[5-(2-pyridylamino)pentanoyl]amino}methyl)-6,9-dihydro-5*H*-benzo[a]cyclohepten-5-yl]acetic acid hydrochloride.

11. Compound of formula (I), which is [7-(2-oxo-2-{[3-(2-pyridylamino)propyl]amino}ethyl)-5*H*-benzo[a]cyclohepten-5-yl]acetic acid.

12. Compound of formula (I), which is [7-({[4-(4,5-dihydro-1*H*-imidazol-2-ylamino)-butanoyl]amino}methyl)-6,9-dihydro-5*H*-benzo[a]cyclohepten-5-yl]acetic acid hydrochloride.

13. Compound of formula (I), which is [7-({[4-(2-pyridylamino)butanoyl]amino}methyl)-5*H*-benzo[a]cyclohepten-5-yl]acetic acid.

14. Compound of formula (I), which is [7-({[4-(1*H*-benzimidazol-2-ylamino)butanoyl]-amino}methyl)-5*H*-benzo[a]cyclohepten-5-yl]acetic acid.

15. Compound of formula (I), which is [7-({[4-(1*H*-benzimidazol-2-ylamino)butanoyl]-amino}methyl)-6,9-dihydro-5*H*-benzo[a]cyclohepten-5-yl]acetic acid.

16. Compound of formula (I), which is [7-({[4-(1,4,5,6-tetrahydro-2-pyrimidinylamino)-butanoyl]amino}methyl)-6,9-dihydro-5*H*-benzo[a]cyclohepten-5-yl]acetic acid hydrochloride.

17. Compound of formula (I), which is [7-({[4-(5,6,7,8-tetrahydro[1,8]naphthyridin-2-yl)-butanoyl]amino}methyl)-6,9-dihydro-5*H*-benzo[a]cyclohepten-5-yl]acetic acid hydrochloride.

18. Process for the preparation of the compounds of formula (I) according to claim 1, **characterised in that** there is used as starting material a compound of formula (II/a) : 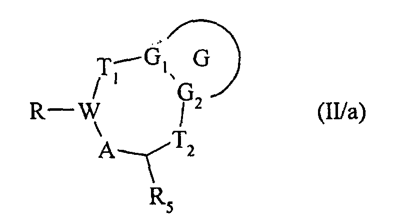 wherein G, G₁, G₂, T₁, T₂ and R₅ are as defined for formula (I), W and A are as defined hereinabove, and R represents a CHO, CN or AlkOOC-CH= group,
which, when R represents a formyl group, is subjected to a Wittig or Homer Emons reaction so as to homologise the chain carrying the aldehyde function, by using the appropriate reagent, to yield a compound of formula (III/a) : 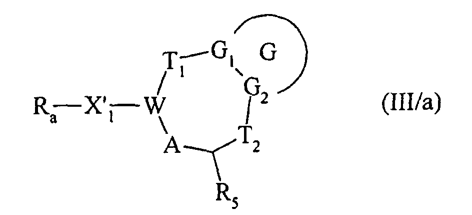 wherein G, G₁, G₂, T₁, T₂, R₅, A and W are as defined hereinabove, X'₁ represents an alkylene or alkenylene group having from two to six carbon atoms and Rₐ represents a cyano, formyl or alkoxycarbonyl group according to the reagent chosen,
which compound (III/a), with the compounds of formula (III/b) : 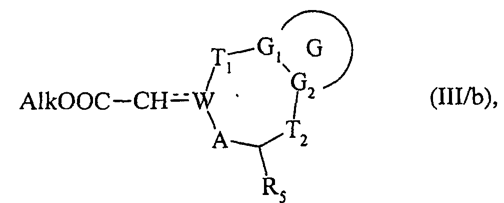 a particular case of the compounds of formula (II/a) wherein G, G₁, G₂, T₁, T₂, R₅, W and A are as defined hereinabove and Alk represents an alkyl group, the broken lines indicating the optional presence of a double bond,
constitute the entirety of the compounds of formula (III) : 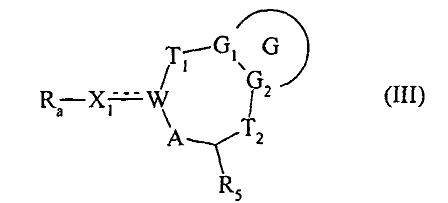 wherein G, G₁, G₂, T₁, T₂, W, A and Rₐ are as defined hereinabove and X₁ is as defined for formula (I),
the Rₐ group of which compound (III) is either reduced in alcohol, or deprotected when it represents a masked hydroxy group, to yield a compound of formula (IV) : 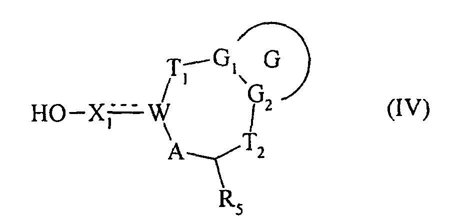 wherein G, G₁, G₂, T₁, T₂, R₅, W, A and X₁ are as defined for formula (I),
which is subjected to a halogenation reaction to yield a compound of formula (V) : 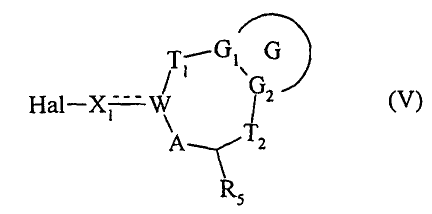 wherein G, G₁, G₂, T₁, T₂, R₅, A, W and X₁ are as defined hereinabove and Hal represents a halogen atom,
which is treated, in basic medium, with a compound of formula Z-Y-OH wherein Z and Y are as defined for formula (I), to yield a compound of formula (I/a) : 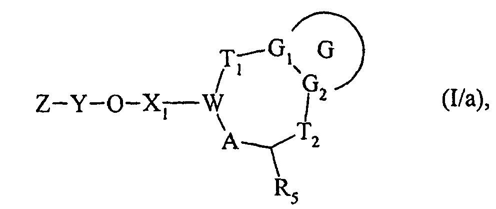 a particular case of the compounds of formula (I) wherein G, G₁, G₂, T₁, T₂, R₅, X₁, W, A, Y and Z are as defined for formula (I),
or which is treated, in basic medium, with a compound of formula Z-Y-SH wherein Z and Y are as defined for formula (I), to yield a compound of formula (I/b) : 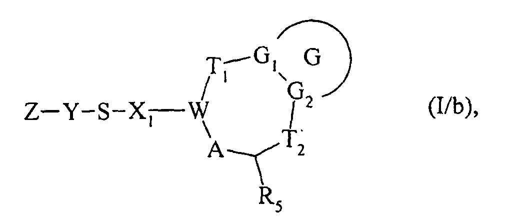 a particular case of the compounds of formula (I) wherein G, G₁, G₂, T₁, T₂, R₅, X₁, W, A, Y and Z are as defined for formula (I),
the sulphur atom of which may be oxidised to yield a compound of formula (I/c) : 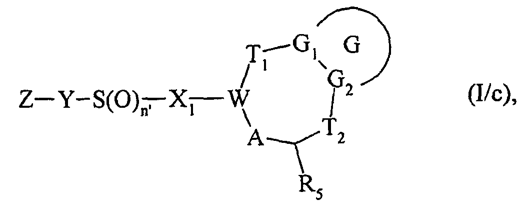 a particular case of the compounds of formula (I) wherein G, G₁, G₂, T₁, T₂, R₅, X₁, W, A, Y and Z are as defined for formula (I) and n' represents an integer 1 or 2,
or,
which compound of formula (V), subjected to the action of a cyclic imide in basic medium, followed by treatment with hydrazine in alcoholic medium, yields an amine of formula (VI) : 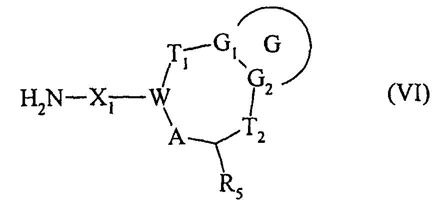 wherein G, G₁, G₂, T₁, T₂, R₅, X₁, A and W are as defined hereinabove,
it being possible furthermore for the compound of formula (VI) to be obtained directly, when X₁ represents a CH₂ group, by reduction of a compound of formula (II/a) wherein R represents a CN group,
which compound of formula (VI) is treated with a compound of formula (VII) : 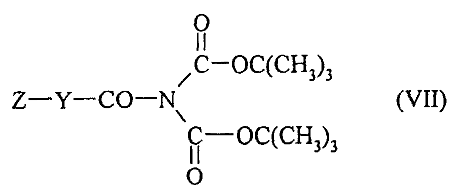 wherein Z and Y are as defined for formula (I),
to yield a compound of formula (I/d) : 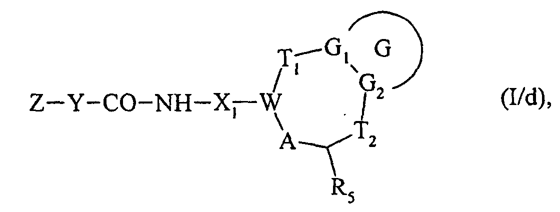 a particular case of the compounds of formula (I) wherein G, G₁, G₂, T₁, T₂, R₅, X₁, A, W, Z and Y are as defined for formula (I),
or is treated with a sulphonyl chloride of formula Z-Y-SO₂-Cl, wherein Z and Y are as defined for formula (I), to yield a compound of formula (I/e) : 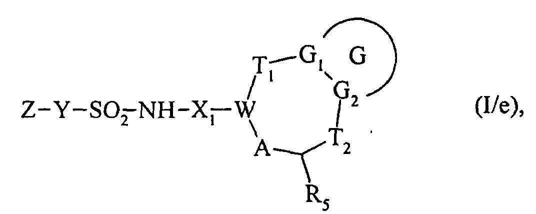 a particular case of the compounds of formula (I) wherein G, G₁, G₂, T₁, T₂, R₅, X₁, A, W, Z and Y are as defined hereinabove for formula (I),
- or the Rₐ group of which compound of formula (III) is converted into the corresponding acid to yield a compound of formula (VIII) : 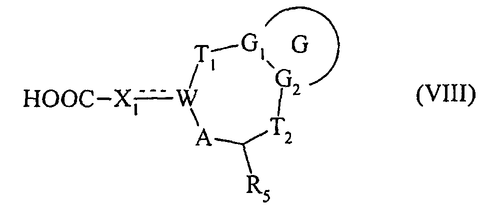 wherein G, G₁, G₂, T₁, T₂, R₅, W, A and X₁ are as defined for formula (I),
which is subjected either to the action of an amine of formula Z-Y-NH₂ or the corresponding salt to yield a compound of formula (I/f) : 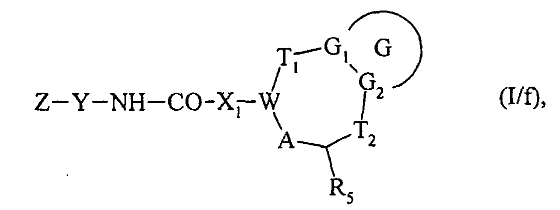 a particular case of the compounds of formula (I) wherein G, G₁, G₂, T₁, T₂, R₅, A, W, X₁, Z and Y are as defined for formula (I),
or to the action of an amine of formula : 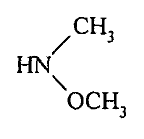 to yield a compound of formula (IX) : 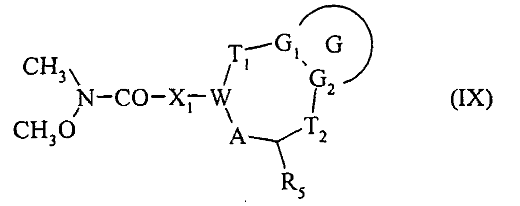 wherein G, G₁, G₂, T₁, T₂, R₅, A, W and X₁ are as defined hereinabove,
which is then subjected to the action of a lithium compound of formula Z-Y-Li, wherein Y and Z are as defined for formula (I), to yield a compound of formula (I/g) : 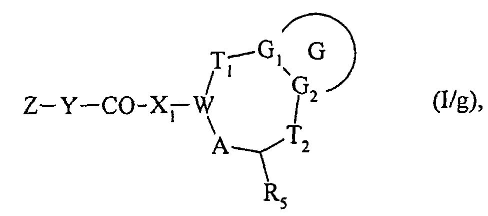 a particular case of the compounds of formula (I) wherein G, G₁, G₂, T₁, T₂, X₁, W, A, Z and Y are as defined for formula (I),
which compounds (I/a) to (I/g), which constitute the totality of the compounds of formula (I),
- may, if desired, be purified according to a conventional purification technique,
- are optionally separated into their stereoisomers according to a conventional separation technique,
- are, if desired, converted into their addition salts with a pharmaceutically acceptable acid or base.

19. Process for the preparation of the compounds of formula (I) according to claim 1, wherein Z represents a Z₁₀NR₆, group, **characterised in that** there is used as starting material a compound of formula (X) : 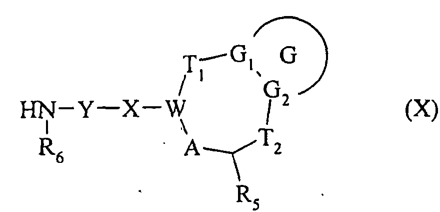 wherein Y, X, W, T₁, G, G₁, G₂, T₂, A, R₅ and R₆ are as defined for formula (I),
which is condensed, in basic medium or in the presence of a catalyst, with a compound of formula (XI) : 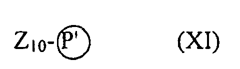 wherein Z₁₀ is as defined for formula (I) and 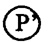 represents a leaving group (for example a halogen atom, a tosyl group or a methylthio or thioxo group),
to yield a compound of formula (I/i), a particular case of the compounds of formula (I) : 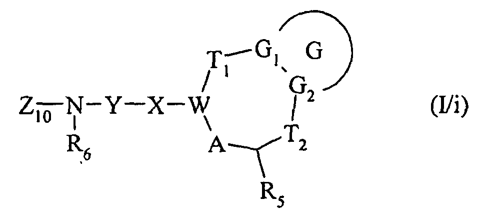 wherein Z₁₀, R₆ Y, X, W, A, T₁, T₂, G₁, G₂, R₅ et G are as defined hereinabove,
- which may, if desired, be purified according to a conventional purification technique,
- which is optionally separated into its stereoisomers according to a conventional separation technique,
- which, if desired, is converted into its addition salts with a pharmaceutically acceptable acid or base.

20. Pharmaceutical compositions comprising as active ingredient at least one compound according to any one of claims 1 to 17, alone or in combination with one or more pharmaceutically acceptable, inert, non-toxic excipients or carriers.

21. Pharmaceutical compositions according to claim 20, containing at least one active ingredient according to any one of claims 1 to 17 for use in the preparation of medicaments useful as vitronectin receptor antagonists, in the treatment of cardiovascular disease, inflammatory disorders, cancer, osteoporosis, rheumatoid arthritis, psoriasis and retinopathy.

22. Pharmaceutical compositions according to claim 20, containing at least one active ingredient according to any one of claims 1 to 17 for use in the preparation of medicaments useful as inhibitors of tumour growth and of metastases formation in the treatment of cancer.
